(19) Europäisches Patentamt
European Patent Office
Office européen des brevets

(11) Publication number: **0 040 696**
**B1**

(12) **EUROPEAN PATENT SPECIFICATION**

(45) Date of publication of patent specification: **27.08.86**

(21) Application number: **81102976.8**

(22) Date of filing: **17.04.81**

(51) Int. Cl.$^4$: **C 07 D 417/00,**
C 07 D 417/12,
C 07 D 417/14,
C 07 D 285/10, A 61 K 31/41
// C07D295/08, C07D307/85,
C07D213/70, C07D213/64,
C07D277/36, C07D307/52

(54) **Aminothiadiazoles as gastric secretion inhibitors.**

(30) Priority: **30.04.80 US 145161**
**30.04.80 US 145162**
**25.02.81 US 239775**
**25.02.81 US 238187**

(43) Date of publication of application:
**02.12.81 Bulletin 81/48**

(45) Publication of the grant of the patent:
**27.08.86 Bulletin 86/35**

(84) Designated Contracting States:
**AT BE CH DE FR GB IT LI LU NL SE**

(56) References cited:
**BE-A- 885 089**
**DE-A-1 961 864**
**GB-A-2 067 987**

**The file contains technical information submitted after the application was filed and not included in this specification**

(73) Proprietor: **MERCK & CO. INC.**
**126, East Lincoln Avenue P.O. Box 2000**
**Rahway New Jersey 07065 (US)**

(72) Inventor: **Baldwin, John J.**
**621 Gypsy Hill Circle**
**Gwynedd Valley, PA (US)**
Inventor: **Bolhofer, William A.**
**Star Route**
**Frederick, PA (US)**
Inventor: **Lumma, William C.**
**25 Newman Road M.R. 1**
**Pennsburg, PA (US)**
Inventor: **Amato, Joseph S.**
**Clinton Street, 323**
**Brooklyn, NY (US)**
Inventor: **Karady, Sandor**
**348 Longview Drive**
**Mountainside, NJ (US)**
Inventor: **Weinstock, Leonard M.**
**Box 218**
**Belle Mead, NJ (US)**

(74) Representative: **Blum, Rudolf Emil Ernst et al**
**c/o E. Blum & Co Patentanwälte Vorderberg 11**
**CH-8044 Zürich (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 040 696 B1**

**Description**

Background of the invention

Inhibitors of gastric acid secretion functioning by antagonism of the histamine H2-receptor are effective antiulcer agents. Structurally, such compounds are typically viewed as molecules having three substituents or fragments; i.e., A—B—C, each of which can independently affect the antisecretory activity. The "A" portion may be a substituted or unsubstituted aromatic or heteroaromatic group such as are disclosed in, for example, U.S. Patent 3,950,333 to Durant *et al,* U.S. Patent 4,128,658 to Price *et al* and Belgian Patent 867,106 (Derwent Abstract 84065A/47).

The central, or "B" portion, may be a connecting chain joined to A such as A—$CH_2SCH_2CH_2$—, $AOCH_2CH_2CH_2$, or A-(m-phenylene)- as disclosed in the aforementioned patents as well as in European Patent 3,640 to Jones *et al* (Derwent Abstract 61827 B/34).

The remaining terminal substituent "C" is structurally distinct from either the A and B portions and may be, for example, a substituted guanidine, a substituted 1,1-diamino ethylene, or a 3,5-diamino-1-alkyl triazole as disclosed in the aforementioned U.S. Patents to Durant *et al* and Price *et al* as well as in Belgian Patent 875,846 (Derwent Abstract 79110 B/44).

The present invention is directed to unique "C" moieties which confer antisecretory activity when combined with the A—B molecular fragments comprising these antiulcer agents. These novel, structural "C" elements are the 3,4-diamino-1,2,5-thiadiazole-1-oxide and the 3,4-diamino-1,2,5-thiadiazole-1,1-dioxide groups. Incorporation of these groups into the A—B molecular fragments affords compounds that exhibit significantly greater activity than known prior art compounds, including those disclosed in the U.S., Belgian and European Patents identified above.

Summary of the invention

This invention is concerned with 3,4-diamino-1,2,5-thiadiazole compounds which have one of the amino groups connected to a substituted or unsubstituted phenyl or heterocyclic moiety through a linear or cyclic connecting group. Thus, it is an object of this invention to describe such compounds. It is a further object of this invention to describe processes for the preparation of such compounds from the appropriately substituted amine and thiadiazole precursors. A still further object is to describe the use of such compounds as gastric secretion inhibitors in mammals. Further objects will become apparent from a reading of the following description.

Description of the invention

The inventive compounds have the following formula:

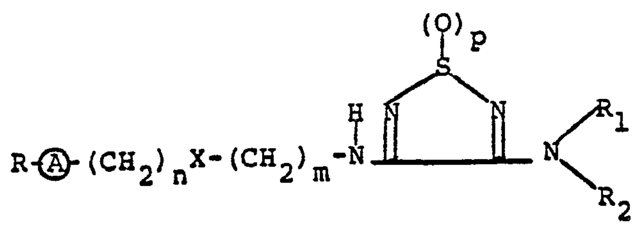

(I)

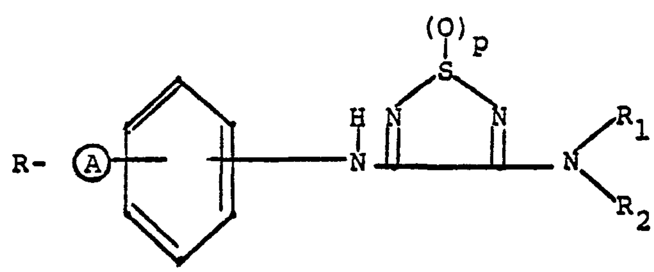

(II)

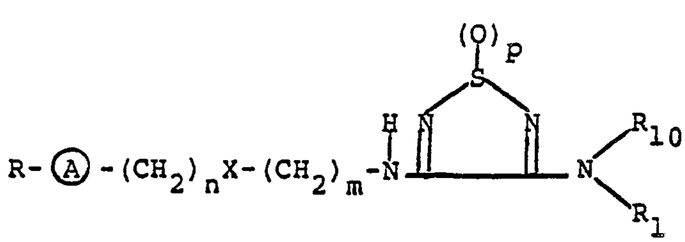

(IIIA)

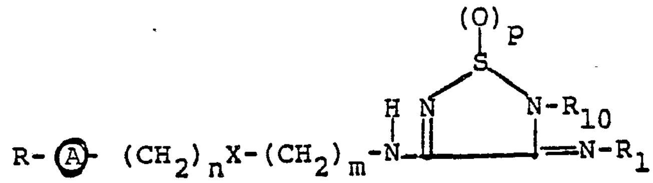

(IIIB)

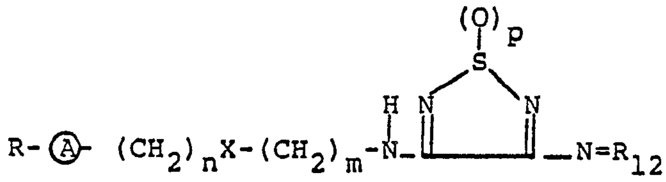

(IVA)

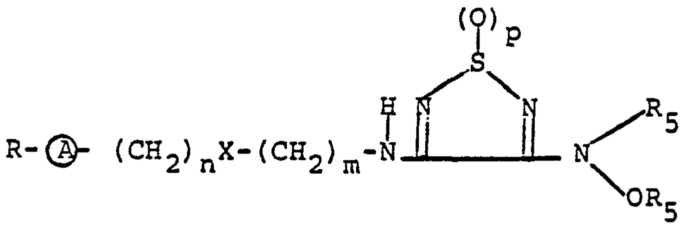

(IVB)

wherein R is hydrogen, alkyl having 1—5 carbon atoms or a group having the formula

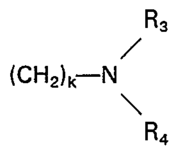

or

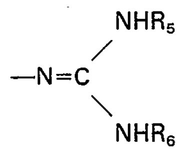

wherein

$R_3$ and $R_4$ are independently hydrogen, alkyl having 1—5 carbon atoms, cycloalkyl having 3—7 carbon atoms or phenylalkyl wherein the alkyl group has 1—5 carbon atoms, or

$R_3$ and $R_4$ form together with the nitrogen atom to which they are attached, a 5- or 6-membered heterocycle, which may also contain an oxygen, sulfur or N—$R_7$ heteroatom, wherein

$R_7$ is hydrogen, alkyl of from 1 to 3 carbon atoms or benzyl;

$R_5$ and $R_6$ are independently hydrogen or alkyl having 1—5 carbon atoms

n is 0 or 1;

m is 2 to 4

k is 1 to 4;

X is oxygen, sulfur or methylene;

p is 1 or 2;

$R_1$ and $R_2$ are independently hydrogen, alkyl having 1—5 carbon atoms, alkenyl having 3—5 carbon atoms, alkynyl having 3—5 carbon atoms, cycloalkyl having 3—7 carbon atoms, phenyl, pyridyl and substituted alkyl having 1—5 carbon atoms in the alkkyl group, wherein the substituent is phenyl, cycloalkyl having 3—7 carbon atoms, pyridyl, imidazolyl, morpholino, hydroxy, alkoxy having 1—5 carbon atoms, alkylthio having 1—5 carbon atoms or dialkylamino wherein each alkyl group has 1—5 carbon atoms or

$R_1$ and $R_2$ form together with the nitrogen to which they are attached, a 5- or 6-membered heterocycle which may also contain an oxygen, sulfur or N—$R_7$ heteroatom wherein $R_7$ is as defined above;

Ⓐ is phenylene or a 5- or 6-membered heterocyclo containing one to three heteroatoms selected from oxygen, sulfur or nitrogen, which may optionally have a benzo ring fused thereon;

provided that when Ⓐ in formula I above is a 5-membered heterocycle or a benzo fused 5-membered heterocycle, n is 1; and,

provided further that when Ⓐ in either formula I or II is a furan having the structure

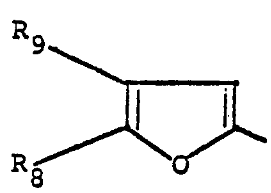

then

$R_8$ is the same as R defined above and

$R_9$ is alkyl having 1—5 carbon atoms, alkoxyalkyl in which the alkoxy group and the alkyl group have each 1—5 carbon atoms, alkoxycarbonyl in which the alkoxy group has 1—5 carbon atoms, halo, preferably chlorine or bromine, or aryl, preferably phenyl;

$R_{10}$ is alkanoyl in which the alkyl group contains 1—5 carbon atoms, a phenylcarbonyl group in which the phenyl nucleus is optionally substituted with 1, 2 or 3 substituents, selected from halo, alkyl and alkoxy both having 1—5 carbon atoms, heteroaroyl in which the heterocyclic ring is a 6-membered N-containing ring having 1 or 2 heteroatoms, alkylsulfonyl in which the alkyl group has 1—5 carbon atoms, arylsulfonyl, heteroarylsulfonyl, in which the heterocyclic ring is a 6-membered N-containing ring having 1 or 2 heteroatoms, cyano, amidino, dialkylcarbamoyl in which the alkyl group contains 1—5 carbon atoms, dialkylsulfamoyl in which the alkyl group contains 1—5 carbon atoms, alkoxycarbonyl, in which the alkoxy group contain 1—5 carbon atoms or a group having the formula

$$—(CH_2)_yCOR_{11}$$

wherein

y is 1 or 2; and

$R_{11}$ is alkyl having 1—5 carbon atoms, hydroxy, alkoxy both having 1—5 carbon atoms, amino, alkylamino in which the alkyl group has 1—5 carbon atoms or dialkylamino in which the alkyl groups each have 1—5 carbon atoms; and

$R_{12}$ is aralkylidene or diarylmethylidene;

or they are salts of the compounds of formulae I, II, IIIa, IIIb, IVa and Vb and solvates of said compounds or solvates of the salts of said compounds as well as quaternary ammonium salts and N-oxides.

If in the inventive compounds the radicals $R_3$ and $R_4$ form together with the nitrogen atom to which they are attached a 5- or 6-membered heterocyclic ring or if the radicals $R_1$ and $R_2$ form together with the nitrogen atom to which they are attached, such a 5- or 6-membered heterocyclic ring, then preferred such ring structures are selected from pyrrolidino, piperidino, thiomorpholino, morpholine, or N-loweralkyl-piperazino.

Ⓐ in the foregoing formulae may be furan, thiphene, pyrrole, oxazole, oxadiazole, thiadiazole, thiazole, triazole, pyrazole, imidazole, pyridine, pyrimidine, pyrazine, and the fused benzo derivatives thereof such as benzofuran, benzoxazole or benzimidazole.

The inventive compounds of formulae IIIA and IIIB are derivatives of the inventive compounds of formula I. The compounds of formulae IIIA and IIIB can be prepared by reacting the compounds of formula I or tautomers thereof with alkylating agents such as, for example, α-haloketones, aldehydes, acids, esters, or amides; or, α,β-unsaturated aldehydes, acids, esters, or amides by alkylation processes known to those skilled in the art.

Further derivatives in which $R_{10}$ in compounds of formulae IIIA and IIIB can be one of the groups hereinbefore specified other than $—(CH_2)_yCOR_{11}$ can be obtained by reacting compounds of formula I with acyl halides, acyl anhydrides, sulfonyl halides, cyanogen halides, S-alkylisothioureas, carbamoyl halides, sulfamoyl halides, or haloformates. In these reactions, mixtures of compounds IIIA and IIIB are obtained which can be separated by conventional means such as, for example, chromatography and fractional crystallization.

The inventive compounds of formula IVA can be prepared by reacting an intermediate compound having the formula VI:

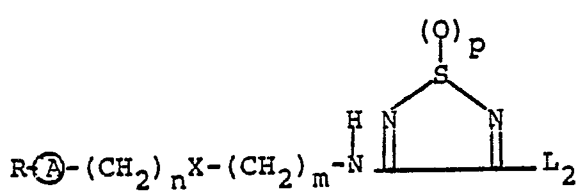

(VI)

wherein R, Ⓐ, X, m, n and p are as defined in formula IVA and $L_2$ is a leaving group with an imine such as

$R_5$
aryl—C=NH or

$(aryl)_2C=NH$ wherein $R_5$ is as defined in formula IVA.

The compounds of formula IVB can be prepared by reacting the intermediate product having the formula VI with a compound having the following formula:

$$\begin{array}{c} R_5 \\ | \\ HN{-}OR_5 \end{array}$$

wherein $R_5$ has the same meaning as in formula IVB.

The alkyl groups having 1—5 carbon atoms can have either a straight or branched configuration. Examples of such alkyl groups are methyl, ethyl, propyl, *iso*-propyl, butyl, *sec*-butyl and pentyl.

Examples for cycloalkyl groups having 3—7 carbon atoms are cyclopropyl, cyclobutyl, cyclopentyl, cyclohexyl, and cycloheptyl.

The alkenyl groups having 3—5 carbon atoms have either a straight or branched configuration and one unsaturation. Examples of such alkenyl groups are propenyl, butenyl and pentenyl.

The alkynyl groups having 3—5 carbon atoms have either a straight or branched configuration, and one triple bond. Examples of such alkynyl groups are propargyl, butynyl and pentynyl.

Examples for alkoxyalkyl groups having 1—5 carbon atoms in each of the alkyl moiety are corresponding groups with straightchained or branched alkyl groups. Examples for such groups are methoxymethyl, ethoxymethyl, methoxypropyl and methoxybutyl.

Examples for hydroxyalkyl groups having 1—5 carbon atoms are hydroxymethyl, hydroxyethyl, hydroxypropyl and hydroxybutyl.

Examples for alkoxycarbonyl groups having 1—5 carbon atoms in the alkoxy group are methoxycarbonyl, ethoxycarbonyl, propoxycarbonyl and butoxycarbonyl.

The preferred compounds of the instant invention will have definitions which vary with the nature of the compounds of formula I and II.

In formula I preferred compounds are realized when $R_1$ and $R_2$ are independently hydrogen, alkyl having 1—5 carbon atoms, alkynyl having 3—5 carbon atoms, substituted alkyl groups having 1—5 carbon atoms in which the substituent is phenyl, pyridyl or imidazolyl; and $R_3$ and $R_4$ are independently hydrogen, alkyl having 1—5 carbon atoms or $R_3$ and $R_4$ are joined to form one of the following heterocyclic rings: piperidine, morpholine or N-alkyl piperazine, wherein the alkyl group has 1—3 carbon atoms.

Further preferred compounds are realized when $R_1$ and $R_2$ are independently hydrogen or alkyl having 1—5 carbon atoms and p is 1 or 2.

Further such preferred compounds are those wherein Ⓐ is m-phenylene or a 6-membered heterocycle as defined above, n is O, X is oxygen, m is 3 and R is:

$$\begin{array}{l} \qquad\qquad\quad R_3 \\ \qquad\qquad\;\; / \\ -(CH_2)_k{-}N \\ \qquad\qquad\;\; \backslash \\ \qquad\qquad\quad R_4 \end{array}$$

In such cases it is further preferred that k be 1 and $R_3$ and $R_4$ be alkyl having 1—5 carbon atoms, preferably methyl, or joined to form a morpholine, piperidine or N-methyl piperazine ring.

Additional preferred variations of Ⓐ are those wherein Ⓐ is furan, imidazole, thiazole, oxazole, thiophene, triazole, thiadiazole, oxadiazole or benzofuran.

When Ⓐ is heterocyclic it is preferred that n = 1, X = sulfur and m = 2.

The preferred values of R will depend upon and vary with the definition of Ⓐ.

When Ⓐ is furan or benzofuran, R is preferred to be:

$$\begin{array}{l} \qquad\qquad\quad R_3 \\ \qquad\qquad\;\; / \\ -(CH_2)_k{-}N \\ \qquad\qquad\;\; \backslash \\ \qquad\qquad\quad R_4 \end{array}$$

wherein it is further preferred that k = 1 and $R_3$ and $R_4$ be hydrogen or alkyl having 1—5 carbon atoms, preferably hydrogen or methyl, or joined to form a morpholine, piperidine or N-methyl piperazine ring.

When Ⓐ is imidazole, R is preferably alkyl having 1—5 carbon atoms, most preferably methyl.

When Ⓐ is thiazole, R is preferably:

$$\begin{array}{l} \qquad\qquad NHR_5 \\ \qquad\quad\;\; / \\ -N{=}C \\ \qquad\quad\;\; \backslash \\ \qquad\qquad NHR_6 \end{array}$$

where $R_5$ and $R_6$ are most preferably hydrogen.

In formula II it is preferred that $R_1$ and $R_2$ are independently hydrogen or alkyl having 1—5 carbon atoms and p is 1 or 2.

It is further preferred that the phenyl ring in formula II be connected to the adjacent groups at its meta positions.

As in formula I compounds the preferred values of R in formula II compounds will vary with the nature of Ⓐ. The preferred values of Ⓐ are imidazole, and thiazole.

When Ⓐ is imidazole, it is preferred that R be alkyl having 1—5 carbon atoms, most preferably methyl.

When Ⓐ is thiazole, it is preferred that R be:

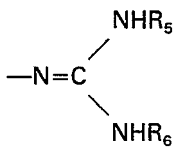

and wherein it is most preferred that $R_5$ and $R_6$ be hydrogen.

A specially preferred compound of formula I is the 3-N-[3-(1-piperidinylmethyl)phenoxy]propyl]amino-4-amino-1,2,5-thiadiazole-1-oxide.

Said specially preferred compound of formula I can be prepared by reacting the 3-ethoxy-4-amino-1,2,5-thiadiazole-1-oxide with 3-[3-(1-piperidinylmethyl)phenoxy]propane-amine yielding the 3-N-[3-(1-piperidinylmethyl)phenoxy)propyl]amino-4-amino-1,2,5-thiadiazole-1-oxide. Said reaction is further described in example 19.

Further specific examples of preferred compounds of the invention are:

3-N-[2-[(2-furanyl)methylthio]ethyl]amino-4-amino-1,2,5-thiadiazole-1,1-dioxide;

3-N-[2-[(5-methylaminomethyl-2-furanyl)methylthio]ethyl]amino-4-amino-1,2,5-thiadiazole-1,1-dioxide;

3-N-[2-[(5-dimethylaminomethyl-2-furanyl)methylthio-ethyl]amino-4-methylamino-1,2,5-thiadiazole-1-oxide;

3-N-[2-[(5-dimethylaminomethyl-2-furanyl)methylthio]ethyl]amino-4-methylamino-1,2,5-thiadiazole-1,1-dioxide;

3-N-[2-[(5-furanyl)methylthio]ethyl]amino-4-amino-1,2,5-thiadiazole-1-oxide;

3-N-[2-[(2-guanidino-4-thiazolyl)methylthio]ethyl]amino-4-methylamino-1,2,5-thiadiazole-1-oxide;

3-N-[2-[(5-dimethylaminomethyl-2-furanyl)methylthio]ethylamino]-4-dimethylamino-1,2,5-thiadiazole-1-oxide;

3-N-[3-[2-guanidino-4-thiazolyl)phenyl]amino-4-methylamino-1,2,5-thiadiazole-1-oxide;

3-N-[3-[2-guanidino-4-thiazolyl)phenyl]amino-4-methylamino-1,2,5-thiadiazole-1,1-dioxide;

3-N-[2-[5-dimethylaminomethyl-2-furanyl)methylthio]ethyl]amino-4-(2-propynyl)amino-1,2,5-thiadiazole-1-oxide;

3-N-[2-[(5-dimethylaminomethyl-2-furanyl)methylthio]ethyl]amino-4-amino-1,2,5-thiadiazole-1-oxide;

3-N-[2-[(2-guanidino-4-thiazolyl)-methylthio]ethyl]amino-4-methylamino-1,2,5-thiadiazole-1,1-dioxide;

3-N-[3-[3-(dimethylaminomethyl)phenoxy]propyl]amino-4-amino-1,2,5-thiadiazole-1-oxide;

3-N-[2-[[3-(dimethylaminomethyl)phenyl]methylthio]ethyl]amino-4-amino-1,2,5-thiadiazole-1-oxide;

3-N-[3-[3-(dimethylaminomethyl)phenoxy]propyl]amino-4-methylamino-1,2,5-thiadiazole-1-oxide;

3-N-[3-[3-[(4-methyl-1-piperazinyl)methyl]phenoxy]propyl]amino-4-methylamino-1,2,5-thiadiazole-1-oxide;

3-N-[3-[3-(4-morpholinomethyl)phenoxy]propyl]amino-4-methylamino-1,2,5-thiadiazole-1-oxide;

3-N-[3-[3-(4-morpholinomethyl)phenoxy]propyl]amino-4-amino-1,2,5-thiadiazole-1-oxide;

3-N-[2-[(4-methyl-5-imidazolyl)methylthio]ethyl]amino-4-methylamino-1,2,5-thiadiazole-1-oxide;

3-N-[2-[(4-methyl-5-imidazolyl)methylthio]ethyl]amino-4-amino-1,2,5-thiadiazole-1-oxide;

3-N-[2-[[5-(1-piperidinylmethyl)-2-furanyl]methylthio]ethyl]amino-4-amino-1,2,5-thiadiazole-1-oxide;

3-N-[2-[[5-(4-morpholinylmethyl)-2-furanyl]methylthio]ethyl]amino-4-amino-1,2,5-thiadiazole-1-oxide;

3-N-[3-[3-(dimethylaminomethyl)phenoxy]propyl]amino-4-amino-1,2,5-thiadiazole-1-oxide;

3-N-[3-[3-(1-piperidinylmethyl)phenoxy]propyl]amino-4-amino-1,2,5-thiadiazole-1-oxide;

3-N-[3-[3-(4-morpholinylmethyl)phenoxy]propyl]amino-4-amino-1,2,5-thiadiazole-1-oxide;

3-N-[2-[[6-(4-morpholinylmethyl)-2-benzofuranyl]methylthio]ethyl]amino-4-amino-1,2,5-thiadiazole-1-oxide;

3-N-[2-[(2-guanidino-4-thiazolyl)methylthio]ethyl]amino-4-amino-1,2,5-thiadiazole-1-oxide;

3-N-[3-(2-guanidino-4-thiazolyl)phenyl]amino-4-amino-1,2,5-thiadiazole-1-oxide;

3-N-[2-[(5-dimethylaminomethyl-2-furanyl)methylthio]ethyl]amino-4-amino-1,2,5-thiadiazole-1,1-dioxide;

3-N-[3-[3-(dimethylaminomethyl)phenoxy]propyl]amino-4-amino-1,2,5-thiadiazole-1,1-dioxide;

3-N-[2-[[6-(4-morpholinyl-2-benzofuranyl]methylthio]ethyl]amino-4-amino-1,2,5-thiadiazole-1,1-dioxide;

3-N-[3-(2-guanidino-4-thiazolyl)phenyl]amino-4-amino-1,2,5-thiadiazole-1,1-dioxide;

3-N-[2-[[6-(4-morpholinyl)-2-benzofuranyl]methylthio]ethyl]amino-4-methylamino-1,2,5-thiadiazole-1-oxide;

3-N-[3-[3-(dimethylaminomethyl)phenoxy]propyl]-amino-4-methylamino-1,2,5-thiadiazole-1,1-dioxide;

3-N-[2-[(2-guanidino-4-thiazolyl)methylthio]ethyl]amino-4-cyclohexylamino-1,2,5-thiadiazole-1-oxide;

3-N-[2-[(5-dimethylaminomethyl-2-furanyl)methylthio]ethyl]amino-4-phenylamino-1,2,5-thiadiazole-1,1-dioxide;

3-N-[2-[[6-(4-morpholinyl)-2-benzofuranyl]methylthio]ethyl]amino-4-(2-pyridylamino)-1,2,5-thiadiazole-1-oxide;

3-N-[2-[(4-methyl-5-imidazolyl)methylthio]ethyl]amino-4-phenethylamino-1,2,5-thiadiazole-1-oxide;

3-N-[3-[3-(4-morpholinylmethyl)phenoxy]propyl]amino-4-cyclohexylmethylamino-1,2,5-thiadiazole-1,1-dioxide;

3-N-[2-[(5-dimethylaminomethyl-2-furanyl)methylthio]ethyl]amino-4-[(2-pyridylmethyl)amino]-1,2,5-thiadiazole-1,1-dioxide;

3-N-[3-[3-(dimethylaminomethyl)phenoxy]propyl]amino-4-[2-(3-pyridyl)ethyl]amino-1,2,5-thiadiazole-1-oxide;

3-N-[3-(2-guanidino-4-thiazolyl)phenyl]amino-4-[2(1-imidazolyl)ethyl]amino-1,2,5-thiadiazole-1,1-dioxide;

3-N-[2-[(5-dimethylaminomethyl-2-furanyl)methylthio]ethyl]amino-4-(2-methoxyethyl)amino-1,2,5-thiadiazole-1-oxide;

3-N-[2[(2-guanidino-4-thiazolyl)methylthio]ethyl]amino-4-dimethylaminoethyl-1,2,5-thiadiazole-1,1-dioxide;

3-N-[2-[(5-dimethylaminomethyl-2-furanyl]methylthio]ethyl]amino-4-(1-piperidino)-1,2,5-thiadiazole-1-oxide;

3-N-[2-[(5-dimethylaminomethyl-2-furanyl)methylthio]ethyl]amino-4-ethoxy-1,2,5-thiadiazole-1-oxide;

3-N-[2-[(5-dimethylaminomethyl-2-furanyl)methylthio]ethyl]amino-4-ethoxy-1,2,5-thiadiazole-1,1-dioxide;

3-N-[3-[(5-dimethylaminoethyl-2-furanyl)methylthio]propyl]amino-4-amino-1,2,5-thiadiazole-1-oxide;

3-N-[4-[(5-dimethylaminoethyl-2-furanyl)-methylthiobutyl]amino-4-methylamino-1,2,5-thiadiazole-1,1-dioxide;

3-N-[3-[3-(dimethylaminomethyl)phenylthio]propyl]amino-4-amino-1,2,5-thiadiazole-1-oxide;

3-N-[2-[3-(4-morpholinylmethylphenyl)methylthio]ethyl]amino-4-methylamino-1,2,5-thiadiazole-1-oxide;

3-N-[4-(3-dimethylaminomethyl)butyl]amino-4-methylamino-1,2,5-thiadiazole-1-oxide;

3-N-[2-[[5-(2-N-methylaminoethyl)-2-thienyl]methylthio]ethyl]amino-4-amino-1,2,5-thiadiazole-1-oxide;

3-N-[3-(6-morpholinylmethyl-2-pyridinyloxy)propyl]amino-4-amino-1,2,5-thiadiazole-1-oxide;

3-N-[2-[[5-(2-methylguanidino)-1,2,4-thiadiazol-3-yl]-methylthio]ethyl]amino-4-amino-1,2,5-thiadiazole-1-oxide;

3-N-[2-[(5-guanidino-1,2,4-oxadiazol-3-yl)methylthio]ethyl]amino-4-amino-1,2,5-thiadiazole-1-oxide;

3-N-[2-[[6-(dimethylaminomethyl)-2-pyrazinyl]methylthio]ethyl]amino-4-amino-1,2,5-thiadiazole-1,1-dioxide;

3-N-[2-[(5-methyl-4-oxazolyl)methylthio]ethyl]amino-4-amino-1,2,5-thiadiazole-1-oxide;

3-N-[2-[6-(dimethylaminomethyl)-2-benzo[b]thienylmethylthio]ethyl]amino-4-amino-1,2,5-thiadiazole-1,1-dioxide;

3-N-[3-[5-(N-ethylamino)methyl-3-pyridyloxy]propyl]amino-4-amino-1,2,5-thiadiazole-1-oxide.

The compounds according to the invention readily form physiologically acceptable salts. Such salts include salts with inorganic acids such as hydrochlorides, hydrobromides, sulfates, nitrates and phosphates. Particularly useful salts of organic acids are formed with aliphatic mono- or dicarboxylic or sulfonic acids. Examples of such salts are acetates, maleates, fumarates, tartrates, citrates, benzoates, succinates, methane sulfonates, and isethionates. The compounds and their salts may also form solvates like e.g. hydrates. In addition, the nitrogen atoms in groups R, Ⓐ, $R_1$ and $R_2$ may also form quaternary salts and N-oxides.

It will also be appreciated by those skilled in the art that the compounds of this invention will have a tautomeric isomerism about the nitrogen atoms adjacent to the thiadiazole ring. Where one or both of the adjacent nitrogen atoms has a hydrogen atom thereon, the *exo*-imino structure may be formed on one or both of such nitrogen atoms as shown in the following structure:

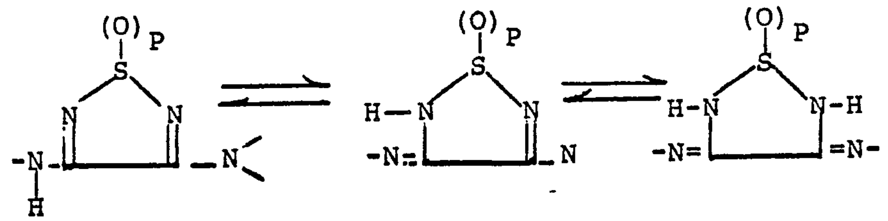

All of the various tautomeric structures of the instant compounds are intended to be included in this invention. In addition, when R is guanidino, three tautomeric structure variations are possible, and all such tautomers are included in this invention.

In addition, when p is 1, there exists the possibility of stereoisomerism in the instant compounds, corresponding to an R or S absolute configuration at the sulfur atom of the thiadiazole ring. It is intended that all such stereoisomers are included within the instant invention.

As stated above, the inventive compounds of formulae I, II, IIIA, IIIB, IVA and IVB suppress gastric acid secretion in the animal body and the human body.

A further subject of the invention, accordingly, is a composition useful for suppressing gastric acid secretion in animals with excess gastric acid secretion which is characterized in that it comprises an inert carrier and an effective amount of a compound of formulae I, II, IIIA, IIIB, IVA or IVB or a pharmaceutically acceptable salt of said compounds or a solvate of the compounds or a solvate of the salt of said compounds or quaternary ammonium salts or N-oxides of said compounds.

A typical example for a solvate of the compounds or the salts thereof is a corresponding hydrate.

The inventive compounds have been found to have pharmacological activity in the animal body as antagonists to certain actions of histamine which are not blocked by "antihistamines" such as mepyramine. For example, they have been found to inhibit selectively the histamine-stimulated secretion of gastric acid in the stomach of chronic fistula dogs at doses of from 0.001 to 5 mg per kilogram intravenously or orally from 0.01 to 10 mg per kilogram. Similarly, the action of these compounds is demonstrated by their antagonism to the effects of histamine on other tissues which are not affected by histamine HI antagonists. An example of such tissue is the isolated guinea-pig right atrium.

These compounds have been found to be up to 200 times more active in animal models than currently marketed gastric antisecretory H2 antihistamines.

The pharmaceutical carrier employed may be for example, either a solid or liquid. Exemplary of solid carriers are lactose, terra alba, sucrose, talc, gelatin, agar, pectin, acacia, magnesium stearate and stearic acid. Exemplary of liquid carriers are syrup, peanut oil, olive oil and water.

A wide variety of pharmaceutical forms can be employed. Thus, if a solid carrier is used, the preparation can be tableted, placed in a hard gelatin capsule in powder or pellet form, or in the form of a troche or lozenge. The amount of solid carrier will vary widely but preferably will be from about 15 mg to about 0.4 gm. If a liquid carrier is used, the preparation may be in the form of a syrup, emulsion, soft gelatin capsule, sterile injectable liquid such as an ampoule, or an aqueous of nonaqueous liquid suspension.

The pharmaceutical compositions are prepared by conventional techniques involving procedures such as mixing, granulating and compressing or dissolving the ingredients as appropriate to the desired preparation.

The active ingredient will be present in the composition in an effective amount to inhibit histamine gastric acid secretory activity. The route of administration may be orally or parenterally.

Preferably, each daily dosage will contain the active ingredient in an amount of from about 1 mg to about 500 mg, most preferably from about 20 mg to about 200 mg given in a single dose or multiple divided doses.

For therapeutic use, the pharmacologically active compounds of the present invention will normally be administered as a pharmaceutical composition comprising as the or an essential active ingredient at least one such compound in the basic form or in the form of an addition salt with a pharmaceutically acceptable acid and in association with a pharmaceutical carrier therefor. Such addition salts include those mentioned above.

Other pharmacologically active compounds may in certain cases be included in the composition. It may be appropriate to combine the instant compound or compounds with anticholinergic agents such as propantheline; H1 antihistamines such as mepyramine, pyribenzamine and chlorpheniramine; or prostanoids.

Advantageously the composition will be made up in a dosage unit form appropriate to the desired mode of administration, for example as a tablet, capsule or injectable solution.

A further subject of the present invention is a process for the preparation of the inventive compounds of which comprises treating a compound having the formula:

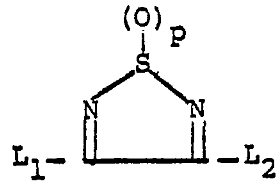

wherein p is 1 or 2 and $L_1$ and $L_2$ are leaving groups, first with one of the amines having the formulae

$R-Ⓐ-(CH_2)_n-X-(CH_2)_m-NH_2$

or

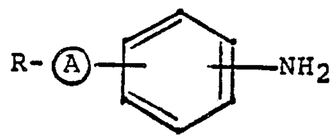

or

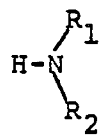

wherein
R, Ⓐ, n, X, m, $R_1$ and $R_2$ are as defined before yielding an intermediate product of formula VI

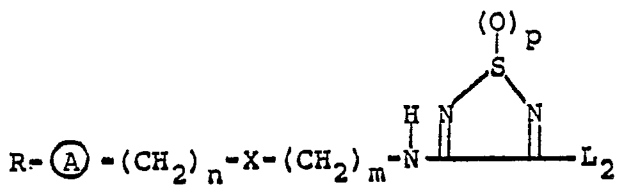

(VI)

or VIa

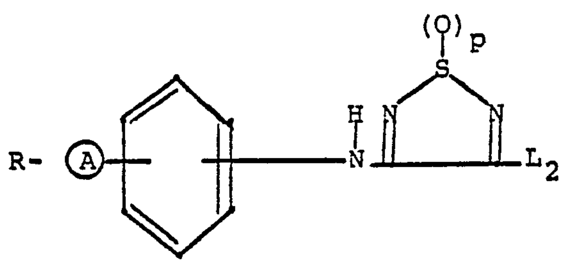

(VIa)

or
VII

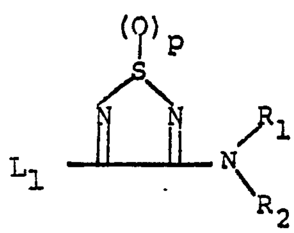

(VII)

and reacting thereafter the intermediate product of formulae VI and VIa with the amine of formula

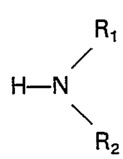

or the intermediate product of formula VII with either the amine

$R-Ⓐ-(CH_2)_n-X-(CH_2)_m-NH_2$

or the amine

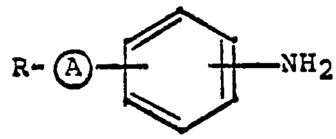

to yield the final products of formula I or II and optionally reacting a compound of formula I or II in which the group

R—Ⓐ—

is an unsubstituted furane ring with either

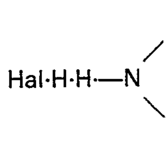

$+ CH_2O$ or

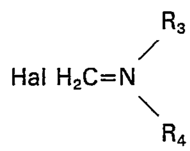

wherein Hal is halo to produce corresponding compounds of formula I or II wherein A is a furane ring substituted with a group R having the formula

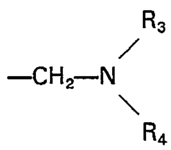

wherein $R_3$ and $R_4$ are as defined before or reacting the intermediate compound of formula VI

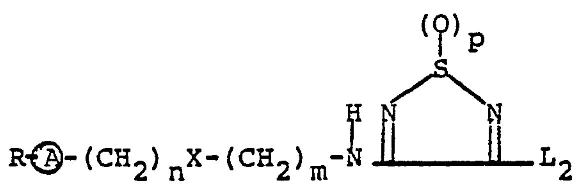

(VI)

with an imine of formula

$H—N=R_{12}$

to produce compounds of formula IVa

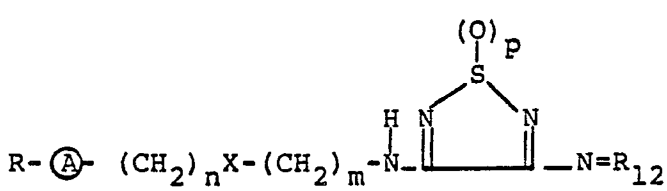

(IVA)

or reacting the intermediate produce of formula VI

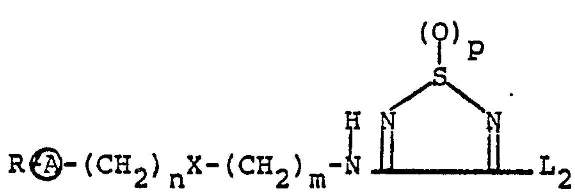

(VI)

with an optionally substituted hydroxyl amine of formula

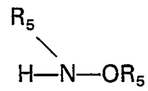

to produce compounds of formula IVb

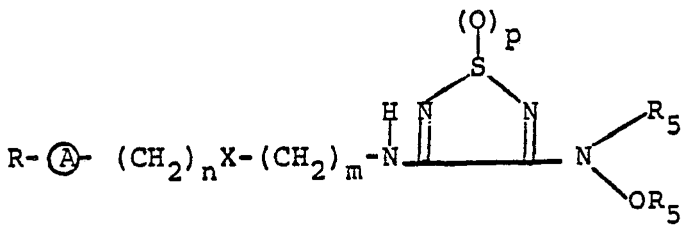

(IVB)

wherein R, $R_5$, Ⓐ, X, m, n and p are as defined before and wherein the compounds of formulae I, II, IVa or IVb are isolated as free bases or salts or as solvantes of the free bases or the salts or are converted into quaternary ammonium salts or N-oxides of said compounds.

A further object of the present invention is a process for the preparation of compounds having the formulae I, II, IIIA or IIIB which comprises reacting an alcohol having the formula:

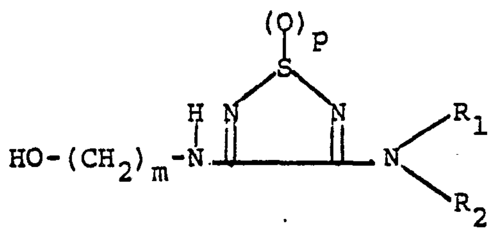

(XVI)

wherein $R_1$, $R_2$ and p are as defined in formulae I and II and $m > 2$, with an acylating agent to obtain an activated ester intermediate; reacting said ester intermediate with an alkali metal salt of 3-hydroxy benzaldehyde to obtain an intermediate having the formula:

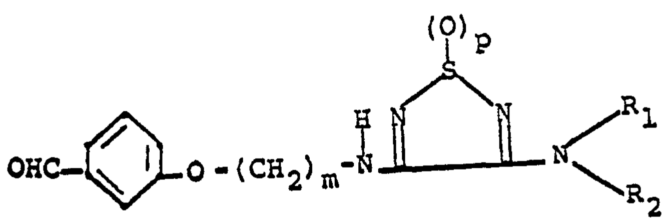

(XIX)

and, reducing said intermediate in the presence of amine $R_3NHR_4$, wherein $R_3$ and $R_4$ are as defined in formulae I or II or IIIA or IIIB, reacting said ester intermediate when $m = 2$ with a mercaptan having the formula R—Ⓐ—$(CH_2)_n$—SH wherein R, Ⓐ and n are as defined in formulae I, II, IIIA and IIIB or reacting an alcohol having the formula

R—Ⓐ—$(CH_2)_n$—X—$CH_2CH_2OH$

wherein R, Ⓐ, X and n are as defined in formulae I, II, IIIA and IIIB with, after activation of said alcohol toward nucleophiles, an amino compound having the formula:

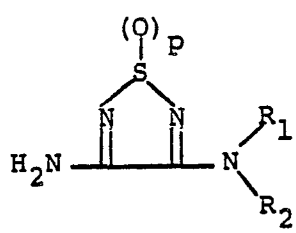

wherein $R_1$, $R_2$ and p are as defined in formulae I, II, IIIA and IIIB, or, reacting a mercaptan having the formula:

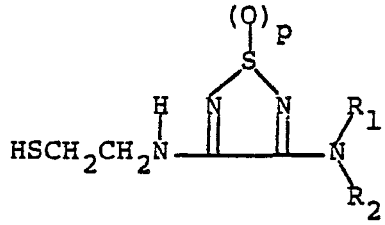

wherein $R_1$, $R_2$ and p are as defined in formulae I and II, with an alcohol having the formula R—Ⓐ—$(CH_2)_n$—OH wherein R, Ⓐ and n are as defined in formulae I and II, in the presence of a mineral acid to afford the compounds of formulae I and II; and the physiologically acceptable salts thereof; or, reacting the compounds of formula I and tautomers thereof with an alkylating reagent, an acylating agent or a sulfonating agent to produce derivatives having the formulae:

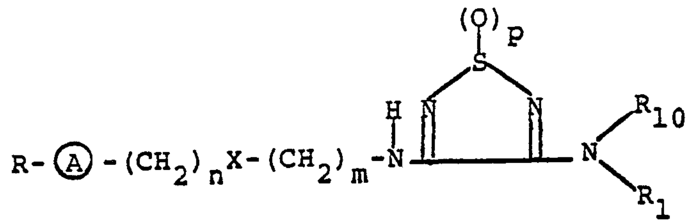

(IIIA)

and

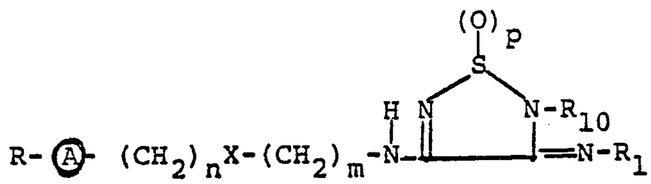

(IIIB)

wherein $R_{10}$ is as defined before; and, the physiologically acceptable salts thereof.

New intermediate products which are prepared in processes for the preparation of the compounds of formulae I, II, IIIA, IIIB, IVA and IVB are the compounds having the formulae

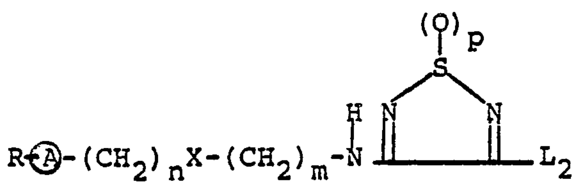

(VI)

and

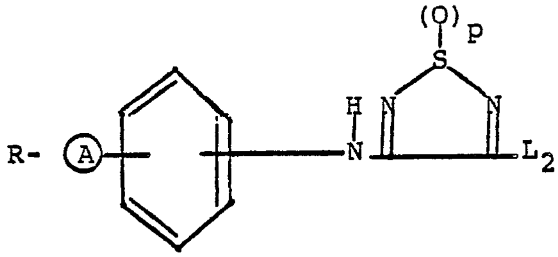

(VIA)

wherein R, Ⓐ, n, X, and m are as defined in formulae I, II, IIIA, IIIB, IVA and IVB

p is 1 or 2;

$L_2$ is alkoxy having 1—5 carbon atoms, alkylthio having 1—5 carbon atoms, phenoxy, phenylthio or halogen; and the salts thereof.

Typical examples for such compounds are 3-N-[2-[(5-dimethylaminomethyl-2-furanyl)methylthio]-ethyl]amino-4-ethoxy-1,2,5-thiadiazole-1-oxide and 3-N-[2-[(5-dimethylaminomethyl-2-furanyl)methylthio]ethyl]amino-4-ethoxy-1,2,5-thiadiazole-1,1-dioxide.

Further new intermediate products which are prepared in the course of the preparation of the inventive compounds are compounds having the following formulae:

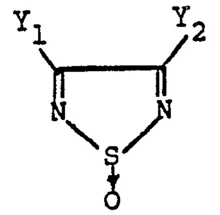

(XX)

wherein

$Y_1$ and $Y_2$ are members selected from the following groups:

a) $Y_1$ and $Y_2$ are the same and are alkoxy having 1—5 carbon atoms, alkylthio having 1—5 carbon atoms, phenoxy or phenylthio; or,

b) $Y_1$ and $Y_2$ are different and one of $Y_1$ and $Y_2$ is alkoxy having 1—5 carbon atoms, phenoxy or phenylthio and the other of $Y_1$ and $Y_2$ is a group having the formula:

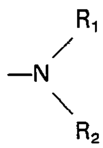

wherein $R_1$ and $R_2$ are independently as defined in formulae I, II, IIIA, IIIB, IVA and IVB or $R_1$ and $R_2$ form together with the nitrogen atom to which they are attached a group as defined in formulae I, II, IIIA, IIIB, IVA and IVB and

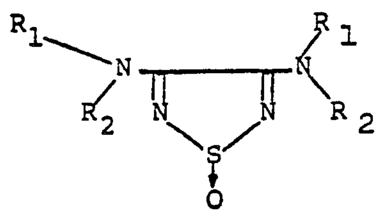

(XXI)

wherein $R_1$ and $R_2$ are independently as defined above or $R_1$ and $R_2$ form together with the nitrogen atom to which they are attached, a group as defined above and

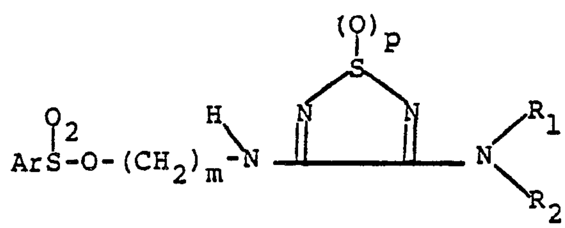

(XVII)

wherein

Ar is aryl and

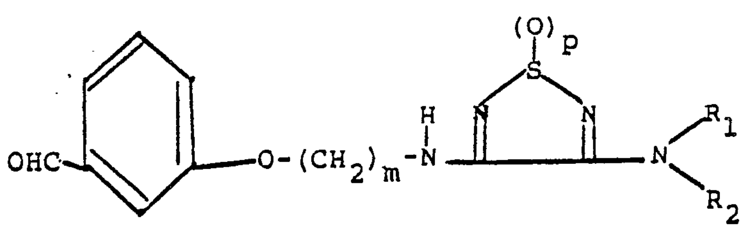

(XIX)

Typical examples for such compounds are:
3,4-diethoxy-1,2,5-thiadiazole-1-oxide;
3,4-bis-methylthio-1,2,5-thiadiazole-1-oxide;
3-isopropylamino-4-methylthio-1,2,5-thiadiazole-1-oxide;
3-ethoxy-4-amino-1,2,5-thiadiazole-1-oxide;
3-ethoxy-4-methylamino-1,2,5-thiadiazole-1-oxide;
3-ethoxy-4-(1-pyrrolidinyl)-1,2,5-thiadiazole-1-oxide;
3-ethoxy-4-(4-morpholine)-1,2,5-thiadiazole-1-oxide;
3,4-diamino-1,2,5-thiadiazole-1-oxide;
3-amino-4-(2-dimethylaminomethyl)amino-1,2,5-thiadiazole-1-oxide;
3-amino-4-methylamino-1,2,5-thiadiazole-1-oxide;
3-amino-4-dodecylamino-1,2,5-thiadiazole-1-oxide;
3-amino-4-(4-fluoranilino)-1,2,5-thiadiazole-1-oxide;
3-amino-4-morpholino-1,2,5-thiadiazole-1-oxide; and,
3-morpholino-4-(2-pyridinomethyl)amino-1,2,5-thiadiazole-1-oxide.

A further object of the invention is a process for the preparation of corresponding intermediate products which are used for preparing the inventive products. Said process comprises treating a compound having the formula:

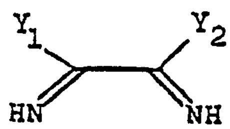

wherein $Y_1$ and $Y_2$ are as defined in a) of formula XX with thionyl chloride in the presence of a base to form a compound having the formula:

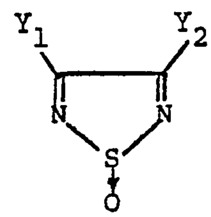

(XX)

which is treated with an amine having the formula:

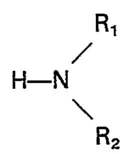

wherein $R_1$ and $R_2$ are as defined in b) of formula XX to yield the corresponding intermediate product of formula XX in which one of the groups $Y_1$ and $Y_2$ is alkoxy having 1—5 carbon atoms, alkylthio having 1—5 carbon atoms, phenoxy or phenylthio and the other of the group $Y_1$ and $Y_2$ is a group having the formula:

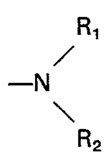

wherein $R_1$ and $R_2$ is as defined in b of formula XX or treating a compound having the formula

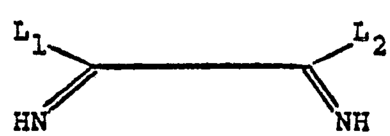

wherein

$L_1$ and $L_2$ are leaving groups selected from the group consisting of alkoxy having 1—5 carbon atoms, alkylthio having 1—5 carbon atoms, phenoxy or phenylthio, with thionyl chloride in the presence of a base to form an intermediate compound having the formula:

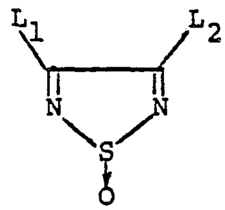

which is reacted with an amine of formula:

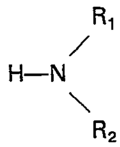

or sequentially with two different amines having said formula
wherein

$R_1$ and $R_2$ are independently as defined in formula XXI or

$R_1$ and $R_2$ form together with the nitrogen atom to which they are attached a group as defined in formula XXI to form compounds having the formula:

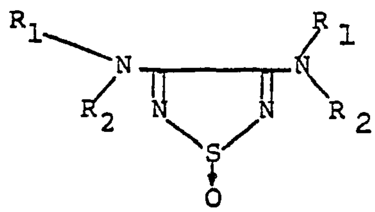

(XXI)

As it can be seen from the above explanations, the inventive compounds of formulae I and II are prepared by reacting a 1,2,5-thiadiazole-1-oxide or -1,1-dioxide substituted with the appropriate amino group and a leaving group, with an appropriately substituted amine.

The reactions performed for preparing the inventive products of formulae I, II, IIIA, IIIB, IVA and IVB and intermediate products are outlined in the following reaction schemes, wherein R, $R_1$, $R_2$, $R_3$, $R_4$, $R_5$, $R_6$, $R_7$, $R_8$, $R_9$, Ⓐ, X, k, m, n, and p are as defined above, unless indicated otherwise, and $L_1$ and $L_2$ denote leaving groups:

REACTION SCHEME I

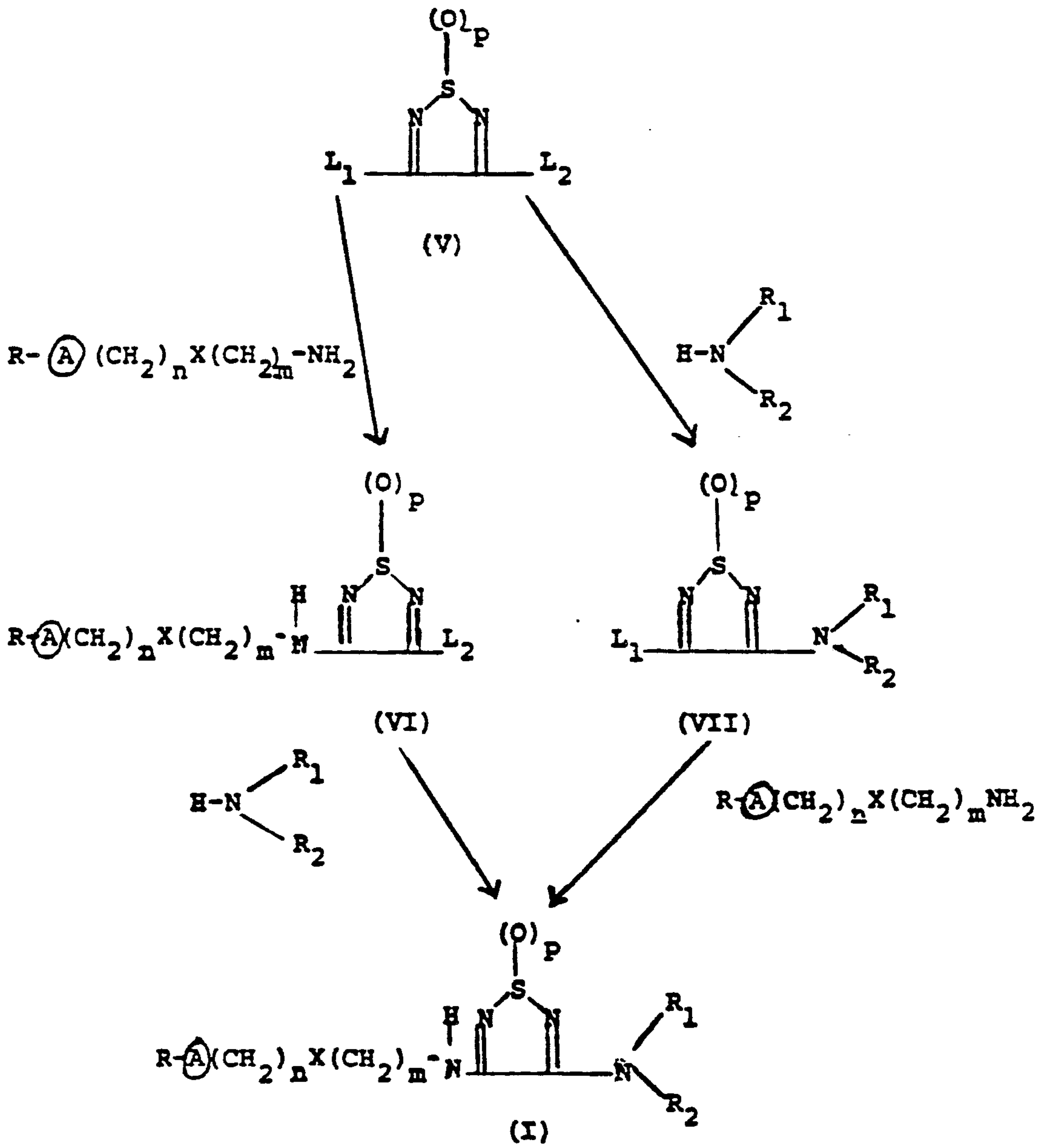

REACTION SCHEME II

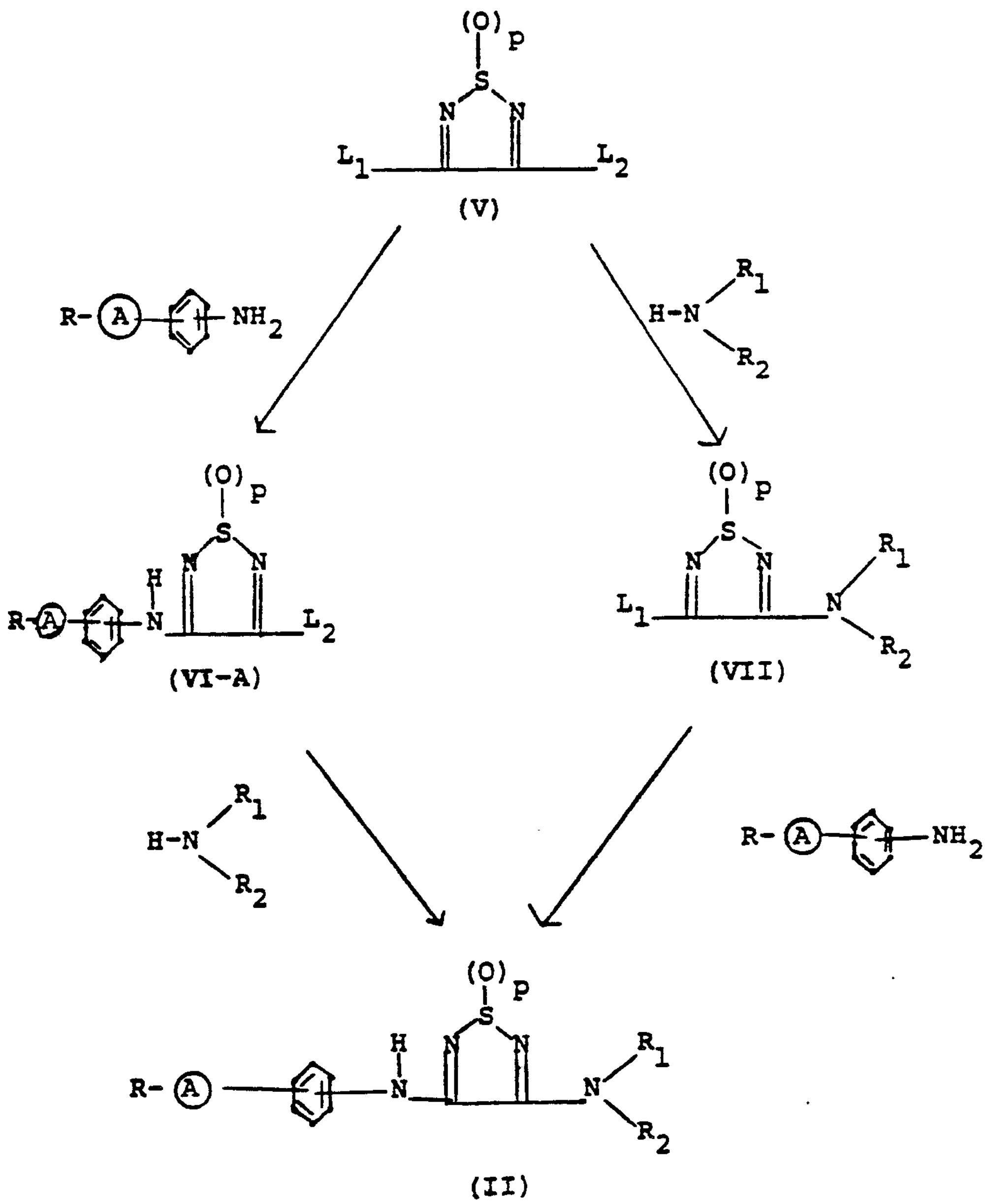

REACTION SCHEME III

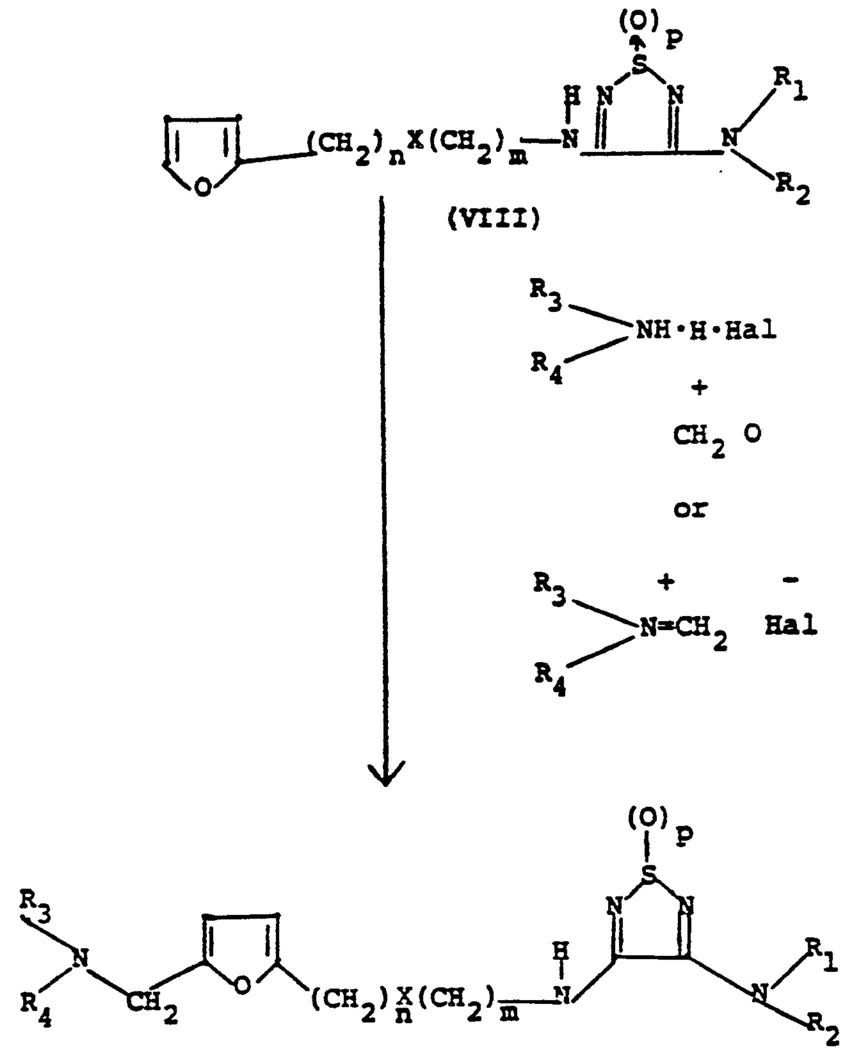


(VIII)

(I)

REACTION SCHEME IV

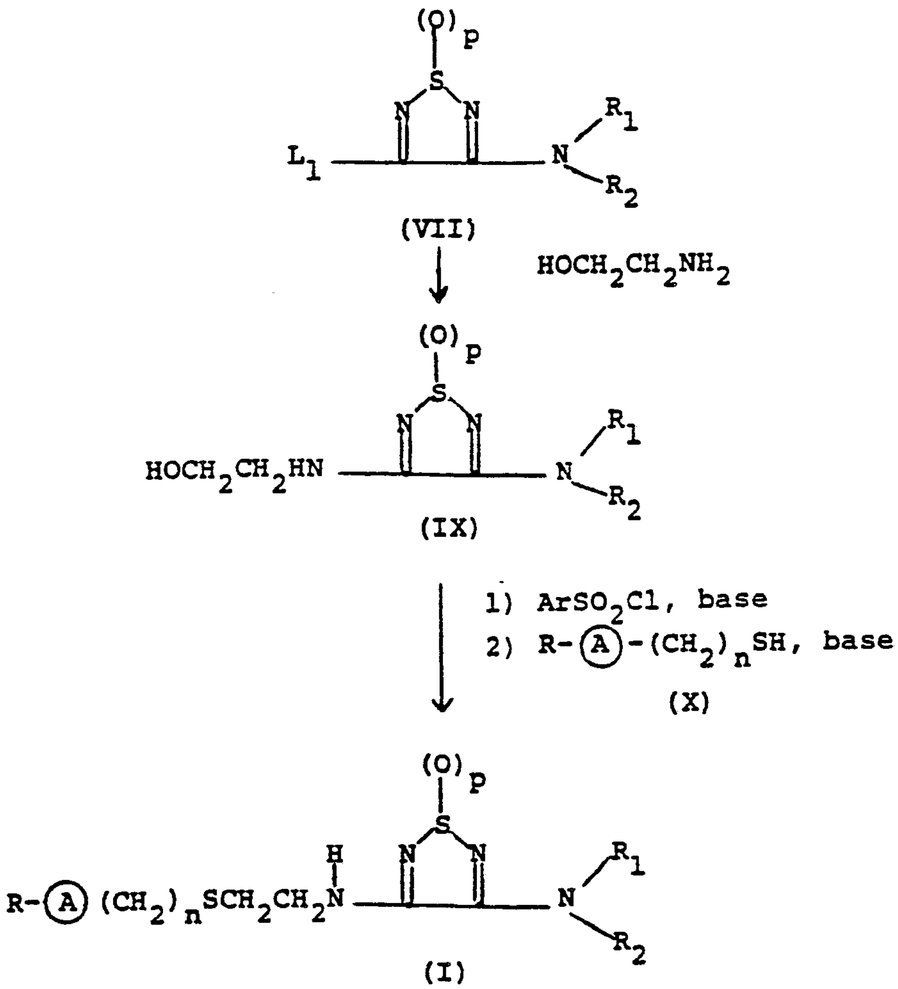


(I)

REACTION SCHEME V

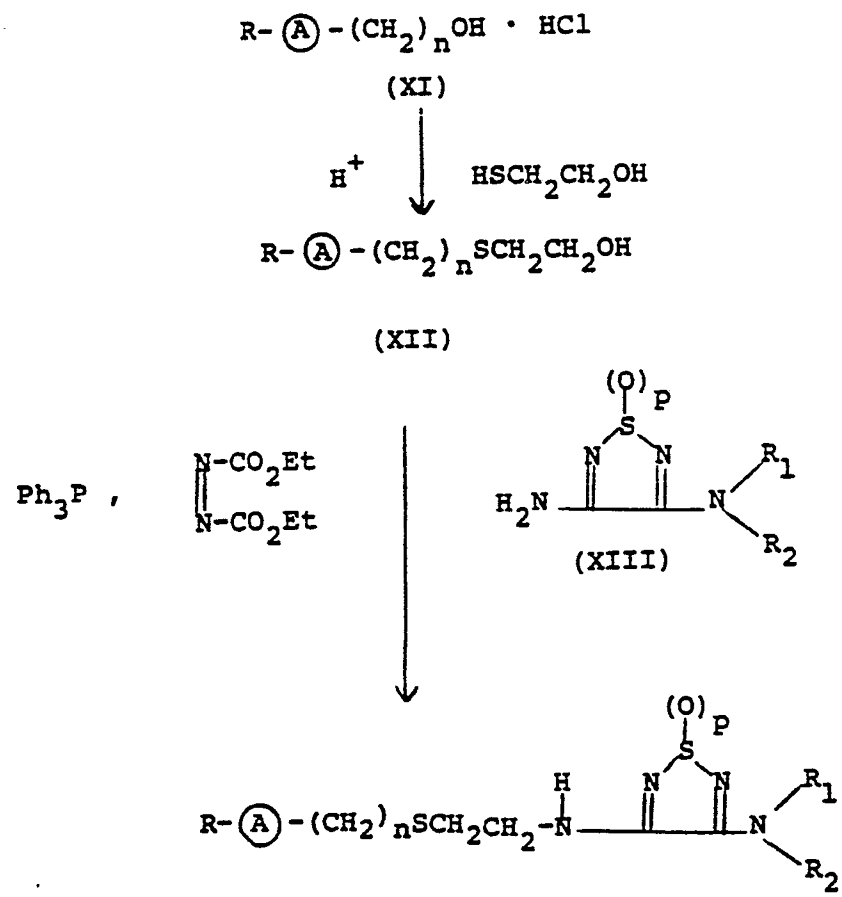


(XI)

(XII)

(XIII)

(I)

REACTION SCHEME VI

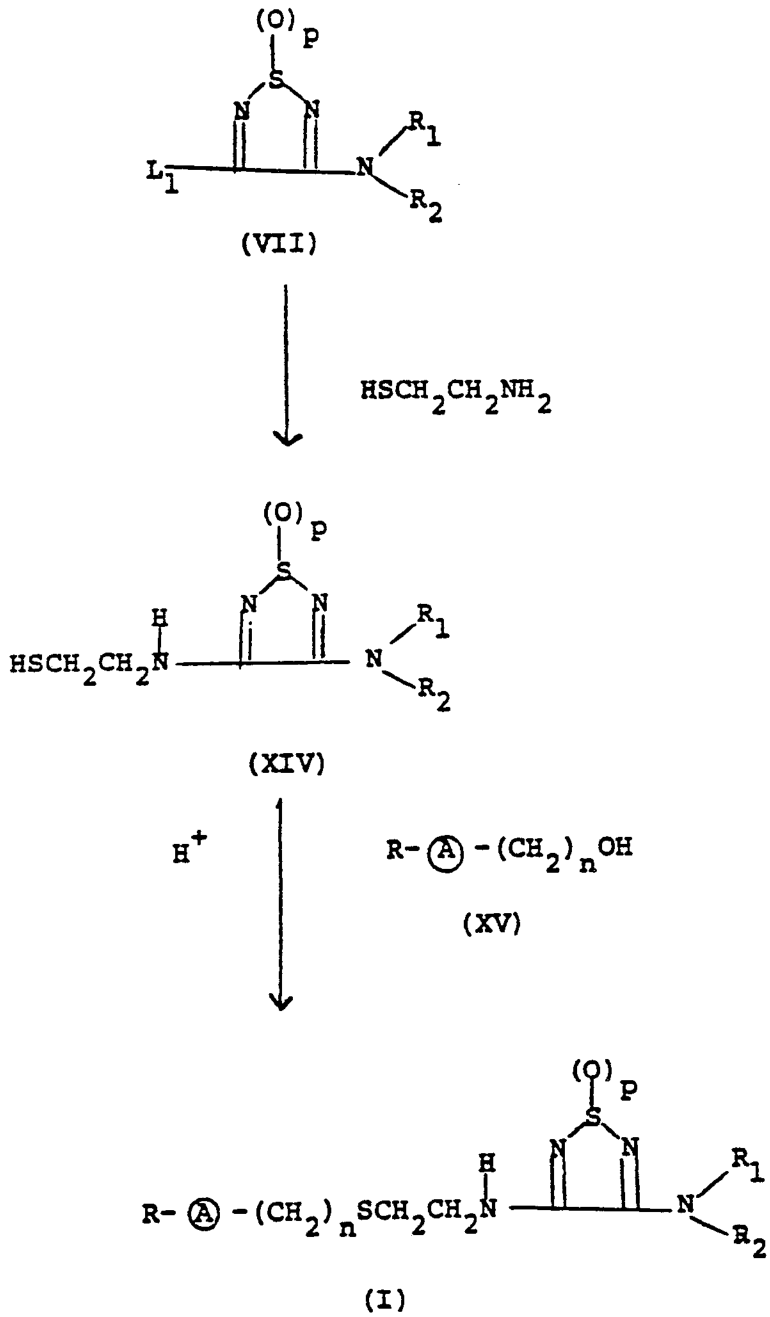


(VII)

(XIV)

(XV)

(I)

REACTION SCHEME VII

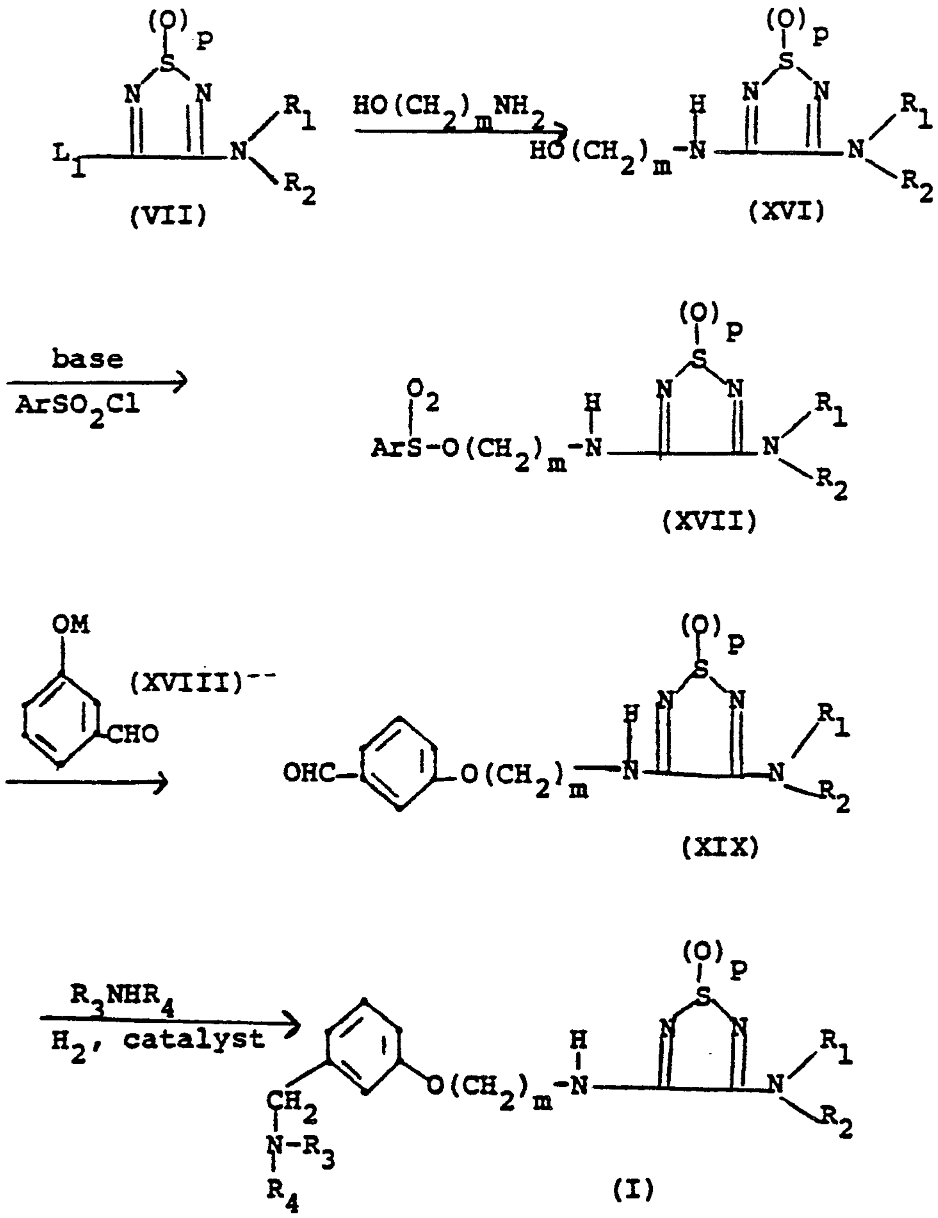


The appropriate leaving groups for these reactions are exemplified by loweralkoxy, loweralkylthio, phenoxy, phenylthio and halogen. Such loweralkoxy and loweralkylthio leaving groups are those such groups containing from 1 to 5 carbon atoms in either a straight or branched configuration. Examples of such groups are methoxy, ethoxy, propoxy, *iso*-propoxy, butoxy, *sec*-butoxy, pentoxy, methylthio, ethylthio, propylthio, isopropylthio, phenoxy and phenylthio. The term "halogen" includes the halogen atoms chlorine, fluorine, and bromine.

The preferred leaving groups are methoxy, ethoxy, methylthio, fluorine, chlorine and bromine.

As is noticed in Reaction Schemes I and II, the diaminothiadiazole compounds are prepared by first displacing one of the leaving groups of Compound III with an amine, either the simple or the complex amine, followed by displacement of the other leaving group by the other amine to produce the desired compounds. This reaction is carried out on the thiadiazole sulfoxide or sulfone.

In the first step of both Reaction Schemes I and II the reagents are combined in preferably an aprotic solvent, such as tetrahydrofuran, acetonitrile, such as tetrahydrofuran, acetonitrile, ethylacetate or ether, at from −78 to +50°C, preferably from −78 to 0°C. The process is a fast reaction and is completed quickly. Generally a gaseous amine is bubbled into the reaction mixture for sufficient time to complete the reaction, preferably 1 equivalent of the amine is added over a period of from 5 minutes to 2 hours. A solid or liquid amine is added in portions over the same period of time. The progress of the reaction may be followed by

taking aliquots of the reaction mixture and analyzing them on thin layer chromatography plates. When the reaction is complete, the reaction mixture is allowed to warm and the product is recovered using techniques known to those skilled in the art.

The reaction with the second amine is generally slower than with the first. The reactivity in each case is determined, however, by the particular thiadiazole substrate and the nature of the amine being reacted therewith.

The reaction with the second amine is carried out in protic or non-protic solvent such as methanol, ethanol, ether, ethylacetate or tetrahydrofuran. The reaction is generally carried out at from 0°C to 100°C and is complete in from 5 minutes to 6 days. If the desired reaction temperature is higher than the boiling point of the reaction mixture, the reaction may be carried out in a pressure vessel. Reactions with the thiadiazole sulfones are generally faster than those with the corresponding sulfoxide and as a result the reaction with sulfones are generally complete in shorter times and at lower temperatures than with the sulfoxides. The products are isolated using techniques known to those skilled in the art.

An alternate procedure for the reaction of the first amine with the starting material (V) consists of reacting the trialkylsilyl derivative or the *bis* (trialkylsilyl) derivative of the amine with Compound V. The reaction may be used with either the complex or the more simple amine, however, it is preferably employed with the simpler, and thus more reactive amines, and ammonia. In the case of ammonia as a reactant to produce the compounds wherein $R_1$ and $R_2$ are hydrogen, the preferred reactant is hexamethylsilazine, a commercially available reagent. This reagent is reacted with Compound V in an aprotic solvent at about room temperature for from 1 to 24 hours. The silyl groups present on the intermediate product are removed by treating such intermediate with water or an alcohol, at about room temperature. When Compound V is to be reacted with an amine other than ammonia, the appropriate reagent is prepared by treating the amine hydrohalide, preferably hydrochloride with an excess of the reagent such as hexamethylsilazane. The reaction is carried out without any solvent from room temperature to 100°C for from 1/2 to 24 hours. The thus produced trialkyl silyl or *bis* (trialkyl silyl) amine is then reacted with Compound V as described above and the product (VI, VI—A, or VII) is isolated using techniques known to those skilled in the art.

As shown in Reaction Scheme III, the preferred compounds of this invention wherein Ⓐ is furan and k is 1 in I are alternatively prepared by a Mannich type reaction on the appropriate furan intermediate VIII (I, R=H).

Compound VIII is allowed to react in an aqueous solution with an amine salt such as the hydrohalide and formaldehyde under conventional conditions utilized for the Mannich reaction. Alternatively, VIII can be reacted with the equivalent methylene ammonium reagent,

$$R_3{-}\overset{+}{N}(R_4){=}CH_2\ \overset{-}{Hal}$$

(wherein "Hal" is chlorine, bromine or iodine), in anhydrous media such as THF or acetonitrile with heating to reflux for 5—60 minutes to give product I.

An alternate process for preparing compound of formula I wherein X = S, m = 2, is shown in Reaction Scheme IV. Key intermediate alcohols IX are synthesized by displacement of a leaving group from intermediate VII with ethanolamine. The alcohol function of Compound IX can be converted to a leaving group (such as $L_1$) which can then be displaced by mercaptans X in the presence of a base to afford Compounds I.

Another process for preparing compounds of Formula I is shown in Reaction Scheme V wherein X = S, m = 2. Key intermediate alcohols XII are synthesized from alcohol XI and mercapto ethanol in the presence of an acid. Alcohols XII are than activated toward nucleophiles with phosphonium or similar reagents in the presence of diaminothiadiazole intermediates XIII forming Compounds I.

A further process for preparing compounds of formula I as shown in reaction scheme VI wherein X = S, m = 2, Key intermediate mercaptans XIV are synthesized from cysteamine and thiadiazole intermediates VII. The mercaptans XIV are then condensed with alcohols XV in the presence of an acid to provide compounds I.

In reaction scheme VII there is shown still another process for obtaining compounds of formula I wherein X = O, k = 1, m = 2 and n = 0. According to said reaction scheme the compound of formula XVI is first reacted with an acylating agent, which is in the reaction scheme of formula VII the compound $ArSO_2Cl$, to obtain the activated ester immediate of formula XVII. Thereafter said ester immediate is reacted with an alkali metal salt of the 3-hydroxy benzaldehyde having the formula XVIII to give the key intermediates XIX. Intermediates XIX can then be reduced in the presence of amine $R_3NHR_4$ to afford Compounds I.

Various intermediate compounds such as, for example, VI and VI-A, prepared from the thiadiazole starting material in which one of the leaving groups has been replaced by the complex amine such as, for example, 3-N-[2-[(5-dimethylaminomethyl-2-furanyl)methylthio]ethyl]amino-4-ethoxy-1,2,5-thiadiazole-1-oxide and 3-N-[2-[(5-dimethylaminomethyl-2-furanyl)methylthio]ethyl]amino-4-ethoxy-1,2,5-thiadiazde-1,1-dioxide have substantial gastric secretion inhibitory activity. Thus, such intermediate compounds are deemed to constitute part of this invention.

The thiadiazole starting materials wherein p = 2 with two leaving groups thereon are compounds generally known in the art. For example see Carmack *et al J. Org. Chem. 30* 2743 (1975).

The complex amines which are reacted with the thiadiazole starting material are also compounds known in the art. See for example the Durant *et al*, Price *et al* and Jones *et al* references discussed in the Background of the invention. It will be of course realized that other amine starting materials, similar to those discussed in the foregoing references, may be prepared using the procedures described in such references.

The thiadiazole starting materials wherein p is 1, with two leaving groups thereon are unknown compounds in the art. Such compounds are prepared by reacting an appropriately disubstituted oxalimidate or oxalthioimidate with thionyl chloride or prepare the thiadiazole compounds with two leaving groups.

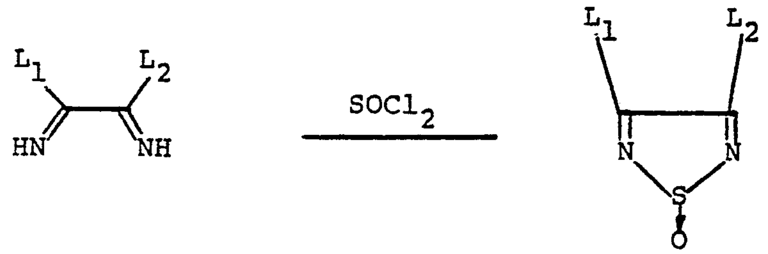


wherein $L_1$ and $L_2$ are as defined above.

The appropriately disubstituted oxalimidate or oxalthioimidate is reacted with thionyl chloride in the presence of a base to neutralize the hydrogen chloride liberated during the course of the reaction. The base may be any organic or inorganic base which does not react with any of the starting materials or products. Generally tertiary amines such as triethylamine and pyridine or inorganic bases such as alkali metal carbonates or bicarbonates are employed. The reactants are generally dissolved in a suitable solvent such as methylene chloride, chloroform, toluene, ether, tetrahydrofuran or acetonitrile, or any other solvent which is non-reactive to the starting materials and products. The reaction is carried out at from −78° (dry-ice bath) to 100°C or the reflux temperature of the reaction mixture. The reaction is preferably carried out at from 0° to room temperature and is then generally complete in from 1/2 to 10 hours. The thionyl chloride is generally used in slight excess although greater amounts of the reagent are not detrimental.

Thus, the intermediate compounds obtained from these novel thiadiazole starting materials are also novel when p is 1 and can be represented by the following formulae:

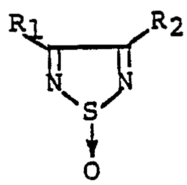

XXII

wherein

a) $R_1$ and $R_2$ are the same and are alkoxy having 1—5 carbon atoms, alkylthio having 1—5 carbon atoms, phenoxy or phenylthio or

b) $R_1$ and $R_2$ are different and one of $R_1$ or $R_2$ is amino or alkylamino having 1—5 carbon atoms and the other is halo or alkylthio having 1—5 carbon atoms or

c) $R_1$ and $R_2$ are different and one of the $R_1$ and $R_2$ is as defined in a) above and the other is a group of formula:

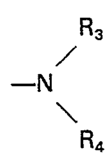

wherein

$R_3$ and $R_4$ are independently hydrogen, alkyl having 1—5 carbon atoms, alkenyl having 3—5 carbon atoms, alkynyl having 3—5 carbon atoms, cycloalkyl having 3—7 carbon atoms, phenyl or substituted alkyl having 1—5 carbon atoms in the alkyl group, wherein the substituent is phenyl, cycloalkyl having 3—7 carbon atoms, pyridyl, imidazolyl, morpholino, hydroxy, alkoxy having 1—5 carbon atoms, alkylthio having 1—5 carbon atoms or dialkylamino wherein each alkyl group has 1—5 carbon atoms or $R_3$ and $R_4$ form together with the nitrogen atom to which they are attached a 5- or 6-membered heterocyclic ring which may also contain an oxygen, sulfur or N-$R_7$ heteroatom wherein $R_7$ is hydrogen or alkyl having 1—3 carbon atoms and which heterocyclic ring is preferably selected from morpholine, thiomorpholine, piperidine, piperazine, N-alkyl piperazine wherein the N-alkyl group has 1—3 carbon atoms; and

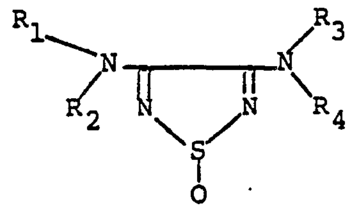

(XXIII)

wherein

$R_1$, $R_2$, $R_3$ and $R_4$ can each independently be hydrogen, alkyl having 1—5 carbon atoms, alkenyl having 3—5 carbon atoms, alkynyl having 3—5 carbon atoms, cycloalkyl having 3—7 carbon atoms, phenyl, pyridyl or substituted alkyl having 1—5 carbon atoms in the alkyl group, wherein the substituent is amino, phenyl, substituted phenyl, cycloalkyl having 3—7 carbon atoms, pyridyl, alkoxy having 1—5 carbon atoms, morpholino, hydroxy, di(alkyl)amino wherein each alkyl group has 1—5 carbon atoms or

$R_3$ and $R_4$ form together with the nitrogen atom to which they are attached, a 5- or 6-membered heterocyclic ring which may also contain an oxygen, sulfur or an N—$R_7$ heteroatom, wherein $R_7$ is hydrogen or alkyl having 1—3 carbon atoms and wherein said heterocyclic ring is morpholine, thiomorpholine, piperidine, piperazine, N-alkyl piperazine wherein the N-alkyl group has 1—3 carbon atoms, and pyrrolidine.

The following examples are provided in order that the invention might be more fully understood. Unless otherwise indicated, all temperatures are in degrees Celsius.

## Example 1

### 3-N-[2-[(5-Dimethylaminomethyl-2-furanyl)methylthiolethyl]amino-4-methylamino-1,2,5-thiadiazole-1-oxide hydrochloride

A. 3-Ethoxy-4-methylamino-1,2,5-thiadiazole-1-oxide

Anhydrous methylamine (7.7 g, 0.25 mol) is dissolved in 100 ml of tetrahydrofuran with dry ice-acetone cooling. This cold solution is added dropwise with stirring to 3,4-diethoxy-1,2,5-thiadiazole-1-oxide in 250 ml of tetrahydrofuran at −15°C over 1 1/2 hours. Stirring is continued for an additional hour at −15°C and the solvent is removed *in vacuo*. The beige solid residue is purified by chromatography on silica gel (100 g) using chloroform. The nearly white solid (39.6 g) melts at 91—92°C.

IR Stretching vibrations = 1095 $cm^{-1}$ for S=O.

$^{13}C$ NMR for thiadiazole-1-oxide carbons: δ = 163.34, 157.36.

B. 3-N-[2-[(5-Dimethylaminomethyl-2-furanyl)methylthio]-ethyl]amino-4-methylamino-1,2,5-thiadiazole-1-oxide hydrochloride

To a stirred solution of 1.75 g (10.0 mmol) of 3-ethoxy-4-methylamino-1,2,5-thiadiazole-1-oxide in 25 ml$CH_2CL_2$ is added 2.14 g (10.0 mmol) of 2-[(5-dimethylaminomethyl-2-furanyl)methylthio]ethylamine. The mixture is stirred three days at room temperature under nitrogen and concentrated under vacuum. The residue is redissolved in the minimum amount of methylene chloride and the solution is chromatographed on a dry column of activity III alumina. Elution with 1—2% methanol—$CH_2Cl_2$ gives the free base of the title compound which is dissolved in 1 equivalent 1N aqueous hydrochloric acid and the solution is filtered through filter aid and the filtrate lyophilized. The residue is dissolved in a mixture of 4 parts of tetrahydrofuran and 1 part absolute ethanol and cooled to give a hygroscopic white solid which is filtered and dried *in vacuo* at 60°C to give 3-N-[2-[(5-dimethylaminomethyl-2-furanyl)methylthio]ethyl]amino-4-methylamino-1,2,5-thiadiazole-1-oxide hydrochloride as the tetrahydrofuran solvate (amorphous). The sample exhibits the correct nuclear magnetic resonance spectrum ($D_2O$) and has an infrared stretching vibration of (KBr) of 1050 $cm^{-1}$ for S=O.

## Example 2

### 3-N-[2-[(5-Dimethylaminomethyl-2-furanyl)methylthio]ethyl]amino-4-methylamino-1,2,5-thiadiazole-1,1-dioxide hydrochloride

A. 3-Ethoxy-4-methylamino-1,2,5-thiadiazole-1,1-dioxide

To a solution of 2.06 g (10.0 mmol) of 3,4-diethoxy-1,2,5-thiadiazole-1,1-dioxide in 25 ml of acacetonitrile is added 0.8 g of a 40% solution of methylamine in water. The resulting clear colorless solution is aged at room temperature for 16 hours and concentrated under vacuum. The residue is stirred with 25 ml of methylene chloride and the mixture is filtered to give 0.35 g of a mixture of the title compound and 3,4-bis-methylamino-1,2,5-thiadiazole-1,1-dioxide. The filtrate is chromatographed on activity III alumina and the title compound eluted with 29% methanol methylene chloride to give 0.65 g (34%) of pure 3-ethoxy-4-methylamino-1,2,5-thiadiazole-1,1-dioxide, m.p. 160—161°.

B. 3-N-[2-[(5-Dimethylaminomethyl-2-furanyl)methylthio]ethyl]amino-4-methylamino-1,2,5-thiadiazole-1,1-dioxide hydrochloride hydrate

To a stirred suspension of 3-ethoxy-4-methylamino-1,2,5-thiadiazole-1,1-dioxide (0.65 g, 3.4 mmol) in 10 ml of methylene chloride is added 0.73 g (3.4 mmol) of 2-[(5-dimethylaminomethyl-2-furanyl)

methylthio]ethylamine. The suspension is stirred under nitrogen at room temperature and becomes clear after 2.5 hours. After 1.5 days the mixture is concentrated under vacuum and the residue chromatographed on activity III alumina as in Example 2A. The free base of the title compound is dissolved in 1 equivalent of 1N hydrochloric acid and the solution is filtered through filter aid and lyophilized to give 3-N-[2-[(5-dimethylaminomethyl-2-furanyl)methylthio]ethyl]amino-4-methylamino-1,2,5-thiadiazole 1, 1-dioxide hydrochloride as the hydrate which is dried *in vacuo* at 60°C (amorphous). The hygroscopic solid exhibits the correct nuclear magnetic resonance spectrum ($D_2O$) and has an infrared stretching vibration (KBr) of 1155 and 1305 $cm^{-1}$ for $SO_2$.

Example 3

3-N-[2-[(2-Guanidino-4-thiazolyl)methylthio]ethyl]amino-4-methylamino-1,2,5-thiadiazole-1-oxide

To a stirred solution of 3.3 g (14 mmol) of 2-[(2-guanidino-4-thiazolyl)methylthio]ethyl amine in 25 ml of ethylacetate and 5 ml of absolute ethanol is added 2.5 g (14 mmol) 3-ethoxy-4-methylamino-1,2,5-thiadiazole-1-oxide. The resulting suspension is stirred for 16 hours at room temperature under nitrogen during which the gum gradually solidifies to a white solid which is collected by filtration to give 5.1 g (100%) of crude title compound. Recrystallization from water containing a little methanol gives a white solid, m.p. 169—171°C (dec) identified as 3-N-[2-[(2-guanidino-4-thiazolyl)methylthio]ethyl]amino-4-methylamino-1,2,5-thiadiazole-1-oxide which exhibits the correct nuclear magnetic resonance spectrum ($D_2O$,DCl) and an infrared stretching vibration (KBr) of 1030 $cm^{-1}$ for S—O.

Example 4

3-N-[2-[(5-Dimethylaminomethyl-2-furanyl)methylthio]ethyl]amino-4-dimethylamino-1,2,5-thiadiazole-1-oxide

A. 3-N-[2-[(5-Dimethylaminomethyl-2-furanyl)methylthio]-ethyl]amino-4-ethoxy-1,2,5-thiadiazole-1-oxide

3,4-Diethoxy-1,2,5-thiadiazole-1-oxide (1.9 g, 10 mmol) is dissolved in 10 ml of tetrahydrofuran and a solution of 2-[(5-dimethylaminomethyl-2-furanyl)methylthio]ethylamine (2.14 g, 10 mmol) in 10 ml of tetrahydrofuran is added dropwise with stirring at ice bath temperature. The solution is stirred at room temperature overnight. Removal of the solvent *in vacuo* gives a viscous amber oil. Chromatography on silica gel (50 g) using 5% ethanol in chloroform gives 3.6 g of 3-N-[2-[(5-dimethylaminomethyl-2-furanyl)-methylthio]ethyl]amino-4-ethoxy-1,2,5-thiadiazole-1-oxide as a viscous oil. Analysis and $^1H$ nuclear magnetic resonance confirm the presence of 1/2 mole of chloroform.

B. 3-N-[2-[(5-Dimethylaminomethyl-2-furanyl)methylthio]-ethyl]amino-4-dimethylamino-1,2,5-thiadiazole-1-oxide

3 - N - [2 - [(5 - dimethylaminomethyl - 2 - furanyl)methylthio]ethyl]amino - 4 - ethoxy - 1,2,5 - thiadiazole - 1 - oxide (3.05 g, 8.5 mmol) is dissolved in 10 ml of tetahydrofuran and dimethylamine (0.38 g, 8.5 mmol) is bubbled into the solution at ice bath temperature. The reaction is stirred in a sealed flask at room temperature overnight. An excess of dimethylamine is then added to force the reaction to completion. Removal of the solvent *in vacuo* gives 2.7 g of viscous amber oil which is purified by chromatography on silica gel. The chromatographed oil solidifies to a pale beige solid upon extended standing and trituration in petroleum ether, m.p. 55—58°C. It recrystallizes from n-butyl chloride affording 3 - N - [2 - [(5 - dimethylaminomethyl - 2 - furanyl)methylthio]ethyl]amino - 4 - dimethylamino - 1,2,5 - thiadiazole - 1 - oxide.

Example 5

3-N-[3-(2-Guanidino-4-thiazolyl)phenyl]amino-4-methylamino-1,2,5-thiadiazole-1-oxide

A. 3-N-[3-(2-Guanidino-4-thiazolyl)phenyl]amino-4-methoxy-1,2,5-thiadiazole-1-oxide

A solution of 2.33 g (0.010 mol) of 3-(2-guanidino-4-thiazolyl)benzeneamine in 50 ml of methanol is added to a suspension of 1.94 g (0.010 mol) of 3,4-bis(methylthio)-1,2,5-thiadiazole-1-oxide in 20 ml of methanol with stirring. All solids dissolve and the solution is stirred for 120 hours. No change in the reaction mixture is noted by thin layer chromatography during the last 24 hours. The reaction mixture is filtered to give 0.71 g of orange solids which gives only an origin spot on thin layer chromatography. The filtrate is evaporated to dryness under vacuum to give 3.30 g of yellow solid. Nuclear magnetic resonance of the material shows the presence of the product. The material is purified on a column of activity III neutral alumina. Elution with chloroform, followed by 1% methanol/chloroform and 5% methanol/chloroform gives thiadiazole starting material. Elution with 10%, 20% and 50% methanol/chloroform gives 2.32 g (14%) of yellow solid containing product. Recrystallization from acetonitrile gives 1.20 g; m.p. 217—221° (d) of 3-N-[3-(2-guanidino-4-thiazolylphenyl]amino-4-methoxy-1,2,5-thiadiazole-1-oxide; proton nuclear magnetic resonance is consistent with this structure.

It is noted that during the course of this reaction the solvent (methanol) exchanged with the residual methylthio group forming the 4-methoxy compound. This does not affect the reaction, since the second amine can react with either the 4-methoxy compound or the 4-methylthio compound.

B. 3-N-[3-(2-Guanidino-4-thiazolyl)phenyl]amino-4-methylamino-1,2,5-thiadiazole-1-oxide

0.37 g of a 33% methylamine/methanol solution (0.12, 0.0040 mol) is added to a suspension of 1.27 g (0.0035 mol) 3-N-[3-(2-guanidino-4-thiazolylphenyl]-amino-4-methoxy-1,2,5-thiadiazole-1-oxide in 30 ml of

methanol with stirring. The solids dissolve and after 30 minutes a precipitate begins to form. The reaction is complete within 2 hours by thin layer chromatography and the precipitate is filtered and dried to give 0.76 g (70%) of crude product. The product is dissolved in 5 ml of dimethyl sulfoxide and filtered. Methanol is added to the solution to give a precipitate which is filtered and dried to give 0.97 g; m.p. 160—161°C(d) of product; proton nuclear magnetic resonance is consistent with structure of 3-N-[3-(2-guanidino-4-thiazolyl)phenyl]amino-4-methylamino-1,2,5-thiadiazole-1-oxide.

### Example 6

3-N-[3-(2-Guanidino-4-thiazolyl)phenyl]amino-4-methylamino-1,2,5-thiadiazole-1,1-dioxide

A. 3-N-[3-(2-guanidino-4-thiazolyl)phenyl]amino-4-ethoxy-1,2,5-thiadiazole-1,1-dioxide

A solution of 1.17 g (0.005 mol) of 3-(2-guanidino-4-thiazolyl)benzeneamine in 30 ml of ethanol is added to a slurry of 1.03 g (0.005 mol) of 3,4-diethoxy-1,2,5-thiadiazole-1,1-dioxide in 30 ml of ethanol with stirring. All solids dissolve within one minute and a precipitate begins to form within two minutes. The reaction mixture is stirred for 5 hours, at which time the reaction is complete by thin layer chromatography. The precipitate is filtered, dried and recrystallized from ethanol to give 1.35 g of 3-N-[3-(2-guanidino-4-thiazolyl)phenyl]amino-4-ethoxy-1,2,5-thiadiazole-1,1-dioxide m.p. 237—239°C.

B. 3-N-[3-(2-Guanidino-4-thiazolyl)phenyl]amino-4-methylamino-1,2,5-thiadiazole-1,1-dioxide

0.70 g of a 33% methylamine/methanol solution (0.23 g, 0.0075 mol) is added to a suspension of 2.91 g (0.0075 mol) of 3-N-[3-(2-guanidino-5-thiazolyl)phenyl]amino-4-ethoxy-1,2,5-thiadiazole-1,1-dioxide from Part A of this example in 75 ml of methanol. The mixture is stirred at room temperature for 3 hours, at which time the reaction is complete by thin layer chromatography. The reaction mixture is filtered to give 1.30 g of yellow solid which gives only an origin spot on thin layer chromatography. The filtrate is evaporated to dryness under vacuum to give 2.00 g (70%) of a yellow solid. Recrystallization from water gives 0.72 gm.p. 263—265°C; proton nuclear magnetic resonance is consistent with the structure of 3-N-[3-(2-guanidino-4-thiazolyl)phenyl]amino-4-methylamino-1,2,5-thiadiazole-1,1-dioxide.

### Example 7

3-N-[2-[(5-Dimethylaminomethyl-2-furanyl)methylthio]ethyl]amino-4-(2-propynyl)amino-1,2,5-thiadiazole-1-oxide

3-N-[2-[(5-Dimethylaminomethyl-2-furanyl)-methylthio]ethyl]amino-4-ethoxy-1,2,5-thiadiazole-1-oxide (5.37 g, 0.015 mol) is dissolved in 30 ml of tetrahydrofuran and solution of 2-propynylamine (0.91 g, 0.0165 mol) in 5 ml of tetrahydrofuran is added dropwise over several minutes. The reaction mixture is stirred at room temperature for 72 hours. Removal of the solvent *in vacuo* gives a dark amber oil. Chromatography on silica gels using 5% methanol/chloroform gives 3.2 g of a light amber oil. Proton nuclear magnetic resonance is consistent with the proposed structure.

### Example 8

3-N-[2-[(5-Dimethylaminomethyl-2-furanyl)methylthio]ethyl]amino-4-amino-1,2,5-thiadiazole-1-oxide

3-N-[2-[(5-Dimethylaminomethyl-2-furanyl)methylthio]ethyl]amino-4-ethoxy-1,2,5-thiadiazole-1-oxide (5.37 g, 0.015 mol) is dissolved in 10 ml of tetrahydrofuran and a solution of anhydrous ammonia (1.7 g, 0.10 mol) dissolved in 10 ml of cold tetrahydrofuran is added at ice bath temperature. The reaction is stirred at ice bath temperature for several hours and then at room temperature overnight. Removal of the solvent *in vacuo* gives a viscous oil which solidifies upon trituration in petroleum ether affording 4.8 g of a pale beige solid. Recrystallization from nitromethane gives 2.7 g of a pale tan solid m.p. dec. 132—136°C.

### Example 9

3-N-[2-[(2-Guanidino-4-thiazolyl)methylthio]ethyl]amino-4-methylamino-1,2,5-thiadiazole-1,1-dioxide

In a procedure similar to Example 3 using 1.16 g (5.0 mmol) of the amine and 0.96 g (5.0 mmol) of 3-ethoxy-4-methylamino-1,2,5-thiadiazole-1,1-dioxide, there is obtained 0.65 g of 3-N-[2-(2-guanidino-4-thiazolyl)methylthio]ethylamino-4-methylamino-1,2,5-thiadiazole-1,1-dioxide, .m.p. 182—184°, dec. after recrystallization from water; IR—$SO_2$ stretching vibrations 1350, 1150 $cm^{-1}$ (KBr); proton magnetic resonance is consistent with structure.

### Example 10

3-N-[3-[3-(Dimethylaminomethyl)-phenoxy]propyl]amino-4-amino-1,2,5-thiadiazole-1-oxide

To a solution of 3-[3-(dimethylaminomethyl)phenoxy]propylamine (2.9 gm, 0.0139 mol) in acetonitrile (9 ml) is added 3-ethoxy-4-amino-1,2,5-thiadiazole-1-oxide (2.25 gm, 0.014 mol). Complete dissolution occurs almost immediately and product begins to precipitate after 1/4 hour. The reaction is stirred at ambient temperature for about 16 hours and pure product is collected by filtration. The white solid is washed with acetonitrile, ether and air dried to give 4.35 gm of 3-N-[3-[3-dimethylaminomethyl)phenoxy]-propyl]amino-4-amino-1,2,5-thiadiazole-1-oxide m.p. 159—162°C.

Example 11

3-N[2-[[3--(Dimethylaminomethyl)phenyl]methylthio]ethyl]amino-4-amino-1,2,5-thiadiazole-1-oxide

To a solution of 2-[[3-(dimethylaminomethyl)phenyl]methylthio]ethylamine (3.36 gm, 0.015 mol) in acetonitrile (10 ml) at room temperature is added 3-ethoxy-4-amino-1,2,5-thiadiazole-1-oxide (2.42 gm, 0.015 mol). Complete dissolution occurs within 15 minutes and product begins to precipitate after 1/2 hour. The reaction is stirred at ambient temperature for about 16 hours and the product collected by filtration, washed with acetonitrile, ether and air dried to give 3.7 gm of crude product. Crystallization from acetonitrile gives pure 3-N-[2-[[3-(dimethylamino-methyl)phenyl]methylthio]ethyl]amino-4-amino-1,2,5-thiadiazole-1-oxide as tan rosettes, m.p. 120—123°C.

Example 12

3-N-[3-[3-(Dimethylaminomethyl)phenoxy]propyl]amino-4-methylamino-1,2,5-thiadiazole-1-oxide hydrochloride

3-[3-(Dimethylaminomethyl)phenoxy]propylamine (1.716 g, 0.00823M) dissolved in THF (7 ml) is added to 3-ethoxy-4-methylamino-1,2,5-thiadiazole-1-oxide (1.443 g, 0.00823M) suspended in THF (7 ml). The resultant solution is stirred at ambient temperature for 18 hours and concentrated under vacuum. The residue is chromatographed on silica and eluted with 10% methanol-chloroform to obtain the product gum (2.5 g). The product is dissolved in ethanol (10 ml), acidified with 6N ethanolic HCl, precipitated with diethyl ether, filtered and dried under vacuum at 55°C to obtain the 3-N-[3-[3-(Dimethylaminomethyl)phenoxy]-propyl]amino-4-methylamino-1,2,5-thiadiazole-1-oxide hydrochloride m.p. 95°(d) solvated with diethyl ether.

Example 13

3-N-[3-[3-(1-piperdinylmethyl)phenoxy]propyl]amino-4-methylamino-1,2,5-thiadiazole-1-oxide hydrochloride

3-[3-(1-Piperidinylmethyl)phenoxy]propaneamine (2.48 g, 0.01M) in THF (4 ml) is reacted with 3-ethoxy-4-methylamino-1,2,5-thiadiazole-1-oxide (1.87 g, 0.0107M) in THF (35 ml) by the method of Example 12 affording 3-N-[3-[3-(-piperidinylmethyl)phenoxy]propyl]amino-4-methylamino-1,2,5-thiadiazole-1-oxide hydrochloride as a non-crystalline solid solvated with ethyl alcohol.

Example 14

3-N-[3-[3-[(4-Methyl-1-piperazinyl)methyl]phenoxy]propyl]amino-4-methylamino-1,2,5-thiadiazole-1-oxide Hydrochloride

A. 3-[3-[(4-Methyl-1-piperazinyl)methyl]phenoxy]propaneamine

To 2-[3-[3-formylphenoxy]propyl]-1H-isoindole-1,3-dione (20 g, 0.0647M) suspended in ethylacetate (50 ml) 1-methylpiperazine (19.53 g, 0.195M) is added and the mixture stirred at ambient temperature 45 minutes longer than the time required to effect solution. Acetic acid (23.1 g, 0.385M) in a mixture of ethylacetate (40 ml) and ethanol (80 ml) is added along with 10% palladium on carbon (3 g). The mixture is hydrogenated 3,4475.$10^5$ Pa (50 lbs/sq. in.), filtered and the filtrate concentrated under vacuum. The residue is dissolved in dilute hydrochloric acid and extracted with methylene chloride. The aqueous phase is basified with solid sodium carbonate and extracted with methylene chloride. The organic extracts are dried over sodium sulfate and concentrated under vacuum. The residual oil (21 g) is stirred in a mixture of ethanol (250 ml) and 64% hydrazine (100 ml) for 90 hours at ambient temperature. The mixture is diluted with diethylether, filtered and the filtrate concentrated under vacuum and the residue distilled. The product is obtained by collecting the fraction distilling at 215—23°/1,9998.$10^2$ Pa (1.5mm Hg) weight 9.7 g.

B. 3-N-[3-[3-[(4-Methyl-1-piperazinyl)methyl]phenoxy]-propyl]amino-4-methylamino-1,2,5-thiadiazole-1-oxide Hydrochloride

By reacting 3-[3-[(4-methyl-1-piperazinyl)methyl]phenoxy]propaneamine (3.1 g, 0.0118M) in THF (3 ml) with 3-ethoxy-4-methylamino-1,2,5-thiadiazole-1-oxide as described in Example 12, 3-N-[3-[3-[(4-methyl-1-piperazinyl)methyl]phenoxy]propyl]amino-4-methylamino-1,2,5-thiadiazole-1-oxide hydrochloride is obtained; m.p. 78° with decomposition.

Example 15

3-N-[3-[3-[(4-Morpholinomethyl)phenoxy]propyl]amino-4-methylamino-1,2,5-thiadiazole-1-oxide Hydrochloride

A. 3-[3-(4-Morpholinomethyl)phenoxy]propaneamine

By reacting 2-[3-[3-formylphenoxy]propyl]-1H-isoindole-1,3-dione (20 g, 0.0647M) with morpholine (16.9 g, 0.194M) under the conditions described in Example 14A, and isolating the product in a similar manner there is obtained 11.6 g of 3-[3-(4-morpholinomethyl)phenoxy]propaneamine b.p. 188—98 at 1,9998·$10_2$ Pa (1,5 mm Hg).

B. 3-N-[3-(4-Morpholinomethyl)phenoxy]propyl]amino-4-methylamino-1,2,5-thiadiazole-1-oxide hydrochloride

By reacting 3-[3-(4-morpholinomethyl)phenoxy]propaneamine (3.0 g, 0.012M) in THF (4 ml) with 3-ethoxy-4-methylamino-1,2,5-thiadiazole-1-oxide (2.27 g, 0.0129M) in THF (4 ml) as described in Example 12,

3 - N - [3 - (4 - morpholinomethyl)phenoxy]propyl]amino - 4 - methylamino - 1,2,5 - thiadiazole - 1 - oxide hydrochloride is obtained; m.p. 90—95°C with decomposition.

Example 16

3-N-[3-[3-(4-Morpholinomethyl)phenoxy]propyl]amino-4-amino-1,2,5-thiadiazole-1-oxide

3-[3-(4-Morpholinomethyl)phenoxy]propaneamine (2.858 g, 0.01142M) in acetonitrile (4 ml) is reacted with 3-ethoxy-4-amino-1,2,5-thiadiazole-1-oxide (1.933 g, 0.012M) suspended in acetonitrile (5 ml). After stirring 20 hours at ambient temperature the crystalline product is filtered, washed with acetonitrile, and dried under vacuum at 65° affording 3-N-[3-[3-(4-morpholinomethyl)phenoxy]propyl]amino-4-amino-1,2,5-thiadiazole-1-oxide; m.p. 185—186.5° with decomposition.

Example 17

3-N-[2-[(4-Methyl-5-imidazolyl)methylthio]ethyl]amino-4-methylamino-1,2,5-thiadiazole-1-oxide hydrochloride

2-[(4-Methyl-5-imidazolyl)methylthio]ethylamine (1.838 g, 0.01073M) dissolved in a mixture of tetrahydrofuran and methylene chloride (3 ml is added to 3-ethoxy-4-methylamino-1,2,5-thiadiazole-1-oxide (1.8804 g, 0.01073M) and stirred at ambient temperature for 20 hours. The reaction mixture is concentrated under vacuum, the residue is dissolved in isopropanol (10 ml) acidified with ethanolic hydrochloride and diluted with diethyl ether. The crude product is filtered, redissolved in isopropanol (15 ml) and this solution diluted to 500 ml with acetonitrile. The mixture is filtered, the solid discarded, the filtrate concentrated in vacuum. The residue (2.6 g) is dissolved in isopropanol (12 ml) and the product precipitated with diethyl ether affording 3-N-[2-[(4-methyl-5-imidazolyl)methylthio]ethyl]amino-4-methylamino-1,2,5-thiadiazole-1-oxide hydrochloride m.p. 118°(d).

Example 18

3-N-[2-[(5-Dimethylaminomethyl-2-furanyl)methylthio]ethyl]amino-4-amino-1,2,5-thiadiazole-1,1-dioxide

To a filtered solution of 2-[(5-dimethylaminomethyl-2-furanyl)methylthio]ethyl amine (8.35 g, 39.0 mmol) in 70 ml methanol is added 6.9 g (38.9 mmol) of 3-ethoxy-4-amino-1,2,5-thiadiazole-1,1-dioxide. The clear solution is stirred 2 hours at room temperature during which a white precipitate separates. The precipitate is collected, boiled with 125 ml ethyl acetate, and refiltered to give 10.4 g of the pure title compound; m.p. 159—160°C with decomposition.

Example 19

3-N[3-[3-(1-Piperidinylmethyl)phenoxy]propyl]amino-4-amino-1,2,5-thiadiazole-1-oxide

A solution of 13.66 g (0.055 mol) of 3-(1-piperidinylmethyl)phenoxy]propane amine in 5 ml of $CH_3CN$ is added to 8.87 g (0.055 mol) of 3-ethoxy-4-amino-1,2,5-thiadiazole-1-oxide in 200 ml of $CH_3CN$. The solution is stirred at room temperature under nitrogen and colorless product begins to precipitate in about 15 minutes. After 24 hours the reaction mixture is filtered and the product is washed with $CH_3CN$. The product is suspended in 75 ml of $CH_3CN$ and stirred for 24 hours in a closed vessel. The solid is collected and again stirred with $CH_3CN$ for 48 hours. The solid is then collected and dried for 4 hours at 78° under vacuum to obtain 16.0 g (80%) of the pure product, m.p. 152.5—154.5°.

Example 20

3-N-[2-[[6-(4-Morpholinylmethyl)-2-benzofuranyl]methylthio)ethyl]amino-4-amino-1,2,5-thiadiazole-1-oxide

A. Ethyl 6-Methylbenzofuran-2-carboxylate

A solution of 6-methylbenzofuran-2-carboxylic acid (111.0 g., 0.636 mol) and concentrated sulfuric acid (4 ml.) in ethanol (1 l.) is boiled under reflux for 16 hours. About 2/3 of the solvent is removed by distillation at reduced pressure. The residue is poured into 1 l. of ice water. The oily ester is taken up in ether, washed with saturated sodium bicarbonate solution and water and dried over sodium sulfate. Distillation at reduced pressure affords 90.5 g. (70%) of ethyl 6-methylbenzofuran-2-carboxylate, b.p. 174—176°C (17 mm). The product crystallizes in the receiver, m.p. 35—42°C.

B. Ethyl 6-(bromomethyl)benzofuran-2-carboxylate

N-Bromosuccinimide (37.4 g., 0.21 mole) is added to a solution of ethyl-6-methylbenzofuran-2-carboxylate (40.8 g., 0.2 mole) and α,α'-azobisisobutyronitrile (500 mg.) in carbon tetrachloride (300 ml.). The suspension is boiled under reflux for 3 hours. It is then cooled and the succinimide removed by filtration. The carbon tetrachloride solution is washed with water and dried over sodium sulfate. The solution is then evaporated at reduced pressure. The solid residue is recrystallized from hexane to yield 47.4 g. (69%) of crystalline ethyl 6-(bromomethyl)benzofuran-2-carboxylate, m.p. 95—102°C. NMR ($CDCl_3$): δ 1.40 (3H, t, $CH_3$), 4.40 (2H, q, $CH_2O$), 4.58 (2H, S, $CH_2Br$).

C. Ethyl 6-(4-Morpholinylmethyl)benzofuran-2-carboxylate

To a solution of 10.0 g (0.035 mol) of ethyl 6-(bromomethyl) benzofuran-2-carboxylate in 100 ml of ether at 0—4° is added dropwise a solution of 7.7 g (0.088 mol) of morpholine in 100 ml of ether. The

mixture is stirred 18 hours, water is added and the layers separated. The organic layer is extracted with 3N hydrochloric acid. The acid extract is made basic with 10N sodium hydroxide and extracted with ethyl acetate. The extract is dried and concentrated to yield 8.4 g (86%) of ethyl 6-(4-morpholinylmethyl)benzofuran-2-carboxylate.

D. 2-Hydroxymethyl-6-(4-morpholinomethyl)benzofuran

To a suspension of 2.1 g (0.03 mol) of lithium aluminum hydride in 50 ml of ether is added, dropwise at room temperature, a solution of 8.4 g (0.03 mol) of ethyl 6-(4-morpholinylmethyl)benzofuran-2-carboxylate in 25 ml of ether. The mixture is stirred 18 hours, saturated $Na_2SO_4$ is added until a white suspension occurs and the solid is filtered. The solid is washed with chloroform and the filtrate is extracted with water. The chloroform-ether solution is concentrated to yield 4.7 g (63%) of 2-hydroxymethyl-6-(4-morpholinylmethyl)benzofuran, m.p. 105—107°C.

E. 2-[[6-(4-Morpholinylmethyl)-2-benzofuranyl]methylthio]ethane amine

2-Hydroxymethyl-6-(4-morpholinylmethyl)benzofuran 4.7 g (0.02 mol) is added in portions to an ice-cooled solution of 2.4 g (0.02 mol) of cysteamine hydrochloride in 10 ml of 12N HCl under nitrogen. The ice bath is removed and the solution is stirred for 17 hours at room temperature. It is cooled, adjusted to pH 10 with 40% NaOH and extracted with methylene chloride. The extract was concentrated to yield 6 g of 2-[[6-(4-morpholinylmethyl)-2-benzofuranyl]methylthio]ethane amine.

F. 3-N-[2-[[6-(4-Morpholinylmethyl)-2-benzofuranyl]methylthio]ethyl]amino-4-amino-1,2,5-thiadiazole-1-oxide

By the method of Example 11, using 2-[[6-(4-morpholinylmethyl)-2-benzofuranyl]methylthio]ethane amine, the title compound is obtained as a white solid, m.p. 143—146°C.

Example 21

3-N-[2-[[6-(4-Morpholinylmethyl)-2-benzofuranyl]methylthio]ethyl]amino-4-amino-1,2,5-thiadiazole-1,1-dioxide

Using the method of Example 18 with 2-[[6-(4-morpholinylmethyl)-2-benzofuranyl]methylthio]ethane amine as the amine reagent and acetonitrile as the solvent, the title product is obtained as a light yellow solid, m.p. 164—167°C.

Example 22

3-N-[2-[[6-(4-Morpholinylmethyl)-2-benzofuranyl]methylthio]ethyl]amino-4-methylamino-1,2,5-thiadiazole-1-oxide

By the method of Example 1B using as the amine reagent 2-[[6-(4-morpholinylmethyl)-2-benzofuranyl]-methylthio]ethane amine and acetonitrile as the solvent, with a six day reaction time, a crude product is obtained. Chromatography on silica gel yields the pure title compound; m.p. 139—143°C.

Example 23

3-N-[2-[[5-(4-Morpholinylmethyl)-2-furanyl]methylthio]ethyl]amino-4-amino-1,2,5-thiadiazole-1-oxide

By the method of Example 11, using 2-[[5-(4-morpholinylmethyl)-2-furanyl]methylthio]ethane amine as the amine reagent, the title compound is obtained as a crystalline solid, m.p. 142—144°C.

Example 24

3-N-[2-[[5-(1-Piperdinylmethyl)-2-furanyl]methylthio]ethyl]amino-4-amino-1,2,5-thiadiazole-1-oxide

By the method of Example 11, using 2-[[5-(1-piperidinylmethyl)-2-furanyl]methylthio]ethane amine as the amine reagent, the title compound is obtained as a solid, m.p. 150°.

Example 25

3-N-[2-[(4-Methyl-5-imidazolyl)methylthio]ethyl]amino-4-amino-1,2,5-thiadiazole-1-oxide

By the method of Example 11, using 2-[(4-methyl-5-imidazoyl)methylthio]ethane amine as the amine reactant, the title product is obtained in an impure condition. Chromatography on silica gel followed by precipitation from an isopropyl alcohol-ether mixture yields the pure title compound containing 0.31 molar equivalent of isopropyl alcohol; m.p. 98—126°.

Example 26

3-N-[2-[(4-Methyl-5-imidazolyl)methylthio]ethyl]amino-4-amino-1,2,5-thiadiazole-1,1-dioxide

By essentially the method of Example 18 using 2-[(4-methyl-5-imidazolyl)methylthio]ethane amine as the amine reactant and acetonitrile as the solvent, the title product is obtained. It is purified by chromatography on silica gel and precipitation from an ethanol-ether mixture to yield the pure title compound containing 0.5 molar equivalent of ethanol; m.p. 78—92°.

Example 27

3-N-[2-[(2-Guanidino-4-thiazolyl)methylthio]ethyl]amino-4-amino-1,2,5-thiadiazole-1-oxide

Replacing the 3-ethoxy-4-methylamino-1,2,5-thiadiazole-1-oxide in Example 3 with 3-ethoxy-4-amino-1,2,5-thiadiazole-1-oxide and purifying the product by chromatography on silica gel and crystallization yields the title compound; m.p. 186—188°

Example 28

3-N-[3-(2-Guanidino-4-thiazolyl)phenyl]amino-4-amino-1,2,5-thiadiazole-1-oxide

Using the procedure of Example 5 and replacing the methylamine solution in part 5B with a solution of ammonia in alcohol there is obtained a crystalline product which is recrystallized from dimethyl sulfoxide-acetonitrile mixture to give the pure title compound containing a molecule of crystallization of dimethyl sulfoxide; m.p. 219—220°.

Example 29

3-N-[2-[(6-Dimethylaminomethyl-2-pyridyl)methylthio]ethyl]amino-4-amino-1,2,5-thiadiazole-1-oxide Hydrochloride Hemihydrate

By replacing the 2[[3-(dimethylaminomethyl)phenyl]methylthio]ethyl amine of Example 11 with 2-[(6-dimethylaminomethyl-2-pyridyl)methylthio]ethane amine [disclosed in British Pat. 2,038,804] and carrying out the reaction and workup, in a similar manner there is obtained 3-N-[2-[(6-dimethylaminomethyl-2-pyridyl)methylethio]ethyl]amino-4-amino-1,2,5-thiadiazole-1-oxide. This base is dissolved in an equivalent amount of aqueous hydrochloric acid and the solution is lyophilized to yield the pure title compound hydrochloride salt as a hemihydrate in a glassy solid form.

Example 30

3-N-[3-[3-(Dimethylaminomethyl)phenoxy]propyl]amino-4-amino-1,2,5-thiadiazole-1,1-dioxide

By replacing the 3-ethoxy-4-amino-1,2,5-thiadiazole-1-oxide of Example 10 with 3-ethoxy-4-amino-1,2,5-thiadiazole-1,1-dioxide and carrying out the reaction in a similar manner in acetonitrile, a crystalline product is obtained. Recrystallization from nitromethane yields the title compound; m.p. 173—175°.

Example 31

3-N-[2-[(5-Dimethylaminomethyl-2-thienyl)methylthio]ethyl]amino-4-amino-1,2,5-thiadiazole-1-oxide

2-[(5-Dimethylaminomethyl-2-thienyl)methylthio]ethane amine and 3-ethoxy-4-amino-1,2,5-thiadiazole-1-oxide are allowed to react in acetonitrile solution according to the procedure of Example 11. The reaction product is purified by chromatography and then crystallization from an acetonitrile-methanol mixture to give the pure title compound; m.p. 143—147°.

Example 32

3-N-[2-[(5-Methylaminomethyl-2-furanyl)methylthio]ethyl]amino-4-amino-1,2,5-thiadiazole-1-oxide

The reaction of 2-[(5-methylaminomethyl-2-furanyl)methylthio]ethane amine with 3-ethoxy-4-amino-1,2,5-thiadiazole-1-oxide in acetonitrile is carried out by the procedure described in Example 11. The crude product is chromatographed on silica gel and then crystallized from ethyl alcohol to yield the pure title compound; m.p. 153—156°

Example 33

3-N-[3-[3-[(4-Methyl-1-piperazinyl)methyl]phenoxy]propyl]amino-4-amino-1,2,5-thiadiazole-1-oxide

By the method of Example 10, using 3-[3-[(4-methyl-1-piperazinyl)methyl]phenoxy]propane amine [prepared as in Example 14] as the propane amine reagent, a product is obtained which is crystallized from a chloroform-ether mixture to give the pure title compound; m.p. 133° with decomposition.

Example 34

3-N-[2-[(4-Methyl-5-oxazolyl)methylthio]ethyl]amino-4-amino-1,2,5-thiadiazole-1-oxide

The reaction of 2-[(4-methyl-5-oxazolyl)methylthio]ethane amine with 3-ethoxy-4-amino-1,2,5-thiadiazole-1-oxide in acetonitrile solvent by the procedure of Example 11 yields the title compound; m.p. 148—150°.

Example 35

3-N-[3-(6-Dimethylaminomethyl-2-pyridylthio)propyl]amino-4-amino-1,2,5-thiadiazole-1-oxide

A. 3-(6-Dimethylaminomethyl-2-pyridylthio)propane amine

A mixture of 3-mercaptopropylamine (4.8 g, 0.053 mol) and 60% sodium hydride in mineral oil (2.18 g, 0.054 mol) in dry dimethylformamide (50 ml) is heated to 80°C and a solution of 6-dimethylaminomethyl-2-chloropyridine (8.7 g, 0.051 mol) in 50 ml of dry DMF is added dropwise. The mixture is heated at 125° for 3 hours, then cooled, diluted with ethanol (70 ml) and, after filtration to remove precipitated salts, evaporated to dryness. This residue is dissolved in water and the product is extracted into chloroform, then into dilute HCl and the acidic extract is neutralized with $Na_2CO_3$ and the product is extracted into chloroform, dried and evaporated to give 9.5 g. This residual oil is distilled to give 5.64 g of the pure title product; bp: 129—133°C/ $0.6666 \cdot 10^2$ Pa (0.5 mm Hg).

B. 3-N-[3-(6-Dimethylaminomethyl-2-pyridylthio)propyl]amino-4-amino-1,2,5-thiadiazole-1-oxide Hydrochloride

The amine product from part A is reacted with 3-ethoxy-4-amino-1,2,5-thiadiazole-1-oxide essentially by the process of Example 10. The reaction product is chromatographed, dissolved in methanol, and an equivalent amount of alcoholic HCl is added. Acetonitrile is added and the pure title compound, m.p. 174—176°, is obtained.

Example 36

3-N-[2-(6-Dimethylaminomethyl-2-pyridylthio)ethyl]amino-4-amino-1,2,5-thiadiazole-1-oxide

A. 2-(6-Dimethylaminomethyl-2-pyridylthio)ethane amine

Cysteamine hydrochloride (3.43 g, 0.030 mol) is added to a suspension of 60% sodium hydride in mineral oil (2.5 g., 0.0625 mol) in dry dimethylformamide (30 ml) and is warmed to 80°C. A solution of 6-dimethylaminomethyl-2-chloropyridine (5.0 g, .0284 mol) in 30 ml of dry DMF is added dropwise and then the reaction temperature is increased to 125°C for 2 hours. Then, the reaction is cooled, diluted with ethanol (60 ml) and the salts are removed by filtration before evaporating to dryness. This residue is dissolved in chloroform, washed with water and extracted into diluted HCl. This acidic extract is neutralized with $Na_2CO_3$ and the product extracted into chloroform dried and evaporated to give 4.4 g. This residual oil is distilled to give 2.74 g of the pure title product; bp: 109—114°C/0,3333·$10^2$ Pa (0,25 mm Hg).

B. 3-N-[2-(6-Dimethylaminomethyl-2-pyridylthio)ethyl]amino-4-amino-1,2,5-thiadiazole-1-oxide

The amine product from part A is reacted with 3-ethoxy-4-amino-1,2,5-thiadiazole-1-oxide essentially by the process of Example 10. The pure title compound, m.p. 172—175°, crystallizes from the reaction mixture.

Example 37

3-N-[3-(6-Dimethylaminomethyl-2-pyridyloxy)propyl]amino-4-amino-1,2,5-thiadiazole-1-oxide

A. N-[3-(6-Dimethylaminomethyl-2-pyridyloxy)propyl]formamide

3-Aminopropanol (2.55 g, 0.034 mol) is added to a suspension of 60% sodium hydride in mineral oil (1.29 g, 0.033 mol) in dry dimethylformamide (30 ml) and the mixture is warmed to 80°C before adding dropwise a solution of 6-dimethylaminomethyl-2-chloropyridine (5.45 g, 0.032 mol) in 30 ml of dry DMF. The reaction temperature is increased to 125°C for 3 hours, then the reaction is cooled, diluted with ethanol and the precipitated salts filtered. The solvents are removed under vacuum. The residue is dissolved in chloroform, washed with water, extracted into dilute HCl and the acidic extract neutralized with $Na_2CO_3$. The crude product is extracted into chloroform, dried and evaporated (7.6 g). This residue is chromatographed on silica gel (200 g) eluting with 3—8% methanol/chloroform to give 3.2 g of the pure title product as an oil.

B. 3-(6-Dimethylaminomethyl-2-pyridyloxy)propylamine

N-[3-(6-Dimethylaminomethyl-2-pyridyloxy)propyl]formamide (2.7 g 0.013 mol) is added to a solution of 85% potassium hydroxide pellets (3.0 g) dissolved in methanol (30 ml) and heated at gentle reflux for 21 hours. The reaction is cooled, diluted with diethyl ether (30 ml), the precipitated salts filtered, and the solvent removed under vacuum. The residue is dissolved in water and the product exhaustively extracted into ethanol/chloroform, dried and evaporated to give 2.1 g of the pure title product.

C. 3-N-[3-(6-Dimethylaminomethyl-2-pyridyloxy)propyl]amino-4-amino-1,2,5-thiadiazole-1-oxide

The amine product from part B is reacted with 3-ethoxy-4-amino-1,2,5-thiadiazole-1-oxide essentially by the process of Example 10. Reaction product is purified by chromatography and crystallization from methyl alcohol-acetonitrile mixture to give the title compound; m.p. 139—142°.

Example 38

3-N-[3-(2-Pyridylthio)propyl]amino-4-amino-1,2,5-thiadiazole-1-oxide

3-(2-Pyridylthio)propyl amine is reacted with 3-ethoxy-4-amino-1,2,5-thiadiazole-1-oxide by the process of Example 10. The reaction product is recrystallized from a mixture of chloroform and methyl alcohol to yield the pure title compound; m.p. 177—179°C.

Example 39

3-N-[2-[(2-Pyridyl)methylthio]ethyl]amino-4-amino-1,2,5-thiadiazole-1-oxide

The reaction of 2-[(2-pyridyl)methylthio]ethane amine with 3-ethoxy-4-amino-1,2,5-thiadiazole-1-oxide is performed essentially as described in Example 11. The reaction product is purified by chromatography and crystallization from a methyl alcohol-acetonitrile mixture to give the title compound; m.p. 102—104°.

Example 40

3-N-[2-[(2-Pyrazinyl)methylthio]ethyl]amino-4-amino-1,2,5-thiadiazole-1-oxide

By reacting 2-[(2-pyrazinyl)methylthio]ethane amine with 3-ethoxy-4-amino-1,2,5-thiadiazole-1-oxide under conditions described in Example 11 and purifying the product by chromatography and crystallization from an acetonitrile-methyl alcohol mixture, the pure title compound; m.p. 148—150°, is obtained.

Example 41

3-N-[2-[(4-Methyl-5-thiazolyl)methylthio]ethyl]amino-4-amino-1,2,5-thiadiazole-1-oxide

A. 2-[(4-Methyl-5-thiazolyl)methylthio]ethaneamine

To 95 ml 48% HBr cooled in an ice bath, cysteamine hydrochloride (7.96 g, 0.07M) is added and the mixture stirred until a solution forms. To this solution 4-methyl-5-thiazolylmethanol (9.05 g, 0.07M) is slowly added. After stirring at 25° for 1/2 hour, the mixture is refluxed for 5 hours then, while cooled in an ice bath, made alkaline with sodium hydroxide (10N). The solution is extracted several times with methylene chloride. The methylene chloride extracts are diluted with an equal volume of ether, washed once with brine and dried over sodium sulfate. The solution is filtered and concentrated under vacuum and the residue distilled to obtain the colorless title product oil (10.05 g, 76%); b.p. 121.5—125°/0,93325·$10^2$ Pa (0,7 mm Hg).

B. 3-N-[2-[(4-Methyl-5-thiazolyl)methylthio]ethyl]amino-4-amino-1,2,5-thiadiazole-1-oxide

The amine from part A is reacted with 3-ethoxy-4-amino-1,2,5-thiadiazole-1-oxide by the procedure of Example 11 using tetrahydrofuran as the reaction solvent. Recrystallization of the product from methyl alcohol yields the pure title compound; m.p. 157—159°.

Example 42

3-N-[2-[(5-Dimethylaminomethyl-2-furanyl)methylthio]ethyl]amino-4-N-(2-phenylethyl)amino-1,2,5-thiadiazole-1-oxide

A. 3-Ethoxy-4-N-(2-phenylethyl)amino-1,2,5-thiadiazole-1-oxide

To a solution of 9.51 g (0.05 ml) of 3,4-diethoxy-1,2,5-thiadiazole-1-oxide in 20 ml of tetrahydrofuran (THF) at −10° is added a solution of 6.6 g (0.05 mol) of 2 phenylethane amine in 50 ml of THF over a 2 hour period. The mixture is stirred for 18 hours in an ice bath and then is concentrated under vacuum. The residue is washed with ether and then recrystallized from acetonitrile to give 10.8 g of 3-ethoxy-4-N-(2-phenylethyl)amino-1,2,5-thiadiazole-1-oxide; m.p. 116—118°.

B. 3-N-[2-[(5-Dimethylaminomethyl-2-furanyl)methylthio]ethyl]amino-4-N-(2-phenylethyl)amino-1,2,5-thiadiazole-1-oxide

The product from Part A and an equimolar amount of 2-[(5-dimethylaminomethyl-2-furanyl)methylthio]ethyl amine are stirred in THF essentially by the procedure of Example 12. The crystalline reaction product is recrystallized from acetonitrile-ether mixture to give the title compound; m.p. 92.5—94.5°.

Example 43

3-N-[2-[(5-Dimethylaminomethyl-2-furanyl)methylthio]ethyl]amino-4-N-[2-(4-pyridyl)ethyl]amino-1,2,5-thiadiazole-1-oxide

A. 3-Ethoxy-4-N-[2-(4-pyridyl)ethyl]amino-1,2,5-thiadiazole-1-oxide

By replacing the 2-phenylethan amine obtained in part A of Example 42 with an equimolar amount of 2-(4-pyridyl)ethane amine and proceeding in a similar manner, 3-ethoxy-4-N-[2-(4-pyridyl)ethyl]amino-1,2,5-thiadiazole-1-oxide crystallized from acetonitrile-ether mixture with m.p. 101—103° is obtained.

B. 3-N-[2-[(5-Dimethylaminomethyl-2-furanyl)methylthio]ethyl]amino-4-N-[2-(4-pyridyl)ethyl]amino-1,2,5-thiadiazole-1-oxide

The product from Part A of this Example 43 and an equimolar amount of 2-[(5-Dimethylaminomethyl-2-furanyl)methylthioethyl]amine are stirred in acetonitrile for 48 hours at room temperature. The reaction product is crystallized from ethyl acetate to give the above title compound; m.p. 88—91°.

Example 44

3-N-[2-[(2-Furanyl)methylthio]ethyl]amino-4-amino-1,2,5-thiadiazole-1-oxide

By reacting 2-[(2-furanyl)methylthio]ethane amine with 3-ethoxy-4-amino-1,2,5-thiadiazole-1-oxide in acetonitrile under conditions described in Example 11 and recrystallizing the solid product from acetonitrile, the pure title compound, m.p. 164° with decomposition, is obtained.

Example 45

3-N-[2-[(5-Dimethylaminomethyl-2-furanyl)methylthio]ethyl]amino-4-amino-1,2,5-thiadiazole-1-oxide

3-N-[2-[(2-Furanyl)methylthio]ethyl]amino-4-amino-1,2,5-thiadiazole-1-oxide (from Example 44) and dimethyl(methylene)ammonium iodide are reacted in acetonitrile solvent and heated under reflux for 10 minutes. The solvent is removed by vacuum concentration and aqueous potassium carbonate is added to the residue. After thorough mixing the insoluble, title product is collected and dried.

Example 46

3-N-[2-[(5-Dimethylaminomethyl-2-furanyl)methylthio]ethyl]amino-4-amino-1,2,5-thiadiazole-1-oxide

A. 3-N-(2-Hydroxyethyl)amino-4-amino-1,2,5-thiadiazole-1-oxide

To a solution of 0.31 g. (0.005 mol) of 2-aminoethanol in 8 ml of acetonitrile and 2 ml of ethanol, one

can add 0.8 g. (0.005 mol) of 3-ethoxy-4-amino-1,2,5-thiadiazole-1-oxide. The mixture can then be stirred at ambient temperature for 2 hours and the title product collected by filtration.

B. 5-(Dimethylaminomethyl)furfuryl Mercaptan

Thiourea (1.52 g., 0.02 mol) is added to a mixture of 1.8 ml. of concentrated HCl and 1.0 ml of water and cooled to 0°C. 5-(Dimethylaminomethyl) furfuryl alcohol hydrochloride (3.83 g., 0.02 mol) is added and the temperature is slowly increased to 60°C and this temperature is maintained one hour. The reaction is then cooled and 1.6 g. (0.04 mol) of solid sodium hydroxide is added. This mixture is heated under reflux for one hour in a nitrogen atmosphere. The reaction is cooled, extracted with methylene chloride and the extracts evaporated. The residue is extracted with ether and the ether is evaporated to yield and oil which is distilled at 11,999·$10^2$ Pa (9 mm Hg) to give the title product boiling at 109—113°C.

C. 3N-[2-[(5-Dimethylaminomethyl-2-furanyl)methylthio]ethyl]amino-4-amino-1,2,5-thiadiazole-1-oxide

To a solution of carbon tetrachloride (0.483 ml., 0.005 mol) and 1.31 g., (0.005 mol) of triphenylphosphine in 10 ml. of dry dimethylformamide, cooled in an ice bath, one can add 0.005 mol of 3-N-(2-hydroxyethyl)amino-4-amino-1,2,5-thiadiazole-1-oxide. The mixture can then be stirred at room temperature for one hour and 0.81 g., (0.005 mol) of 5-(dimethylaminomethyl)furfuryl mercaptan (from part B above) added. After stirring for 16 hours, the solvent can be evaporated and the residue digested in acetonitrile. The title product can then be collected by filtration.

Example 47

3-N-[2-(5-Dimethylaminomethyl-2-furanyl)methylthio]ethyl]amino-4-amino-1,2,5-thiadiazole-1-oxide

A. 2-[(5-Dimethylaminomethyl-2-furanyl)methylthio]ethanol.

A solution of 32.4 g. (0.17 mol) of 5-(dimethylaminomethyl)-2-furanemethanol hydrochloride and 17.6 ml of 2-mercaptoethanol in 160 ml 2 N hydrochloric acid is heated with stirring under nitrogen for 1.5 hr then cooled to room temperature overnight. The mixture is then reheated to 70° for 2 hrs., cooled, basified with 50 ml concentrated aqueous ammonia, and extracted with three 100 ml portions of $CH_2Cl_2$. The combined extracts are dried ($Na_2SO_4$), filtered and concentrated *in vacuo* to give a thick oil which is distilled to give 24.6 g of 2-[(5-dimethylaminomethyl-2-furanyl) methylthio]ethanol, bp 135° 0,6666·$10^2$ Pa (0,5 mm Hg).

B. 3-N-[2-[(5-Dimethylaminomethyl-2-furanyl)methylthio]ethyl]amino-4-amino-1,2,5-thiadiazole-1-oxide

A solution of triphenylphosphine (2.62 g. 10 mmol) in 25 ml dry $CH_3CN$ can be treated with diethylazodicarboxylate (1.8 g., 10 mmol) with cooling under dry nitrogen. The alcohol from step A (2.15 g. 10.0 mmol) can be added, followed immediately by 3,4-diamino-1,2,5-thiadiazole-1-oxide (1.32 g. 10 mmol). The mixture can then be stirred 36 hr at room temperature, filtered, and the filtrate concentrated *in vacuo*. The residue can be partitioned between 0.1N HCl and $CH_2Cl_2$ and the aqueous acid extracts saturated with $Na_2CO_3$ and extracted with 5% ethanol in ethyl acetate. The ethyl acetate extracts can be combined, dried over $Na_2SO_4$ filtered and concentrated *in vacuo*. The title product can then be separated from the starting alcohol by preparative high pressure liquid chromatography.

Example 48

3-N-[2-[(5-Dimethylaminomethyl-2-furanyl)methylthio]ethyl]amino-4-amino-1,2,5-thiadiazole-1-oxide

A. 3-N-(2-Mercaptoethyl)amino-4-amino-1,2,5-thiadiazole-1-oxide

3-Ethoxy-4-amino-1,2,5-thiadiazole-1-oxide (0.80 g., 0.005 mol) can be added to a solution of 0.385 g (0.005 mol) 2-mercaptoethane amine in 10 ml of ethanol (from 2-mercaptoethane amine hydrochloride and sodium ethoxide) and the mixture stirred for 16 hours. The precipitated title product can then be collected by filtration.

B. 3-N-[2-[(5-Dimethylaminoethyl-2-furanyl)methylthio]ethyl]amino-4-amino-1,2,5-thiadiazole-1-oxide

5-Dimethylaminomethylfurfuryl alcohol (0.775 g., 0.005 mol) can be added to a mixture of carbon tetrachloride (0.483 ml., 0.005 mole) and triphenylphosphine (1.31 g., 0.005 mole) in 10 ml of dry DMF at 5°C. The reaction mixture can then be stirred for one hour at room temperature and 0.005 mol of 3-N-(2-mercaptoethyl)amino-4-amino-1,2,5-thiadiazole-1-oxide added. Stirring can then be continued for 16 hours and the solvent evaporated under reduced pressure. The residue can be digested in acetonitrile and the title product collected by filtration.

Example 49

Comparison of Compounds Exhibiting Gastric Antisecretory Activity

Studies are performed in female beagle dogs with a chronic gastric fistula. The test compounds are administered i.v. at 0 min. Gastric secretion is stimulated by administration of a maximal secretory dose of histamine dihydrochloride (64 µg/kg, base, s.c.) at 0 min. Gastric output is collected by gravity drainage for three 30 min periods (0—30, 30—60 and 60—90 min). Output volume (ml) is measured and an aliquot is titrated to pH 7 to determine acid concentration (titratable acid mEq/L); total acid output (mEq) is calculated as the product of volume and acid concentration. Percent inhibition of volume and acid concentration.

Percent inhibition of volume, acid concentration and total acid output is calculated relative to a placebo trial in the same animals; $ED_{50}$ values are determined by linear regression analysis.

The results obtained with the test compounds are presented in Table I below wherein A and B are representative thiadiazole-1-oxide and thiadiazole-1,1-dioxide compounds of the present invention, and C and D are representative compounds disclosed in the prior art.

TABLE I

Inhibition of histamine evoked gastric secretion in dogs with a chronic gastric fistula (1)

| Test compounds | No. of doses | No. of animals/ dose(3) | IV $ED_{50}$ (mg/kg) (2) Total acid output |
|---|---|---|---|
| A | 5 | 4 | 0.007 |
| B | 5 | 3 | 0.005 |
| C | 2 | 3 | 1.1 |
| D | 4 | 4 | 1.8 |

(1) Histamine dihydrochloride (64 μg/kg base s.c.), a maximally effective secretory stimulus.
(2) $ED_{50}$ calculated at period of maximum output in placebo trials (0—30 min. after histamine).
(3) (N) minimum number of dogs per dose level.

A. 3-N-[2-[(5-Dimethylaminomethyl-2-furanyl)methylthio]ethyl]amino-4-amino-1,2,5-thiadiazole-1-oxide. (Example 8).

B. 3-N-[2-[(5-Dimethylaminomethyl-2-furanyl)methylthio]ethyl]amino-4-amino-1,2,5-thiadiazole-1,1-dioxide. (Example 18).

C. 3-N-[2-[(5-Dimethylaminomethyl-2-furanyl)methylthio]ethyl]amino-5-amino-1,2,4-triazole, disclosed in Belgian Patent 875,846 (Example 5) (Derwent Abstract 79110 B/44).

D. N-Cyano-N'-methyl-N''-[2-((4-methyl-5-imidazolyl)methylthio)ethyl]guanidine, disclosed in U.S. patent 3,950,333 (Examples 24 and 36; Claim 8); in clinical use under the generic name "Cimetidine".

As can be seen from the results shown in the foregoing table, compounds A and B of the present invention have potencies that range from about 150 to about 350 times greater than those of prior art compounds C and D.

The following preparations are provided to illustrate the processes which can be employed to obtain the novel intermediate compounds of the invention. Unless otherwise indicated, all temperatures are in degrees Celsius.

Preparation 1

3,4-Diethoxy-1,2,5-thiadiazole-1-oxide

To a solution of 441 g (3.06 moles) of diethyl oximidate in 300 ml of methylene chloride and 780 ml (9.63 mol) of pyridine is added dropwise with cooling 195 ml (3.2 mol) of thionyl chloride at 10° ± 5°C. The reaction mixture is stirred for 2 hours at room temperature. The mixture is partitioned between dilute hydrochloric acid and methylene chloride. The methylene chloride is washed once with dilute hydrochloric and, twice with saturated sodium bicarbonate solution and once with saturated sodium chloride solution. The organic layer is dried with magnesium sulfate, concentrated to a low volume and diluted with hexane. The crystals are removed by filtration, washed in water and dried *in vacuo* to yield 410 g of 3,4-diethoxy-1,2,5-thiadiazole-1-oxide m.p. = 74—78°C.

A sample is recrystallized from cyclohexane to yield white crystals m.p. = 75—78°C.

Mass spectrum 190 ($M^+$ $C_6H_{10}N_2OS$), 161 ($M^+$ —$C_2H_5$).

IR ($CHCl_3$) 1625 (C=N), 1060 $CM^{-1}$ (SO).

Proton N.M.R. ($CDCl_3$), δ 4.5 (q, J=7Hz, 4H, δ 1.5 (t, J=7Hz, 6H).

Preparation 2

3,4-Bis-methylthio-1,2,5-thiadiazole-1-oxide

To a solution of 353 g [2.39 moles] of bismethylthio oximidate in 1500 ml of methylene chloride and 580 ml of pyridine [7.2 moles] is added dropwise 188 ml [2.59 moles] of thionyl chloride at 0 + 5°C. The temperature of the mixture is raised to room temperature and stirred for 2 hours. The mixture is partitioned between methylene chloride and dilute hydrochloric acid. The organic layer is washed once with dilute hydrochloric acid, twice with saturated sodium bicarbonate and once with saturated sodium chloride solution. The organic layer is dried with magnesium sulfate and concentrated to a low volume and diluted

with ether. The crystals are filtered, washed with ether and dried to yield 401 g of 3,4-*bis*-methylthio-1,2,5-thiadiazole-1-oxide m.p. = 109—112°C.

Mass spectrum 194 ($M^+$ $C_4H_6OS_3$).

IR ($CHCl_3$) 1625 (C=N), 1050 $CM^{-1}$ (SO).

Proton N.M.R. ($CDCl_3$) 2.7 ppm s.

Preparation 3

3-Ethoxy-4-methylamino-1,2,5-thiadiazole-1-oxide

Anhydrous methylamine (7.7 g, 0.25 mole) is dissolved in 100 ml of tetrahydrofuran with dry ice-acetone cooling. This cold solution is added dropwise with stirring to 3,4-diethoxy-1,2,5-thiadiazole-1-oxide (47.5 g, 0.25 mole) in 250 ml of tetrahydrofuran at −15°C over 1—1/2 hours. Stirring is continued for an additional hour at −15°C and the solvent is removed *in vacuo*. The beige solid residue is purified by chromatography on silica gel (100 g) using chloroform. The nearly white solid (39.6 g) melts at 91—92 °C.

IR S=O Stretching vibration 1095 $cm^{-1}$.

$^{13}C$ NMR for thiadiazole-1-oxide carbons: 163.34, 157.36.

Preparation 4

3-Ethoxy-4-(1-pyrrolidinyl)-1,2,5-thiadiazole-1-oxide

To 50.0 g (263 mmoles) of 3,4-diethoxy-1,2,5-thiadiazole-1-oxide in 250 ml of ethanol is added 18.7 g (263 mmoles) of pyrrolidone. The mixture is stirred for 30 minutes at room temperature then concentrated to 1/4 volume and diluted with an equal volume of ether. The reaction mixture is filtered and the filtrate concentrated *in vacuo*, dissolved in methylene chloride and washed 3 times with dilute hydrochloric acid and twice with a saturated salt solution. The organic layer is dried over anhydrous magnesium sulfate and concentrated *in vacuo*. The oil crystallizes with scratching in ethyl acetate. The solid material is filtered affording 23.5 g of white crystals m.p. = 59—62°C identified as 3-ethoxy-4-(1-pyrrolidinyl)-1,2,5-thiadiazole-1-oxide.

Mass spectrum 215 ($M^+$), 186 ($M^+$—$C_2H_5$), 170 ($M^+$ —$C_4H_8N$), 119 ($M^+-C_5H_8N_2$), 70 ($C_4H_8N$).

IR ($CHCl_3$) 1625 (C=N), 1125 $CM^{-1}$ (SO).

Proton N.M.R. ($CDCl_3$) 4.5 (t, J=7Hz, 2H) 3.7 (m, 4H) 2.0 (M, 4H) 1.5 (t, J=7Hz 3H).

Preparation 5

3-Methylthio-4-(4-morpholino)-1,2,5-thiadiazole-1-oxide

1.9 g (10 mmoles) of 3,4-dimethylthio-1,2,5-thiadiazole-1-oxide and 0.9 ml (10 mmoles) of morpholine are dissolved in 25 ml of acetonitrile and stirred for 30 minutes at room temperature. The reaction mixture is filtered and 500 mg of crystals are obtained which proton nuclear magnetic resonance shows to be the 3-morpholino adduct. 200 Mg of this material is redissolved in 5 ml of acetonitrile and heated at reflux for 1 hour. The reaction mixture is evaporated to dryness and the residue is treated with ether and filtered. The solid material has a melting point of 142—148°C and nuclear magnetic resonance confirms the structure as that of 3-methylthio-4-(4-morpholino)-1,2,5-thiadiazole-1-oxide.

Preparation 6

3-Ethoxy-4-amino-1,2,5-thiadiazole-1,1-dioxide

To a suspension of 3,4-diethoxy-1,2,5-thiadiazole-1,1-dioxide (5.15 g, 25 mmol) in 25 ml of dry $CH_2Cl_2$ is added 4.05 g (25 mmol) of hexamethyldisilazane. The mixture is stirred 18 hours at room temperature under nitrogen and the resulting clear solution is filtered through a short column of silica gel. Elution of the column with 1—4% ethanol —$CH_2Cl_2$ gives fractions containing 3.8 g of pure 3-ethoxy-4-amino-1,2,5-thiadiazole-1,1-dioxide, m.p. 176—177°C.

Preparation 7

3-Ethoxy-4-amino-1,2,5-thiadiazole-1-oxide

To a partial suspension of 28.5 g (0.15 mol) of 3,4-diethoxy-1,2,5-thiadiazole-1-oxide in 75 ml of ethanol at room temperature is added dropwise a solution of 114 ml of ca 1.4 M ammonia in ethanol over a one hour period. Reaction progress is monitored by T.L.C. When about half complete, product begins to crystallize out. The precipitated product is filtered and washed with ethanol to give a white crystalline product, 12.4 g, m.p. 130—134°. Ethanol mother liquor is concentrated to give a second crop.

Preparation 8

3,4-Diamino-1,2,5-Thiadiazole-1-oxide

Into a reaction vessel containing ethanol (300 mls) immersed in a cooling ice bath, there is added with stirring until a slurry is formed 40.0 g (210 mmols) of 3,4-diethoxy-1,2,5-thiadiazole-1-oxide from preparation 1 above. Excess ammonia gas is slowly bubbled through the slurry for ca. 5 minutes until a clear solution is obtained. The ice bath is then removed and excess ammonia gas is continued to be bubbled through the clear solution for an additional 20 minutes, the rate of bubbling being at a moderate rate for the first 5 minutes and then increased to a rapid rate for the remaining 15 minutes until heavy crystals form. More ethanol (200 mls) is added and the rapid bubbling of ammonia gas through the solvent

is continued for an additional 5 minutes. The heavy crystals are then filtered, washed with ethanol, and dried at about $1{,}33 \cdot 10^2$ Pa (1 mm Hg) overnight to afford 98.0% of 3,4-amino-1,2,5-thiadiazole-1-oxide; m.p., 300°C.

NMR is found to be consistent with the structure.

The processes set forth in the foregoing Preparations are employed to obtain the following intermediate compounds of Formula XX:

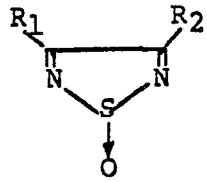

| $R_1$ | $R_2$ | Melting Point |
|---|---|---|
| $C_2H_5O$— | 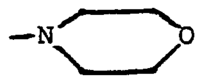 | 123—125°C |
| $C_2H_5O$ | 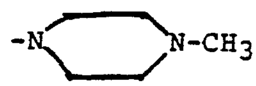 | 130°C |
| $C_2H_5O$— | —$NH_2$ | 130—133°C |
| $CH_3S$— | 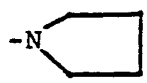 | 119—120°C |
| $C_2H_5O$— | 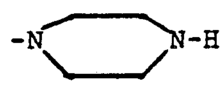 | 218°C |
| $CH_3S$— | —NH—$CH(CH_3)_2$ | 135—136° |

Similarly, the foregoing Preparations are employed to obtain the following intermediate compounds of Formula XXI:

3-amino-4-methylamino-1,2,5-thiadiazole-1-oxide;
3-amino-4-(2-dimethylaminoethyl)amino-1,2,5-thiadiazole-1-oxide;
3-amino-4-dodecylamino-1,2,5-thiadiazole-1-oxide;
3-amino-4-(4-fluoroanilino)-1,2,5-thiadiazole-1-oxide;
3-amino-4-morpholino-1,2,5-thiadiazole-1-oxide;
3-morpholino-4-(2-pyridinomethyl)amino-1,2,5-thiadiazole-1-oxide;
3,4-diamino-1,2,5-thiadiazole-1-oxide.

**Claims for the Contracting States: BE CH DE FR GB IT LI LU NL SE**

1. Compounds having the formulae:

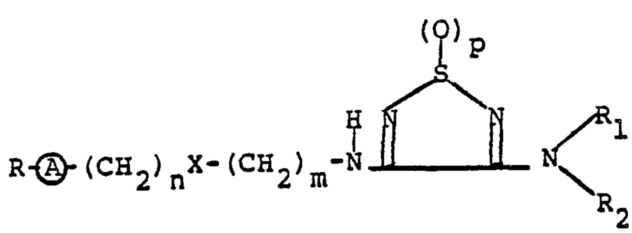

(I)

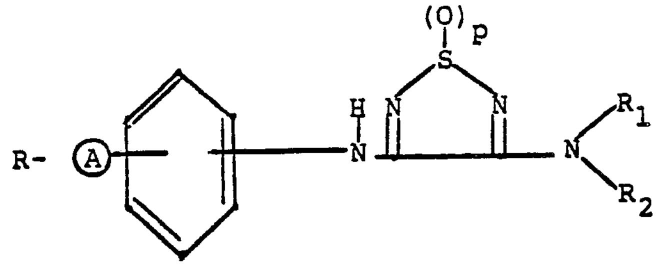

(II)

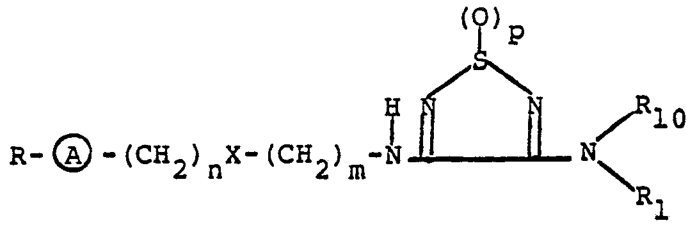

(IIIA)

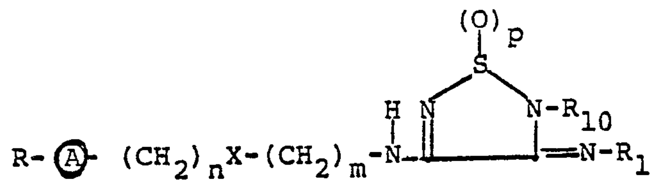

(IIIB)

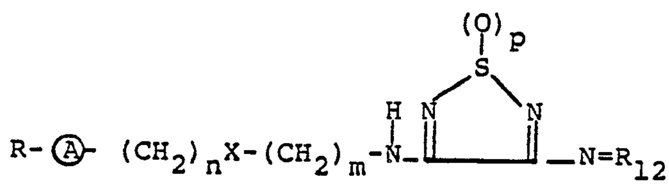

(IVA)

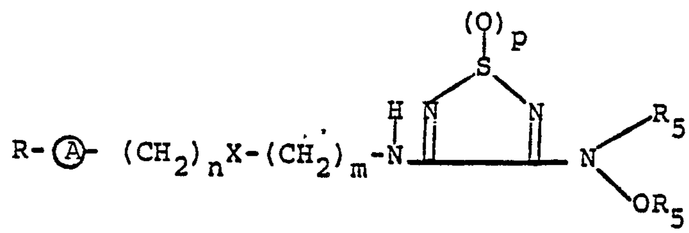

(IVB)

wherein R is hydrogen, alkyl having 1—5 carbon atoms or a group having the formula

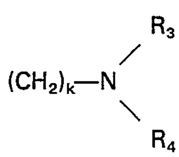

or

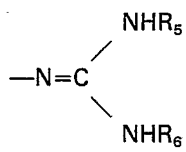

wherein

$R_3$ and $R_4$ are independently hydrogen, alkyl having 1—5 carbon atoms, cycloalkyl having 3—7 carbon atoms or phenylalkyl wherein the alkyl group has 1—5 carbon atoms, or

$R_3$ and $R_4$ form together with the nitrogen atom to which they are attached, a 5- or 6-membered heterocycle, which may also contain an oxygen, sulfur or N—$R_7$ heteroatom, wherein

$R_7$ is hydrogen, alkyl of from 1 to 3 carbon atoms or benzyl;

$R_5$ and $R_6$ are independently hydrogen or alkyl having 1—5 carbon atoms

n is 0 or 1;

m is 2 to 4;

k is 1 to 4;

X is oxygen, sulfur or methylene;

p is 1 or 2;

$R_1$ and $R_2$ are independently hydrogen, alkyl having 1—5 carbon atoms, alkenyl having 3—5 carbon atoms, alkynyl having 3—5 carbon atoms, cycloalkyl having 3—7 carbon atoms, phenyl, pyridyl and substituted alkyl having 1—5 carbon atoms in the alkyl group, wherein the substituent is phenyl, cycloalkyl

having 3—7 carbon atoms, pyridyl, imidazolyl, morpholino, hydroxy, alkoxy having 1—5 carbon atoms, alkylthio having 1—5 carbon atoms or dialkylamino wherein each alkyl group has 1—5 carbon atoms or

$R_1$ and $R_2$ form together with the nitrogen to which they are attached, a 5- or 6-membered heterocycle which may also contain an oxygen, sulfur or N—$R_7$ heteroatom wherein $R_7$ is as defined above;

Ⓐ is phenylene or a 5- or 6-membered heterocyclo containing one to three heteroatoms selected from oxygen, sulfur or nitrogen, which may optionally have a benzo ring fused thereon;

provided that when Ⓐ in formula I above is a 5-membered heterocycle or a benzo fused 5-membered heterocycle, n is 1; and,

provided further that when Ⓐ in either formula I or II is a furan having the structure

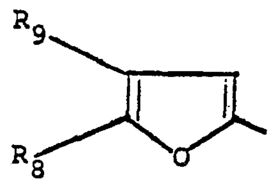

then

$R_8$ is the same as R defined above and

$R_9$ is alkyl having 1—5 carbon atoms, alkoxy-alkyl in which the alkoxy group and the alkyl group have each 1—5 carbon atoms, alkoxycarbonyl in which the alkoxy group has 1—5 carbon atoms, halo, preferably chlorine or bromine, or aryl, preferably phenyl;

$R_{10}$ is alkanoyl in which the alkyl group contains 1—5 carbon atoms, a phenylcarbonyl group in which the phenyl nucleus is optionally substituted with 1, 2 or 3 substituents, selected from halo, alkyl and alkoxy both having 1—5 carbon atoms, heteroaroyl in which the heterocyclic ring is a 6-membered N-containing ring having 1 or 2 heteroatoms, alkylsulfonyl in which the alkyl group has 1—5 carbon atoms, arylsulfonyl, heteroarylsulfonyl, in which the heterocyclic ring is a 6-membered N-containing ring having 1 or 2 heteroatoms, cyano, amidino, dialkylcarbamoyl in which the alkyl group contains 1—5 carbon atoms, dialkylsulfamoyl in which the alkyl group contains 1—5 carbon atoms, alkoxycarbonyl, in which the alkoxy group contains 1—5 carbon atoms or a group having the formula

$$—(CH_2)_yCOR_{11}$$

wherein

y is 1 or 2; and

$R_{11}$ is alkyl having 1—5 carbon atoms, hydroxy, alkoxy both having 1—5 carbon atoms, amino, alkylamino in which the alkyl group has 1—5 carbon atoms or dialkylamino in which the alkyl groups each have 1—5 carbon atoms; and

$R_{12}$ is aralkylidene or diarylmethylidene; and the salts of the compounds of formulae I, II, IIIa, IIIb, IVa and IVb and solvates of said compounds or solvates of the salts of said compounds as well as quaternary ammonium salts and N-oxides.

2. The compounds of claim 1 which are members selected from the group consisting of:
3-N-[3-(2-guanidino-4-thiazolyl)phenyl]amino-4-methylamino-1,2,5-thiadiazole-1-oxide;
3-N-[3-(2-guanidino-4-thiazolyl)phenyl]amino-4-amino-1,2,5-thiadiazole-1-oxide;
3-N-[3-[3-(dimethylaminomethyl)phenoxy]propyl]amino-4-amino-1,2,5-thiadiazole-1-oxide;
3-N-[3-[3-(dimethylaminomethyl)phenoxy]propyl]amino-4-methylamino-1,2,5-thiadiazole-1-oxide
3-N-[3-[3-(dimethylaminomethyl)phenoxy]propyl]amino-4-amino-1,2,5-thiadiazole-1,1-dioxide;
3-N-[2-[(2-furanyl)methylthio]ethyl]amino-4-amino-1,2,5-thiadiazole-1-oxide;
3-N-[2-[(5-dimethylaminomethyl-2-thienyl)methylthio]ethyl]amino-4-amino-1,2,5-thiadiazole-1-oxide;
3-N-[2-[(4-methyl-5-oxazolyl)methylthio]ethyl]amino-4-amino-1,2,5-thiadiazole-1-oxide;
3-N-[2-[(2-furanyl)methylthio]ethyl]amino-4-amino-1,2,5-thiadiazole-1,1-dioxide;
3-N-[2-[[5-(1-piperidinylmethyl)-2-furanyl]methylthio]ethyl]amino-4-amino-1,2,5-thiadiazole-1-oxide;
3-N-[2-[(5-dimethylaminomethyl-2-furanyl)methylthio]ethyl]amino-4-amino-1,2,5-thiadiazole-1-oxide;
3-N-[2-[(5-dimethylaminomethyl-2-furanyl)methylthio]ethyl]amino-4-methylamino-1,2,5-thiadiazole-1-oxide;
3-N-[2-[(5-dimethylaminomethyl-2-furanyl)methylthio]ethyl]amino-4-methylamino-1,2,5-thiadiazole-1,1-dioxide;
3-N-[2-[(5-dimethylaminomethyl-2-furanyl)methylthio]ethyl]-amino-4-amino-1,2,5-thiadiazole-1,1-dioxide;
3-N-[2-[(5-methylaminomethyl-2-furanyl)methylthio]ethyl]amino-4-amino-1,2,5-thiadiazole-1-oxide;
3-N-[2-[(5-methylaminomethyl-2-furanyl)methylthio]ethyl]amino-4-amino-1,2,5-thiadiazole-1,1-dioxide;
3-N-[2-[(2-guanidino-4-thiazolyl)methylthio]ethyl]amino-4-methylamino-1,2,5-thiadiazole-1,1-dioxide;
3-N-[2-[(2-guanidino-4-thiazolyl)methylthio]ethyl]amino-4-amino-1,2,5-thiadiazole-1-oxide;
3-N-[2-[(2-guanidino-4-thiazolyl)methylthio]ethyl]amino-4-methylamino-1,2,5-thiadiazole-1-oxide; and,
3-N-[2-[(6-(4-morpholinylmethyl-2-benzofuranyl)methylthio]ethyl]amino-4-amino-1,2,5-thiadiazole-1-oxide.

3. A compound of formula I of claim 1 which is the 3-N-[3-[3-(1-piperidinylmethyl)phenoxy]propyl]-amino-4-amino-1,2,5-thiadiazole-1-oxide.

4. Compounds having the formulae:

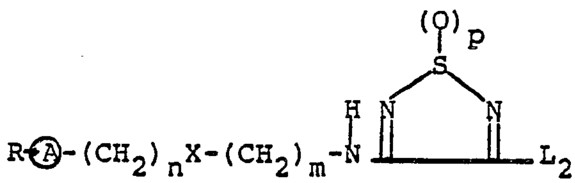

(VI)

and

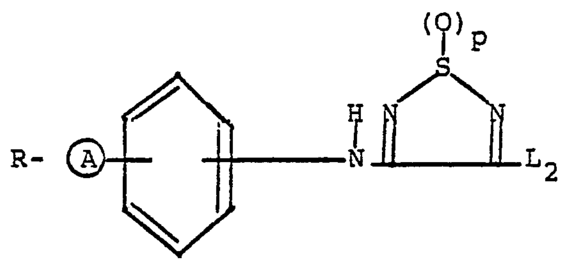

(VI A)

wherein R, Ⓐ, n, X, and m are as defined in claim 1; p is 1 or 2; $L_2$ is alkoxy having 1—5 carbon atoms, alkylthio having 1—5 carbon atoms, phenoxy, phenylthio or halogen; and the salts thereof.

5. The compounds of claim 4 which are members selected from the group consisting of:
3-N-[2-[5-dimethylaminomethyl-2-furanyl)methylthio]ethyl]amino-4-ethoxy-1,2,5-thiadiazole-1-oxide and
3-N-[2-[(5-dimethylaminomethyl-2-furanyl)methylthio]ethyl]amino-4-ethoxy-1,2,5-thiadiazole-1,1-dioxide.

6. Compounds having the formulae:

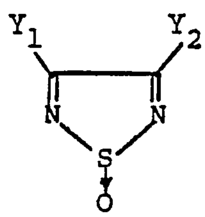

(XX)

wherein $Y_1$ and $Y_2$ are members selected from the following groups:

a) $Y_1$ and $Y_2$ are the same and are alkoxy having 1—5 carbon atoms, alkylthio having 1—5 carbon atoms, phenoxy or phenylthio; or,

b) $Y_1$ and $Y_2$ are different and one of $Y_1$ and $Y_2$ is alkoxy having 1—5 carbon atoms, phenoxy or phenylthio and the other of $Y_1$ and $Y_2$ is a group having the formula:

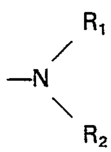

wherein $R_1$ and $R_2$ are independently as defined in claim 1 or $R_1$ and $R_2$ form together with the nitrogen atom to which they are attached a group as defined in claim 1, and

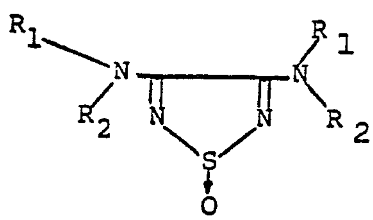

(XXI)

wherein $R_1$ and $R_2$ are independently as defined in claim 1 or $R_1$ and $R_2$ form together with the nitrogen atom to which they are attached a group as defined in claim 1.

7. Compounds having the formulae

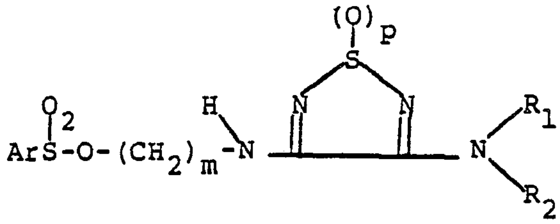

(XVII)

wherein Ar is aryl and $R_1$, $R_2$, m and p are as defined in claim 1 and

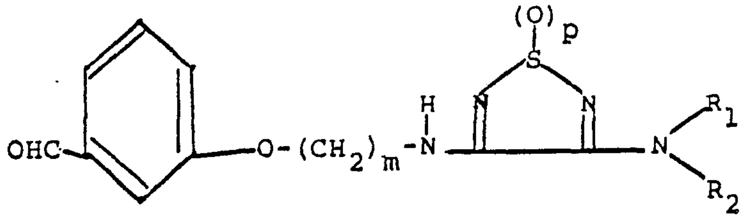

(XIX)

wherein $R_1$, $R_2$, m and p are as defined in claim 1.

8. Compounds of claim 6 which are compounds of formula XX or XXI and members selected from the group consisting of:
3,4-diethoxy-1,2,5-thiadiazole-1-oxide;
3,4-bis-methylthio-1,2,5-thiadiazole-1-oxide;
3-isopropylamino-4-methylthio-1,2,5-thiadiazole-1-oxide;
3-ethoxy-4-amino-1,2,5-thiadiazole-1-oxide;
3-ethoxy-4-methylamino-1,2,5-thiadiazole-1-oxide;
3-ethoxy-4-(1-pyrrolidinyl)-1,2,5-thiadiazole-1-oxide;
3-ethoxy-4-(4-morpholino)-1,2,5-thiadiazole-1-oxide;
3,4-diamino-1,2,5-thiadiazole-1-oxide;
3-amino-4-(2-dimethylaminoethyl)amino-1,2,5-thiadiazole-1-oxide;
3-amino-4-methylamino-1,2,5-thiadiazole-1-oxide;
3-amino-4-dodecylamino-1,2,5-thiadiazole-1-oxide;
3-amino-4-(4-fluoroanilino)-1,2,5-thiadiazole-1-oxide;
3-amino-4-morpholino-1,2,5-thiadiazole-1-oxide; and,
3-morpholino-4-(2-pyridinomethyl)amino-1,2,5-thiadiazole-1-oxide.

9. A process for the preparation of a compound of formulae I, II, IV, A or IV B according to Claim 1 which comprises treating a compound having the formula:

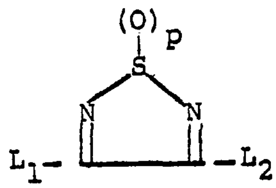

wherein p is 1 or 2 and $L_1$ and $L_2$ are leaving groups, first with one of the amines having the formulae

$$R-\textcircled{A}-(CH_2)_n-X-(CH_2)_m-NH_2$$

or

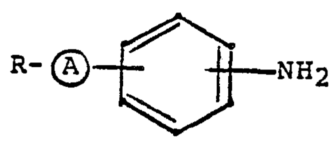

or

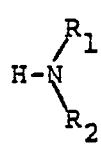

wherein
R, Ⓐ, n, X, m, $R_1$ and $R_2$ are as defined in claim 1, yielding an intermediate product of formula VI

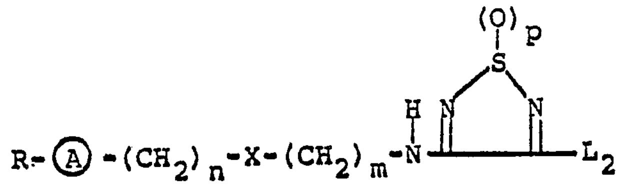

(VI)

or VIa

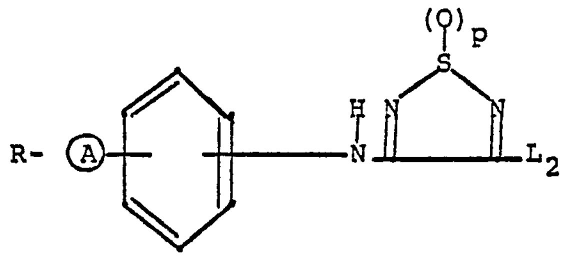

(VIa)

or
VII

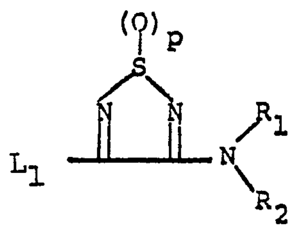

(VII)

and reacting thereafter the intermediate product of formulae VI and VIa with the amine of formula

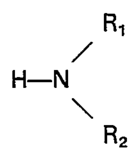

or the intermediate product of formula VII with either the amine

R-Ⓐ-$(CH_2)_n$-X-$(CH_2)_m$-$NH_2$

or the amine

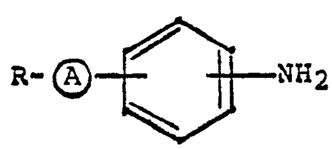

to yield the final products of formula I or II and optionally reacting a compound of formula I or II in which the group

R—Ⓐ—

is an unsubstituted furane ring with either

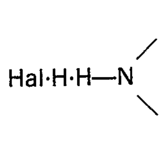 + $CH_2O$ or 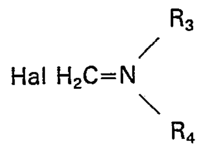

wherein Hal is halo to produce corresponding compounds of formula I or II wherein A is a furane ring substituted with a group R having the formula

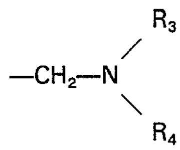

wherein $R_3$ and $R_4$ are as defined in claim 1 or reacting the intermediate compound of formula VI

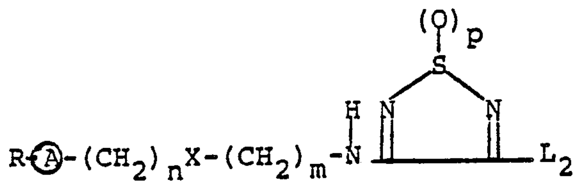

(VI)

with an imine of formula

$$H—N=R_{12}$$

to produce compounds of formula IVa

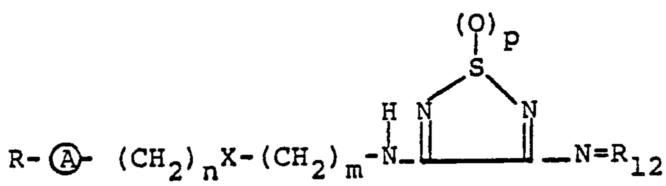

(IVA)

or reacting the intermediate product of formula VI

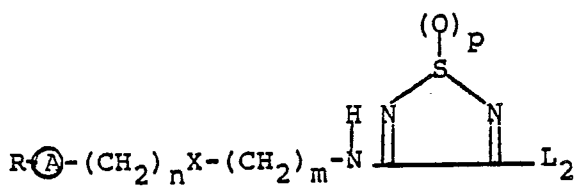

(VI)

with an optionally substituted hydroxyl amine of formula

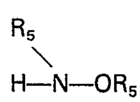

to produce compounds of formula IVb

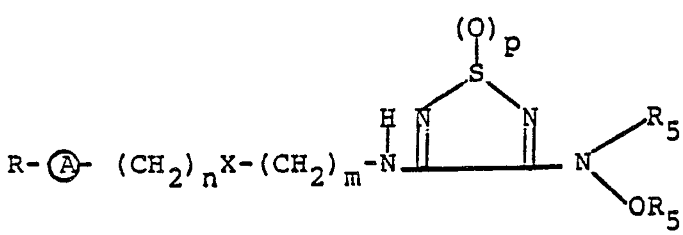

(IVB)

wherein R, $R_5$, Ⓐ, X, m, n and p are as defined in claim 1 and that the compounds of formulae I, II, IVa or IVb are isolated as free bases or salts or as solvates of the free bases or the salts or are converted into quaternary ammonium salts or N-oxides of said compounds.

10. Process for the preparation of 3-N-[3-[3-(1-piperidinylmethyl)phenoxy]propyl]amino-4-amino-1,2,5-thiadiazole-1-oxide according to claim 3, characterized in that 3-ethoxy-4-amino-1,2,5-thiadiazole-1-oxide is reacted with 3-[3-(1-piperidinylmethyl)phenoxy]propane-amine yielding the desired final product.

11. Process for the preparation of compounds of formulae I, II, IIIa and IIIb according to claim 1 which comprises reacting an alcohol having the formula XVI

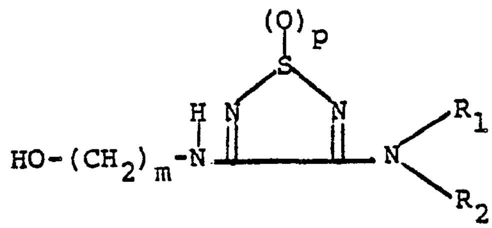

(XVI)

wherein $R_1$, $R_2$ and p are as defined in claim 1 and m> 2, with an acylating agent to obtain an activated ester intermediate; reacting said ester intermediate with an alkali metal salt of 3-hydroxy benzaldehyde to obtain an intermediate having the formula:

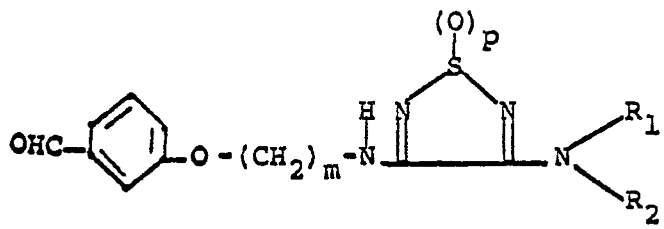

(XIX)

and reducing said intermediate in the presence of amine $R_3NHR_4$, wherein $R_3$ and $R_4$ are as defined in Claim 1; or, reacting said ester intermediate when m=2 with a mercaptan having the formula R—Ⓐ—$(CH_2)_n$—SH wherein R, Ⓐ and n are as defined in Claim 1; or reacting an alcohol having the formula

R—Ⓐ—$(CH_2)_n$—X—$CH_2CH_2OH$

wherein R, Ⓐ, X and n are as defined in Claim 1 with, after activation of said alcohol toward nucleophiles, an amino compound having the formula:

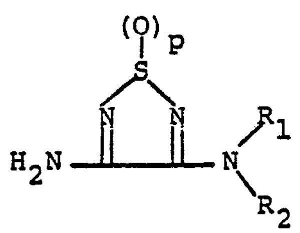

wherein $R_1$, $R_2$ and p are as defined in Claim 1; or, reacting a mercaptan having the formula:

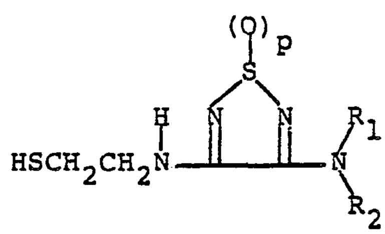

wherein $R_1$, $R_2$ and p are as defined in Claim 1, with an alcohol having the formula R—Ⓐ—$(CH_1)_n$—OH wherein R, Ⓐ and n are as defined in Claim 1, in the presence of a mineral acid to afford the compounds of Claim 1; and the physiologically acceptable salts thereof; or, reacting the compounds defined in Claim 1 and tautomers thereof with an alkylating reagent, an acylating agent or a sulfonating agent to produce derivatives having the formulae:

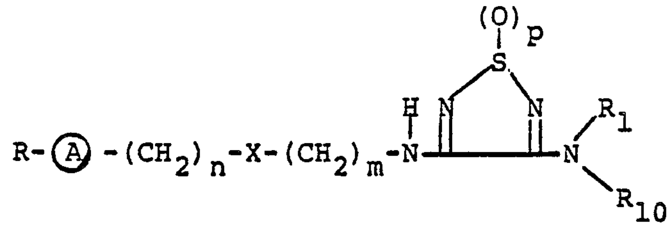

(IIIA)

and

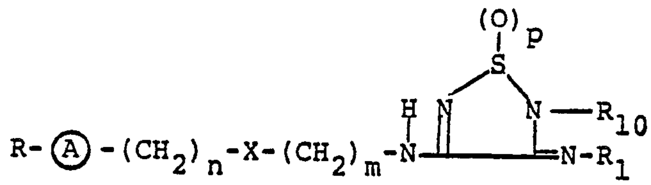

(IIIB)

wherein R, $R_{10}$, $R_1$, Ⓐ, X, m, n and p are as defined in Claim 1; and, the physiologically acceptable salts thereof.

12. A process for the preparation of compounds of claim 6 which comprises treating a compound having the formula:

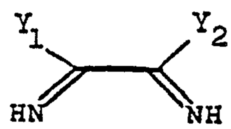

wherein $Y_1$ and $Y_2$ are as defined in a) in claim 6 with thionyl chloride in the presence of a base to form a compound having the formula:

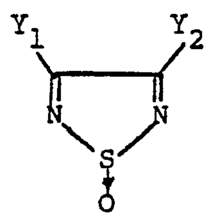

(XX)

which is treated with an amine having the formula:

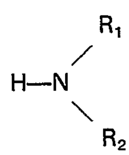

wherein $R_1$ and $R_2$ are as defined in claim 6 to form compounds wherein $Y_1$ and $Y_2$ are as defined in b) in claim 6 or, treating a compound having the formula:

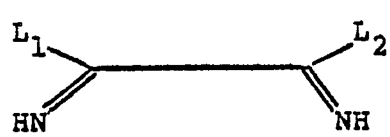

wherein $L_1$ and $L_2$ are leaving groups selected from the group consisting of alkoxy having 1—5 carbon atoms, alkylthio having 1—5 carbon atoms, phenoxy, or phenylthio, with thionyl chloride in the presence of a base to form an intermediate compound having the formula:

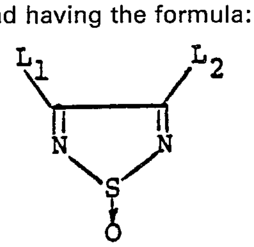

which is reacted with an amine of formula

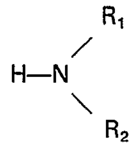

or sequentially with two different amines having said formula wherein $R_1$ and $R_2$ are independently as defined in claim 6 or $R_1$ and $R_2$ form together with the nitrogen atom to which they are attached a group as defined in claim 6 to yield a compound having the formula XXI

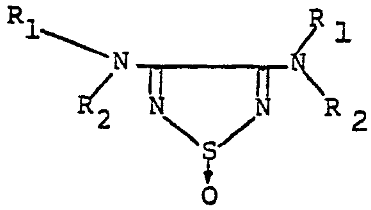

(XXI)

wherein $R_1$ and $R_2$ are as defined in claim 6.

13. A composition useful for suppressing gastric acid secretions in an animal with excess gastric acid secretions which comprises an inert.carrier and an effective amount of a compound of claim 1.

**Claims for the Contracting State: AT**

1. Process for the preparation of compounds having the formulae

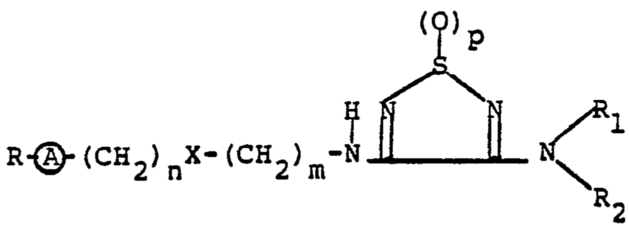

(I)

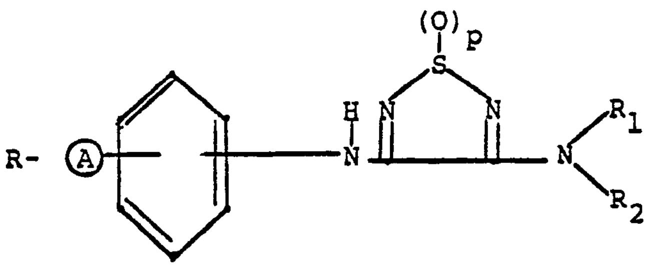

(II)

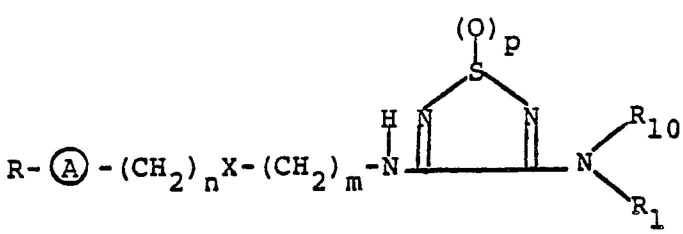

(IIIA)

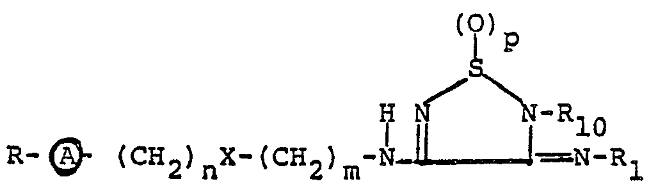

(IIIB)

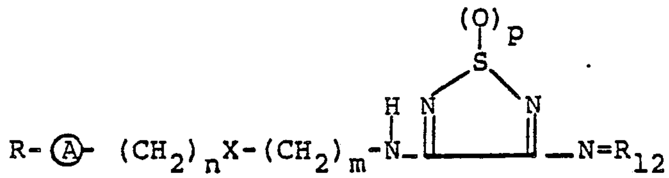

(IVA)

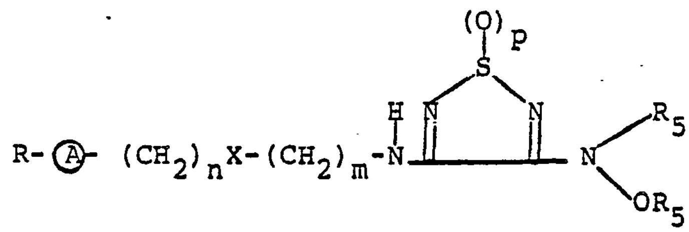

(IVB)

wherein

R is hydrogen, alkyl having 1—5 carbon atoms or a group having the formula

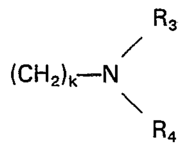

or

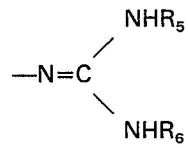

wherein

$R_3$ and $R_4$ are independently hydrogen, alkyl having 1—5 carbon atoms, cycloalkyl having 3—7 carbon atoms or phenylalkyl wherein the alkyl group has 1—5 carbon atoms, or

$R_3$ and $R_4$ form together with the nitrogen atom to which they are attached, a 5- or 6-membered heterocycle, which may also contain an oxygen, sulfur or N—$R_7$ heteroatom, wherein

$R_7$ is hydrogen, alkyl of from 1 to 3 carbon atoms or benzyl;

$R_5$ and $R_6$ are independently hydrogen or alkyl having 1—5 carbon atoms

n is 0 or 1;

m is 2 to 4;

k is 1 to 4;

X is oxygen, sulfur or methylene;

p is 1 or 2;

$R_1$ and $R_2$ are independently hydrogen, alkyl having 1—5 carbon atoms, alkenyl having 3—5 carbon atoms alkynyl having 3—5 carbon atoms, cycloalkyl having 3—7 carbon atoms, phenyl, pyridyl and substituted alkyl having 1—5 carbon atoms in the alkyl group, wherein the substituent is phenyl, cycloalkyl having 3—7 carbon atoms, pyridyl, imidazolyl, morpholino, hydroxy, alkoxy having 1—5 carbon atoms, alkylthio having 1—5 carbon atoms or dialkylamino wherrein each alkyl group has 1—5 carbon atoms or

$R_1$ and $R_2$ form together with the nitrogen to which they are attached, a 5- or 6-membered heterocycle which may also contain an oxygen, sulfur or N—$R_7$ heteroatom wherein

$R_7$ is as defined above;

Ⓐ is phenylene or a 5- or 6-membered heterocyclo containing one to three heteroatoms selected from oxygen, sulfur or nitrogen, which may optionally have a benzo ring fused thereon;

provided that when Ⓐ in formula I above is a 5-membered heterocycle or a benzo fused 5-membered heterocycle, n is 1; and,

provided further that when Ⓐ in either formula I or II is a furan having the structure

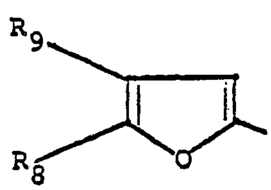

then

$R_8$ is the same as R defined above and

$R_9$ is alkyl having 1—5 carbon atoms, alkoxyalkyl in which the alkoxy group and the alkyl group have each 1—5 carbon atoms, alkoxycarbonyl in which the alkoxy group has 1—5 carbon atoms, halo, preferably chlorine or bromine, or aryl, preferably phenyl;

$R_{10}$ is alkanoyl in which the alkyl group contains 1—5 carbon atoms, a phenylcarbonyl group in which the phenyl nucleus is optionally substituted with 1, 2 or 3 substituents, selected from halo, alkyl and alkoxy both having 1—5 carbon atoms, heteroaroyl in which the heterocyclic ring is a 6-membered N-containing

ring having 1 or 2 heteroatoms, alkylsulfonyl in which the alkyl group has 1—5 carbon atoms, arylsulfonyl, heteroarylsulfonyl, in which the heterocyclic ring is a 6-membered N-containing ring having 1 or 2 heteroatoms, cyano, admidino, dialkylcarbamoyl in which the alkyl group contains 1—5 carbon atoms, dialkylsulfamoyl in which the alkyl group contains 1—5 carbon atoms, alkoxycarbonyl, in which the alkoxy group contains 1—5 carbon atoms or a group having the formula

$$—(CH_2)_yCOR_{11}$$

wherein

y is 1 or 2; and $R_{11}$ is alkyl having 1—5 carbon atoms, hydroxy, alkoxy in which the alkyl group contains 1—5 carbon atoms, amino, alkylamino in which the alkyl group has 1—5 carbon atoms or dialkylamino in which the alkyl groups each have 1—5 carbon atoms; and

$R_{12}$ is aralkylidene or diarylmethylidene, and the salts of the compounds of formulae I, II, IIIa, IIIb, IV and IVb and solvates of said compounds or solvates of the salts of said compounds as well as quaternary ammonium salts and N-oxides, characterized in that the compounds of formulae I, II, IVa or IVb are prepared by treating a compound having the formula:

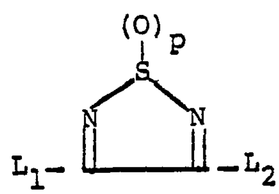

wherein $L_1$ and $L_2$ are leaving groups, first with one of the amines having the formulae

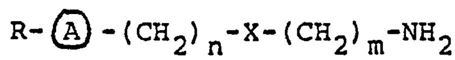

or

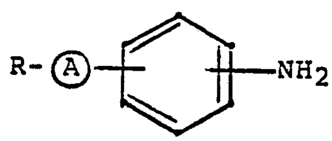

or

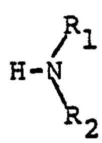

yielding an intermediate product of formula VI

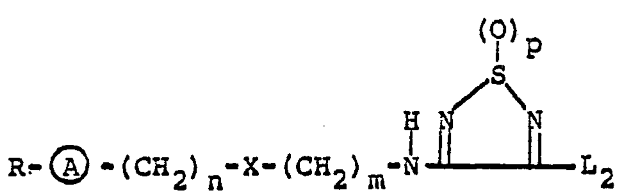

(VI)

or VIa

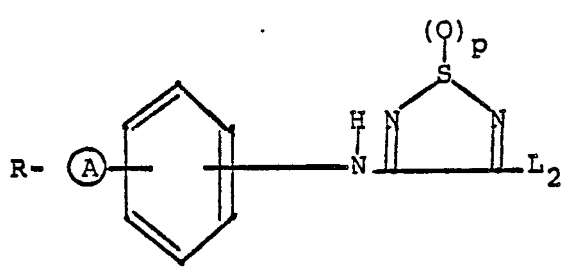

(VIa)

or

VII

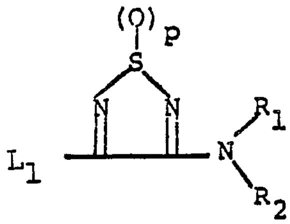

(VII)

and reacting thereafter the intermediate product of formulae VI and VIa with the amine of formula

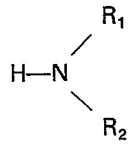

or the intermediate product of formula VII with either the amine

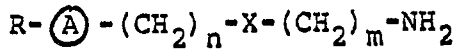

or the amine

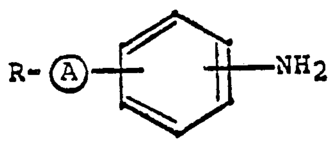

to yield the final products of formula I or II and optionally reacting a compound of formula I or II in which the group

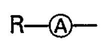

is an unsubstituted furane ring with either

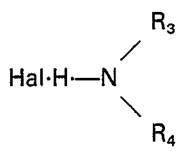 + $CH_2O$ or 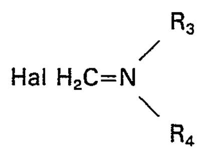

wherein Hal is halo to produce corresponding compounds of formula I or II wherein A is a furane ring substituted with a group R having the formula

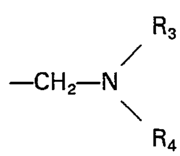

or reacting the intermediate compound of formula VI

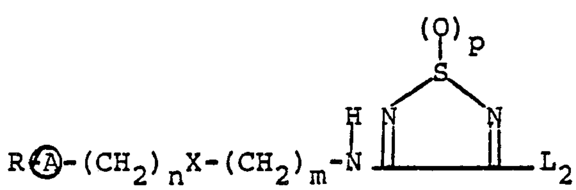

(VI)

with an imine of formula

$$H—N=R_{12}$$

to produce compounds of formula IVa

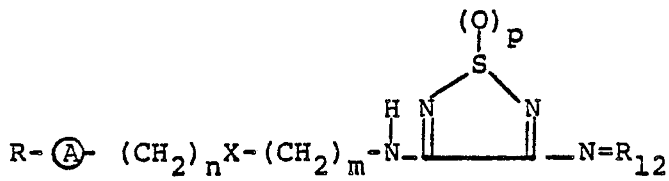

(IVA)

or reacting the intermediate product of formula VI

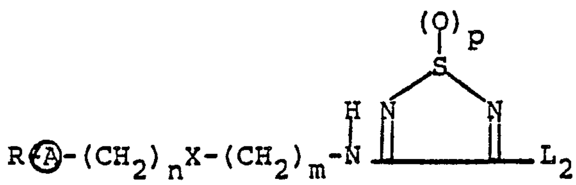

(VI)

with an optionally substituted hydroxyl amine of formula

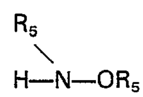

to produce compounds of formula IVb

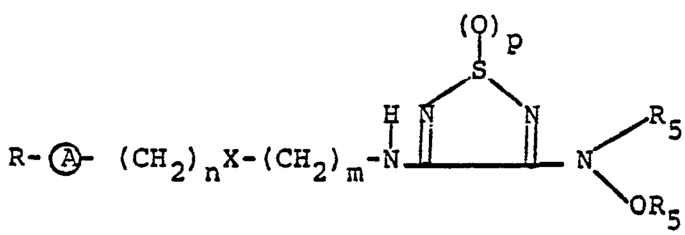

(IVB)

and that the compounds of formulae I, II, IVa or IVb are isolated as free bases or salts or as solvates of the free bases or the salts or are converted into quaternary ammonium salts or N-oxides of said compounds or that the compounds of formulae I, II, IIIa and IIIb are prepared by reacting an alcohol having the formula XVI

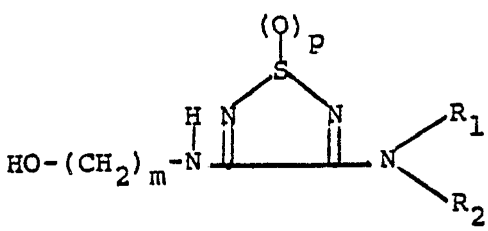

(XVI)

wherein m>2, with an acylating agent to obtain an activated ester intermediate; reacting said ester intermediate with an alkali metal salt of 3-hydroxy benzaldehyde to obtain an intermediate having the formula:

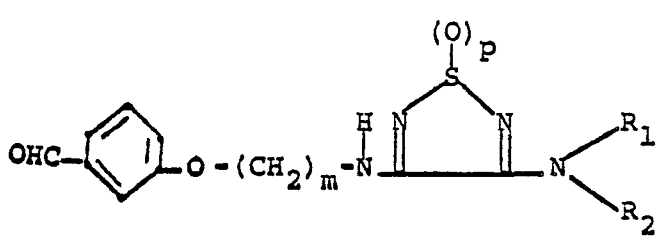

(XIX)

and reducing said intermediate in the presence of amine $R_3NHR_4$; or reacting said ester intermediate when M=2 with a mercaptan having the formula R—Ⓐ—$(CH_2)_n$—SH; or reacting an alcohol having the formula

R—Ⓐ—$(CH_2)_n$—X—$CH_2CH_2OH$

with, after activation of said alcohol toward nucleophiles, an amino compound having the formula:

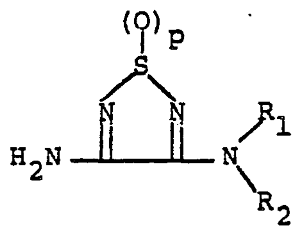

or reacting a mercaptan having the formula:

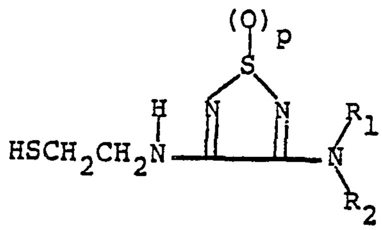

with an alcohol having the formula

R—Ⓐ—$(CH_2)_n)$—OH

in the presence of a mineral acid to afford the compounds of formula I or II and the physiologically acceptable salts thereof; or reacting the compounds of formula I and tautomers thereof with an alkylating reagent, an acylating agent or a sulfonating agent to produce derivatives having the formulae:

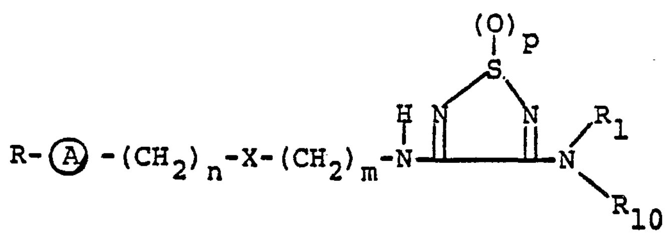

(IIIA)

and

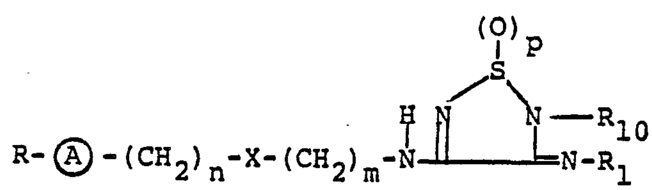

(IIIB)

and, the physiologically acceptable salts thereof.

2. Process according to claim 1 characterized in that compounds are prepared which are members selected from the group consisting of:
3-N-[3-(2-guanidino-4-thiazolyl)phenyl]amino-4-methylamino-1,2,5-thiadiazole-1-oxide;
3-N-[3-(2-guanidino-4-thiazolyl)phenyl]amino-4-amino-1,2,5-thiadiazole-1-oxide;
3-N-[3-[3-(dimethylaminomethyl)phenoxy]propyl]amino-4-amino-1,2,5-thiadiazole-1-oxide;
3-N-[3-[3-(dimethylaminomethyl)phenoxy]propyl]amino-4-methylamino-1,2,5-thiadiazole-1-oxide;
3-N-[3-[3-(dimethylaminomethyl)phenoxy]propyl]amino-4-amino-1,2,5-thiadiazole-1,1-dioxide;
3-N-[2-[(2-furanyl)methylthio]ethyl]amino-4-amino-1,2,5-thiadiazole-1-oxide;
3-N-[2-[(5-dimethylaminomethyl-2-thienyl)methylthio]ethyl]amino-4-amino-1,2,5-thiadiazole-1-oxide;
3-N-[2-[(4-methyl-5-oxazolyl)methylthio]ethyl]amino-4-amino-1,2,5-thiadiazole-1-oxide;
3-N-[2-[(2-furanyl)methylthio]ethyl]amino-4-amino-1,2,5-thiadiazole-1,1-dioxide;
3-N-[2-[[5-(1-piperidinylmethyl)-2-furanyl]methylthio]ethyl]amino-4-amino-1,2,5-thiadiazole-1-oxide;
3-N-[2-[(5-dimethylaminomethyl-2-furanyl)methylthio]ethyl]amino-4-amino-1,2,5-thiadiazole-1-oxide;

3-N-[2-[(5-dimethylaminomethyl-2-furanyl)methylthio]ethyl]amino-4-methylamino-1,2,5-thiadiazole-1-oxide;
3-N-[2-[(5-dimethylaminomethyl-2-furanyl)methylthio]ethyl]amino-4-methylamino-1,2,5-thiadiazole-1,1-dioxide;
3-N-[2-[(5-dimethylaminomethyl-2-furanyl)methylthio]ethyl]-amino-4-amino-1,2,5-thiadiazole-1,1-dioxide;
3-N-[2-[(5-methylaminomethyl-2-furanyl)methylthio]ethyl]amino-4-amino-1,2,5-thiadiazole-1-oxide;
3-N-[2-[(5-methylaminomethyl-2-furanyl)methylthio]ethyl]amino-4-amino-1,2,5-thiadiazole-1,1-dioxide;
3-N-[2-[(2-guanidino-4-thiazolyl)methylthio]ethyl]amino-4-methylamino-1,2,5-thiadiazole-1,1-dioxide;
3-N-[2-[(2-guanidino-4-thiazolyl)methylthio]ethyl]amino-4-amino-1,2,5-thiadiazole-1-oxide;
3-N-[2-[(2-guanidino-4-thiazolyl)methylthio]ethyl]amino-4-methylamino-1,2,5-thiadiazole-1-oxide; and,
3-N-[2-[(6-(4-morpholinylmethyl-2-benzofuranyl)methylthio]ethyl]amino-4-amino-1,2,5-thiadiazole-1-oxide.

3. Process according to claim 1 characterized in that a compound of formula I is prepared which is the 3-N-[3-[3-(1-piperidinylmethyl)phenoxy]propyl]amino-4-amino-1,2,5-thiadiazole-1-oxide.

4. Process according to claim 3 characterized in that 3-N-[3-[3-(1-piperidinylmethyl)phenoxy]propyl]-amino-4-amino-1,2,5-thiadiazole-1-oxide is prepared by reacting 3-ethoxy-4-amino-1,2,5-thiadiazole-1-oxide with 3-[3-(1-piperidinylmethyl)phenoxy]propane-amine yielding the desired final product.

5. Process for the preparation of compounds of formulae:

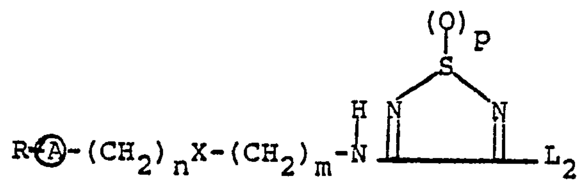

(VI)

and

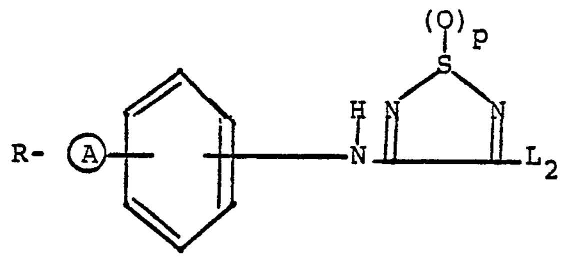

(VI A)

wherein R, Ⓐ, n, X, and m are as defined in claim 1; p is 1 or 2;
$L_2$ is alkoxy having 1—5 carbon atoms, alkylthio having 1—5 carbon atoms, phenoxy, phenylthio or halogen; and the salts thereof, characterized in that a compound having the formula

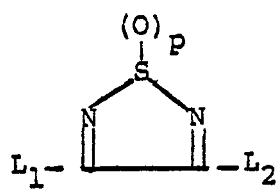

wherein $L_1$ is a leaving group is reacted with an amine having the formulae

R-Ⓐ-$(CH_2)_n$-X-$(CH_2)_m$-$NH_2$

or

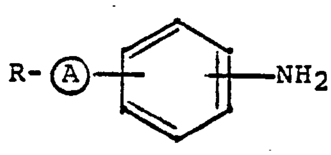

and that the compounds of formulae VI or VIA or salts thereof are isolated.

6. Process according to claim 5 characterized in that compounds are prepared which are members selected from the group consisting of:
3-N-[2-[5-dimethylaminomethyl-2-furanyl)methylthio]ethyl]amino-4-ethoxy-1,2,5-thiadiazole-1-oxide and
3-N-[2-[(5-dimethylaminomethyl-2-furanyl)methylthio]ethyl]amino-4-ethoxy-1,2,5-thiadiazole-1,1-dioxide.

7. Process for the preparation of compounds having the formulae

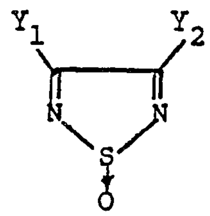

(XX)

wherein

$Y_1$ and $Y_2$ are members selected from the following groups:

a) $Y_1$ and $Y_2$ are the same and are alkoxy having 1—5 carbon atoms, alkylthio having 1—5 carbon atoms, phenoxy or phenylthio; or,

b) $Y_1$ and $Y_2$ are different and one of $Y_1$ and $Y_2$ is alkoxy having 1—5 carbon atoms, phenoxy or phenylthio and the other of $Y_1$ and $Y_2$ is a group having the formula:

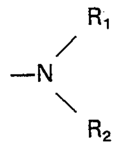

wherein $R_1$ and $R_2$ are independently as defined in claim 1 or $R_1$ and $R_2$ form together with the nitrogen atom to which they are attached a group as defined in claim 1, and

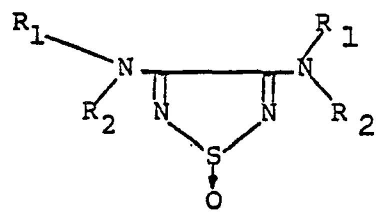

(XXI)

wherein $R_1$ and $R_2$ are independently as defined in claim 1 or

$R_1$ and $R_2$ form together with the nitrogen atom to which they are attached a group as defined in claim 1, characterized in that a compound having the formula

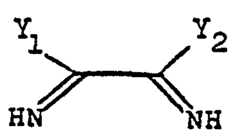

wherein $Y_1$ and $Y_2$ are as defined in a) is treated with thionyl chloride in the presence of a base to form a compound having the formula:

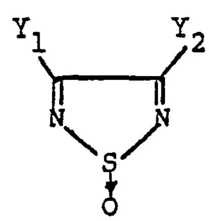

(XX)

which is treated with an amine having the formula:

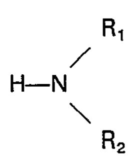

to form compounds wherein $Y_1$ and $Y_2$ are as defined in b), or treating a compound having the formula:

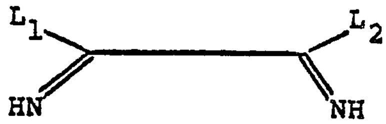

wherein $L_1$ and $L_2$ are leaving groups selected from the group consisting of alkoxy having 1—5 carbon atoms, alkylthio having 1—5 carbon atoms, phenoxy, or phenylthio, with thionyl chloride in the presence of a base to form an intermediate compound having the formula:

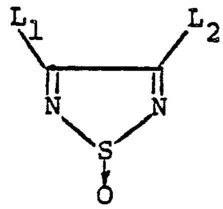

which is reacted with an amine of formula

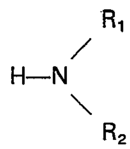

or sequentially with two different amines having said formula to yield a compound having the formula XXI

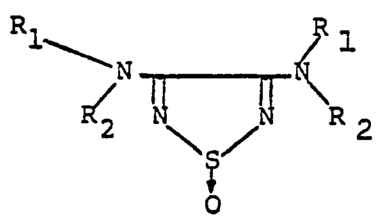

(XXI)

8. Process according to claim 7 characterized in that compounds of formulae XX or XXI are prepared which are members selected from the group consisting of
3,4-diethoxy-1,2,5-thiadiazole-1-oxide;
3,4-bis-methylthio-1,2,5-thiadiazole-1-oxide;
3-isopropylamino-4-methylthio-1,2,5-thiadiazole-1-oxide;
3-ethoxy-4-amino-1,2,5-thiadiazole-1-oxide;
3-ethoxy-4-methylamino-1,2,5-thiadiazole-1-oxide;
3-ethoxy-4-(1-pyrrolidinyl)-1,2,5-thiadiazole-1-oxide;
3-ethoxy-4-(4-morpholino)-1,2,5-thiadiazole-1-oxide;
3,4-diamino-1,2,5-thiadiazole-1-oxide;
3-amino-4-(2-dimethylaminoethyl)amino-1,2,5-thiadiazole-1-oxide;
3-amino-4-methylamino-1,2,5-thiadiazole-1-oxide;
3-amino-4-dodecylamino-1,2,5-thiadiazole-1-oxide;
3-amino-4-(4-fluoroanilino)-1,2,5-thiadiazole-1-oxide;
3-amino-4-morpholino-1,2,5-thiadiazole-1-oxide; and,
3-morpholino-4-(2-pyridinomethyl)amino-1,2,5-thiadiazole-1-oxide.

9. Process for the preparation of compounds having the formulae

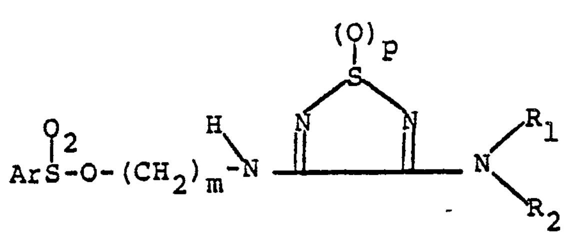

(XVII)

wherein

Ar is aryl and $R_1$, $R_2$, m and p are as defined in claim 1 and

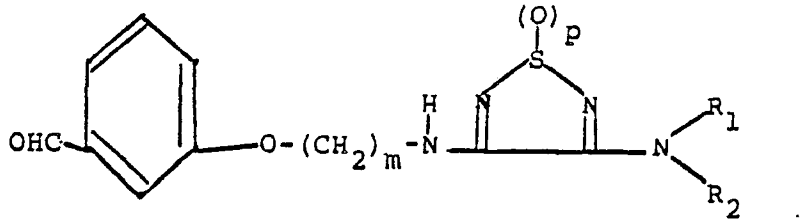

(XIX)

wherein

$R_1$, $R_2$, m and p are as defined in claim 1, characterized in that a compound of formula

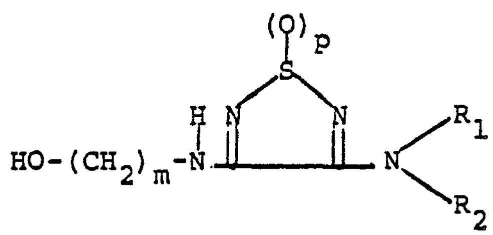

(XVI)

is reacted in the presence of a base with a compound having the formula

$ArSO_2Cl$

yielding the compound of formula XVII, which is optionally reacted with a compound having the formula XVIII

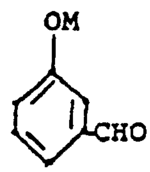

(XVIII)

yielding a compound of formula XIX.

10. Process for the preparation of a composition useful for suppressing gastric acid secretions in an animal with excess gastric acid secretions characterized in that a compound of formulae I, II, III, IIIa, IIIb, IVa or IVb or solvates of said compounds or solvates of the salt of said compounds as well as quaternary ammonium salts and N-oxides thereof prepared according to the process of patent claim 1 are formulated with an inert carrier.

**Patentansprüche für die Vertragsstaaten: BE CH DE FR GB IT LI LU NL SE**

1. Verbindungen der Formel

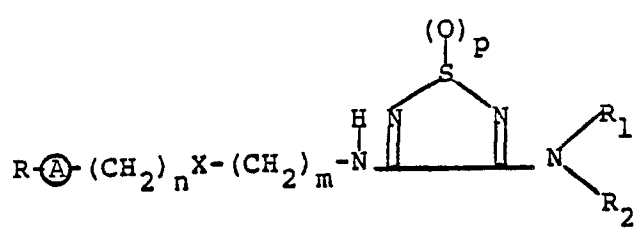

(I)

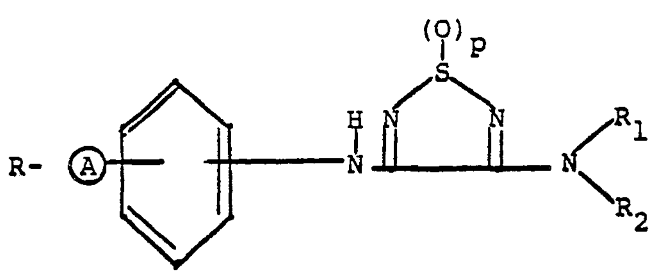

(II)

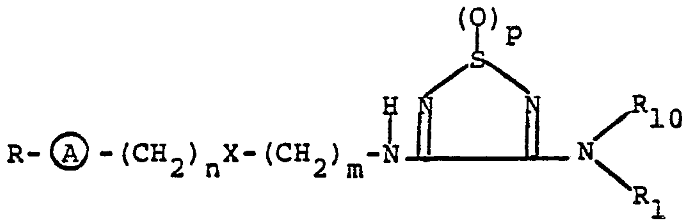

(IIIA)

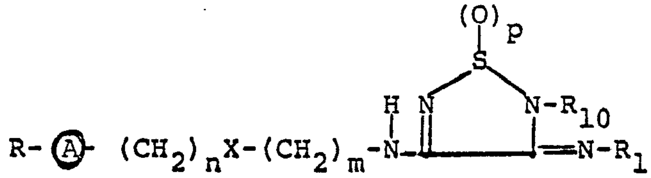

(IIIB)

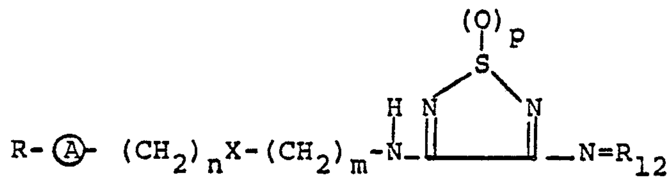

(IVA)

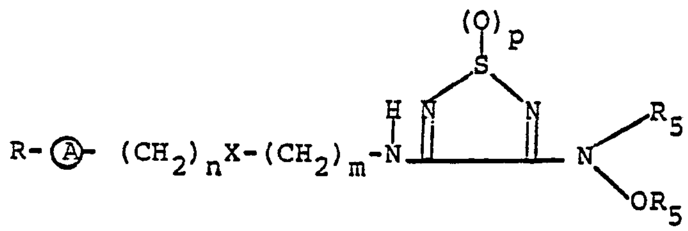

(IVB)

worin R Wasserstoff, Alkyl mit 1 bis 5 C-Atomen oder eine Gruppe von der Formel

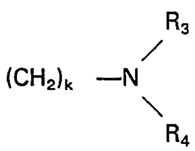 oder 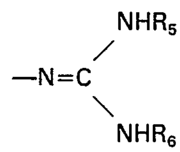

ist,

worin

$R_3$ und $R_4$ unabhängig Wasserstoff, Alkyl mit 1 bis 5 C-Atomen, Cycloalkyl mit 3 bis 7 C-Atomen oder Phenylalkyl sind, worin die Alkylgruppe 1 bis 5 C-Atome hat, oder

$R_3$ und $R_4$ zusammen mit dem Stickstoffatom, an das sie gebunden sind, einen 5- oder 6-gliedrigen Heterozyklus bilden, der auch ein Sauerstoff-, Schwefel- oder N—$R_7$-Heteroatom enthalten kann, worin $R_7$ Wasserstoff, Alkyl mit 1 bis 3 C-Atomen oder Benzyl ist;

$R_5$ und $R_6$ unabhängig Wasserstoff oder Alkyl mit 1 bis 5 C-Atomen sind,

n 0 oder 1,

m 2 bis 4,

k 1 bis 4,

X Sauerstoff, Schwefel oder Methylen,

p 1 oder 2 ist;

$R_1$ und $R_2$ unabhängig Wassterstoff, Alkyl mit 1 bis 5 C-Atomen, Alkenyl mit 3 bis 5 C-Atomen, Alkynyl mit 3 bis 5 C-Atomen, Cycloalkyl mit 3 bis 7 C-Atomen, Phenyl, Pyridyl und substituiertes Alkyl mit 1 bis 5 C-Atomen in der Alkylgruppe, worin der Substituent Phenyl, Cycloalkyl mit 3 bis 7 C-Atomen, Pyridyl, Imidazolyl, Morpholin, Hydroxy, Alkoxy mit 1 bis 5 C-Atomen, Alkylthio mit 1 bis 5 C-Atomen oder Dialkylamino ist, worin jede Alkylgruppe 1 bis 5 C-Atome hat, oder

$R_1$ und $R_2$ zusammen mit dem Stickstoff, an das sie gebunden sind, einen 5- oder 6-gliedrigen Heterozyklusbilden, der auch ein Sauerstoff-, Schwefel- oder N—$R_7$-Heteroatom enthalten kann, worin $R_7$ wie oben definiert ist;

Ⓐ Phenylen oder ein 5- oder 6-gliedriger Heterozyklus ist, der ein bis drei Heteroatome aus der Gruppe Sauerstoff, Schwefel oder Stickstoff enthält, der gegebenenfalls mit einem Benzolring kondensiert ist;

vorausgesetzt, dass wen Ⓐ in Formel I wie oben ein 5-gliedriger Heterozyklus oder ein mit Benzol kondensierter 5-gliedriger Heterozyklus ist, n 1 ist, und

vorausgesetzt ferner, dass wenn Ⓐ in Formel I oder II ein Furan von der Struktur

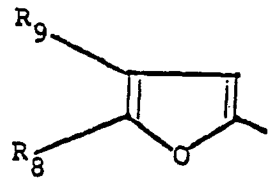

ist,

$R_8$ die gleiche Bedeutung wie oben für R definiert hat und

$R_9$ Alkyl mit 1 bis 5 C-Atomen, Alkoxyalkyl, in welchem die Alkoxygruppe und die Alkylgruppe jeweils 1 bis 5 C-Atome enthalten, Alkoxycarbonyl, in welcher die Alkoxygruppe 1 bis 5 C-Atome hat, Halogen, vorzugsweise Chlor oder Brom, oder Aryl, vorzugsweise Phenyl,

$R_{10}$ Alkanoyl, in welchem die Alkylgruppe 1 bis 5 C-Atome enthält, eine Phenylcarbonylgruppe, in welcher der Phenylkern gegebenenfalls mit 1, 2 oder 3 Substituenten, ausgewählt aus der Gruppe Halogen, Alkyl und Alkoxy, die beide 1 bis 5 C-Atome enthalten, Heteroaroyl, in welcher der heterocyclische Ring ein 6-gliedriger N-enthaltender Ring mit 1 bis 2 Heteroatomen is, Alkylsulfonyl, in welcher die Alkylgruppe 1 bis 5 C-Atome enthält, Arylsulfonyl, Heteroarylsulfonyl, in welcher der heterocyclische Ring ein 6-gliedriger N-enthaltender Ring mit 1 bis 2 Heteroatomen ist, Cyan, Amidin, Dialkylcarbamoyl, in welcher die Alkylgruppe 1 bis 5 C-Atome enthält, Dialkylsulfamoyl, in welcher die Alkylgruppe 1 bis 5 C-Atome enthält, Alkoxycarbonyl, in welcher die Alkoxygruppe 1 bis 5 C-Atome enthält, oder eine Gruppe der Formel $-(CH_2)_yCOR_{11}$, in der

y 1 oder 2 und

$R_{11}$ ein Alkyl mit 1 bis 5 C-Atomen, Hydroxy, Alkoxy mit 1 bis 5 C-Atomen, Amino, Alkylamino, in der die Alkylgruppe 1 bis 5 C-Atome hat, oder Dialkylamino, in der die Alkylgruppe jeweils 1 bis 5 C-Atome hat, und

$R_{12}$ Aralkyliden oder Diarylmethyliden ist und die Salze der Verbindungen von Formel, I, II, IIIa, IIIb, IVa und IVb und Solvate dieser Verbindungen oder Solvate der Salze dieser Verbindungen sowie quartäre Ammoniumsalze und N-Oxide sind.

2. Die Verbindungen von Anspruch 1, ausgewählt aus der Gruppe von:

3-N[3-(2-Guanidino-4-thiazolyl)phenyl]amino-4-methylamino-1,2,5-thiadiazol-1-oxid;
3-N(3-2-Guanidino-4-thiazolyl)phenyl]amino-4-amino-1,2,5-thiadiazol-1-oxid;
3-N-[3-[3-[Dimethylaminomethyl)phenoxy]propyl]amino-4-amino-1,2,5-thiadiazol-1-oxid;
3-N-[3-[3-(Dimethylaminomethyl)phenoxy]propyl]amino-4-methylamino-1,2,5-thiadiazol-1-oxid;
3-N-[3-[3-(Dimethylaminomethyl)phenoxy]propyl]amino-4-amino-1,2,5-thiadiazol-1,1-dioxid;
3-N-[2-[(2-Furanyl)methylthio]ethyl]amino-4-amino-1,2,5-thiadiazol-1-oxid;
3-N-[2-[(5-Dimethylaminomethyl-2-thienyl)methylthio]ethyl]amino-4-amino-1,2,5-thiadiazol-1-oxid;
3-N-[2-[(4-methyl-5-oxazolyl)methylthio]ethyl]amino-4-amino-1,2,5-thiadiazol-1-oxid;
3-N-[2-[(2-Furanyl)methylthio]ethyl]amino-4-amino-1,2,5-thiadiazol-1,1-dioxid;
3-N-[2-[[5-(1-Piperidinylmethyl)-2-furanyl]methylthio]ethyl]amino-4-amino-1,2,5-thiadiazol-1-oxid;
3-N-[2-[(5-Dimethylaminomethyl-2-furanyl)methylthio]ethyl]amino-4-amino-1,2,5-thiadiazol-1-oxid;
3-N-[2-[(5-Dimethylaminomethyl-2-furanyl)methylthio]ethyl]amino-4-methylamino-1,2,5-thiadiazol-1-oxid;
3-N-[2-[(5-Dimethylaminomethyl-2-furanyl)methylthio]ethyl]amino-4-methylamino-1,2,5-thiadiazol-1,1-oxid;
3-N-[2-[(5-Dimethylaminomethyl-2-furanyl)methylthio]ethyl]amino-4-amino-1,2,5-thiadiazol-1,1-dioxid;
3-N-[2-[(5-Methylaminomethyl-2-furanyl)methylthio]ethyl]amino-4-amino-1,2,5-thiadiazol-1-oxid;
3-N-[2-[(5-Methylaminomethyl-2-furanyl)methylthio]ethyl]amino-4-amino-1,2,5-thiadiazol-1,1-dioxid;
3-N-[2-[(2-Guanidino-4-thiazolyl)methylthio]ethyl]amino-4-methylamino-1,2,5-thiadiazol-1,1-dioxid;
3-N-[2-[(2-Guanidino-4-thiazolyl)methylthio]ethyl]amino-4-amino-1,2,5-thiadiazol-1-oxid;
3-N-[2-[(2-Guanidino-4-thiazolyl)methythio]ethyl]amino-4-methylamino-1,2,5-thiadiazol-1-oxid, und
3-N-[2[[(6-(4-Morpholinylmethyl-2-benzofuranyl)methylthio]ethyl]-amino-4-amino-1,2,5-thiadiazol-1-oxid.

3. Eine Verbindung der Formel I von Anspruch 1, nämlich das 3-N-[3-[3-(1-Piperidinylmethyl)phenoxy]propyl]amino-4-amino-1,2,5-thiadiazol-1-oxid.

4. Verbindungen der Formeln;

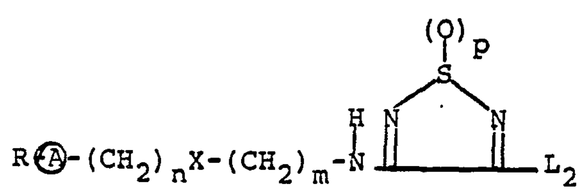

(VI)

und

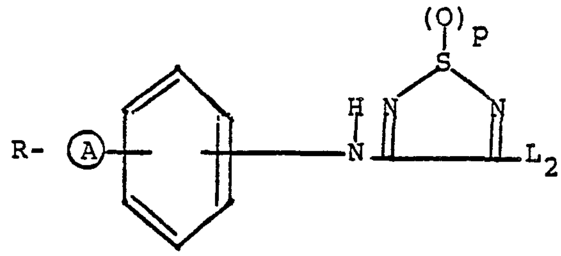

(VI A)

worin R, Ⓐ, n, X und m wie in Anspruch 1 definiert sind, p 1 oder 2, $L_2$ Alkoxy mit 1 bis 5 C-Atomen, Alkylthio mit 1 bis 5 C-Atomen, Phenoxy, Phenylthio oder Halogen ist; und die Salze derselben.

5. Die Verbindungen von Anspruch von Anspruch 4, ausgewählt aus:

3-N-[2-[(5-Dimethylaminomethyl-2-furanyl)methylthio]ethyl]amino-4-ethoxy-1,2,5-thiadiazol-1-oxid und

3-N-[2-[(5-DImethylaminomethyl-2-furanyl)methylthio]ethyl]amino-4-ethoxy-1,2,5-thiadiazol-1,1-dioxid.

6. Verbindungen der Formel

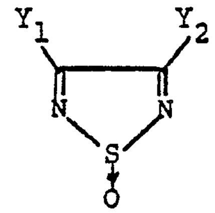

(XX)

worin $Y_1$ und $Y_2$ ausgewählt sind aus den folgenden Gruppen;

a) $Y_1$ und $Y_2$ sind gleich und sind Alkoxy mit 1 bis 5 C-Atomen, Alkylthio mit 1 bis 5 C-Atomen, Phenoxy oder Phenylthio, oder

b) $Y_1$ und $Y_2$ sind underschiedlich und eines von $Y_1$ und $Y_2$ is Alkoxy mit 1 bis 5 C-Atomen, Phenoxy oder Phenylthio, und das andere von $Y_1$ und $Y_2$ is eine Gruppe der Formel

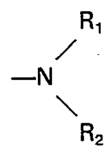

worin $R_1$ und $R_2$ unabhängig wie in Anspruch 1 definiert sind oder $R_1$ und $R_2$ zusammen mit dem Stickstoffatom, an das sie gebunden sind, eine Gruppe wie in Anspruch 1 definiert bilden, und

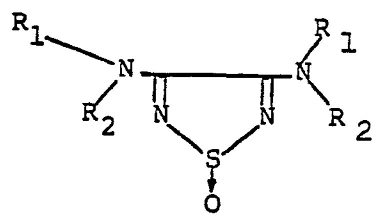

(XXI)

worin $R_1$ und $R_2$ unabhängig wie in Anspruch 1 definiert sind oder $R_1$ und $R_2$ zusammen mit dem Stickstoffatom, an das sie gebunden sind, eine Gruppe wie in Anspruch 1 definiert bilden.

7. Verbindugen der Formel

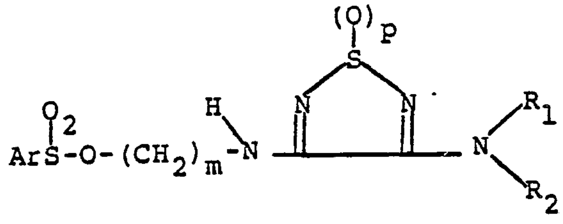

. (XVII)

worin Ar Aryl ist und $R_1$, $R_2$, m und p wie in Anspruch 1 definiert sind und

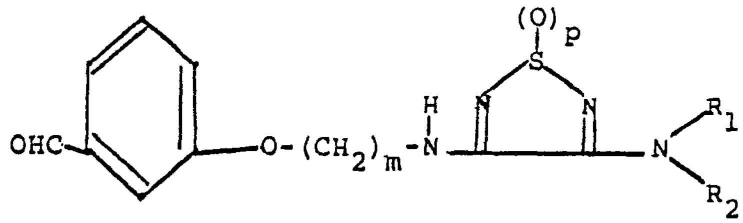

(XIX)

worin $R_1$, $R_2$, m und p wie in Anspruch 1 definiert sind.

8. Verbindungen von Anspruch 6, die Verbindungen der Formel XX oder XXI sind und Glieder ausgewählt aus der Gruppe:

3,4-Diethoxy-1,2,5-thiadiazol-1-oxid;
3,4-Bis-methylthio,1,2,5-thiadiazol-1-oxid;
3-Isopropylamino-4-methylthio-1,2,5-thiadiazol-1-oxid;
3-Ethoxy-4-amino,1,2,5-thiadiazol-1-oxid;
3-Ethoxy-4-methylamino-1,2,5-thiadiazol-1-oxid;
3-Ethoxy-4-(1-pyrrolidinyl)-1,2,5-thiadiazol-1-oxid;
3-Ethoxy-4-(4-morpholin)-1,2,5-thiadiazol-1-oxid;
3,4-Diamino-1,2,5-thiadiazol-1-oxid;
3-Amino-4-(2-dimethylaminoethyl)amino-1,2,5-thiadiazol-1-oxid;
3-Amino-4-methylamino-1,2,5-thiadiazol-1-oxid;
3-Amino-4-dodecylamino-1,2,5-thiadiazol-1-oxid;
3-Amino-4-(4-fluoranilino)-1,2,5-thiadiazol-1-oxid;
3-Amino-4-Morpholino-1,2,5-thiadiazol-1-oxid; und
3-Morpholino-4-(2-pyridinomethyl)amino-1,2,5-thiadiazol-1-oxid.

9. Ein Verfahren zur Herstellung einer Verbindung von Formel I, II, IVA oder IVB gemäss Anspruch 1 durch Behandlung einer Verbindung der Formel

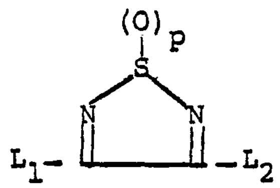

worin p 1 oder 2 ist und $L_1$ und $L_2$ austretende Gruppen sind, erstens mit einem der Amine von der Formel

R-Ⓐ-$(CH_2)_n$-X-$(CH_2)_m$-$NH_2$

oder 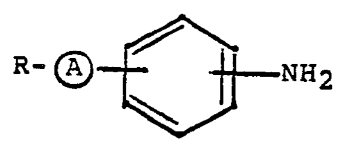

oder 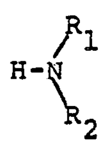

woring R, Ⓐ, n, X, m, $R_1$ und $R_2$ wie in Anspruch 1 definiert sind, unter Entstehung eines Zwischenproduktes von Formel VI

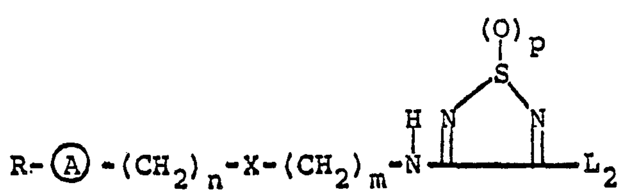

(VI)

oder VIa

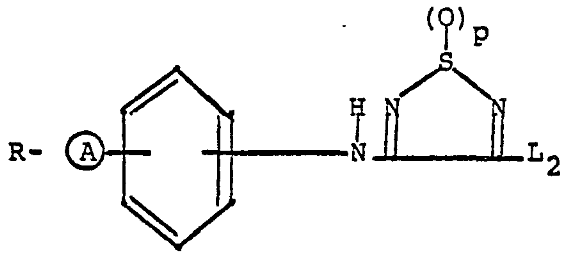

(VIa)

oder

VII

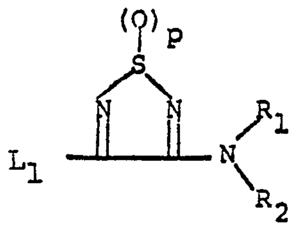

(VII)

und Umsetzung des Zwischenproduktes von Formel VI und VIa mit dem Amin der Formel

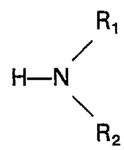

oder dem Zwischenprodukt von Formel VII mit entweder dem Amin

R-Ⓐ-$(CH_2)_n$-X-$(CH_2)_m$-$NH_2$

oder dem Amin

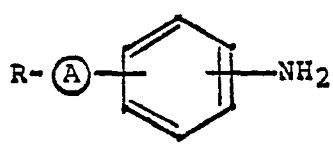

unter Entstehung der Endprodukte von Formel I oder II, und eventuelle Umsetzung einer Verbindung der Formel I oder II, in der die Gruppe

R—Ⓐ—

ein unsubstituierter Furanring ist mit entweder

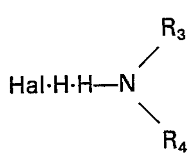 + $CH_2O$ oder 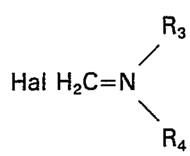

worin Hal Halogen ist, um die entsprechenden Verbindungen der Formel I oder II zu erhalten, in denen Ⓐ eine Furanring ist, der mit einer Gruppe R der Formel

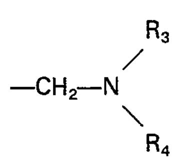

substituiert ist, in der $R_3$ und $R_4$ wie in Anspruch 1 definiert sind, oder Umsetzung des Zwischenproduktes von Formel VI

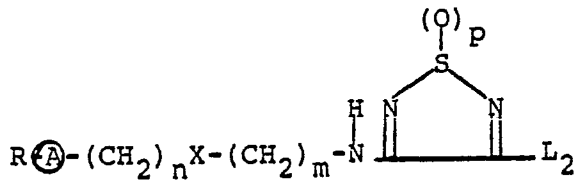

(VI)

mit einem Diamin der Formel

$$H—N=R_{12}$$

um Verbindungen der Formel IVa

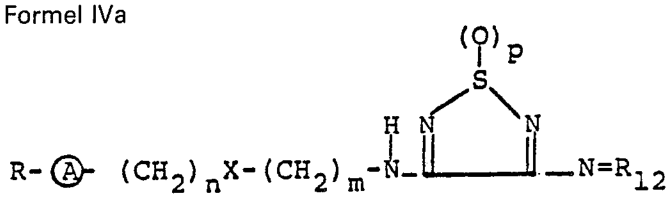

(IVA)

zu erhalten, oder Umsetzung des Zwischenproduktes von Formel VI

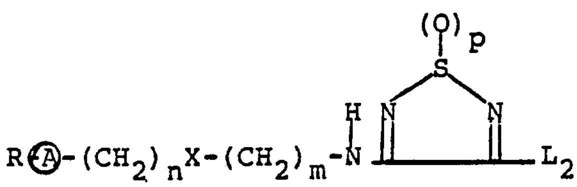

(VI)

mit einem gegebenenfalls substituierten Hydroxylamin der Formel

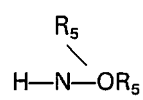

um Verbindungen der Formel IVb zu erhalten

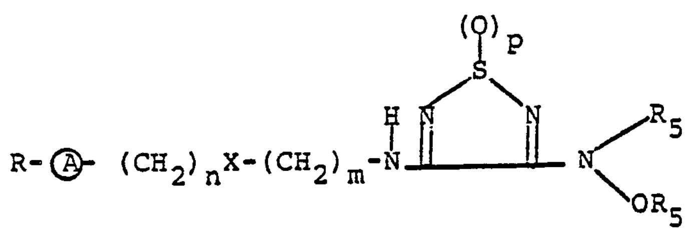

(IVB)

in der R, $R_5$, Ⓐ, X, m, n und p wie in Anspruch 1 definiert sind und dass die Verbindungen von Formel I, II, IVa oder IVb als freie Basen oder Salze oder als Solvate der freien Basen oder der Salze isoliert oder in quartäre Ammoniumsalze oder N-Oxide dieser Verbindungen umgewandelt werden.

10. Verfahren zur Herstellung von 3-N-[3-[3-(1-Piperidinylmethyl)phenoxy]propyl]amino-4-amino-1,2,5-thiadiazol-1-oxid gemäss Anspruch 3, dadurch gekennzeichnet, dass 3-Ethoxy-4-amino-1,2,5-thiadiazol-1-oxid mit 3-[3-(1-Piperidinylmethyl)phenoxy]propan-amin umgesetzt wird, um das gewünschte Endprodukt zu ergeben.

11. Verfahren zur Herstellung von Verbindungen der Formel I, II, IIIa und IIIb gemäss Anspruch 1 durch Umsetzung eines Alkohols der Formel XVI

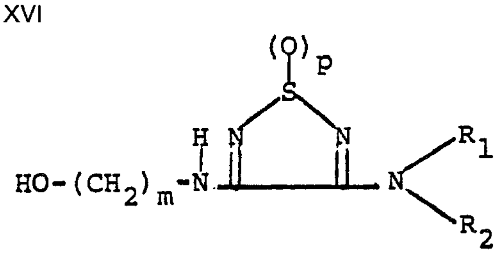

(XVI)

woring $R_1$, $R_2$ und p wie in Anspruch 1 definiert sind und $m > 2$ ist, mit einem Acylierungsmittel, um ein aktiviertes Ester-Zwischenprodukt zu erhalten; Umsetzung dieses Ester-Zwischenproduktes mit einem Alkalisalz von 3-Hydroxybenzaldehyd, um ein Zwischenprodukt der Formel

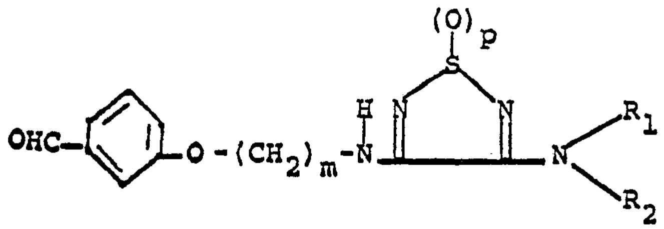
(XIX)

zu erhalten, und Reduktion des genannten Zwischenproduktes in Anwesenheit von Amin $R_3NHR_4$, worin $R_3$ und $R_4$ wie in Anspruch 1 definiert sind; oder Umsetzung des genannten Ester-Zwischenproduktes, wenn $m = 2$, mit einem Mercaptan der Formel R—Ⓐ—$(CH_2)_n$—SH, worin R, Ⓐ und n wie in Anspruch 1 definiert sind; oder Umsetzung eines Alkohols der Formel

R—Ⓐ—$(CH_2)_n$—X—$CH_2CH_2OH$

worin R, Ⓐ, X und n wie in Anspruch 1 definiert sind, mit, nach Aktivierung des Alkohols gegen Nucleophile, einer Aminoverbindung der Formel

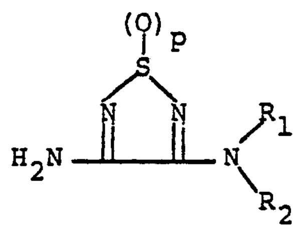

worin $R_1$, $R_2$ und p wie in Anspruch 1 definiert sind; oder Umsetzung eines Mercaptans der Formel

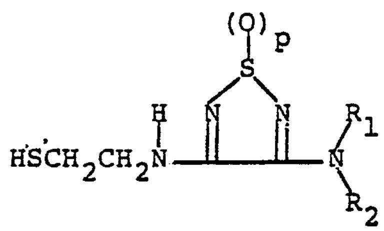

worin $R_1$, $R_2$ und p wie in Anspruch 1 definiert sind, mit einem Alkohol der Formel

R—Ⓐ—$(CH_2)_n$—OH,

worin R, Ⓐ und n wie in Anspruch 1 definiert sind, in Anwesenheit einer Mineralsäure, um die Verbindungen von Anspruch 1 zu erhalten; und die physiologisch akzeptablen Salze derselben; oder Umsetzung der Verbindungen, wie in Anspruch 1 definiert, und ihrer Tautomeren mit einem Alkylierungsmittel, einem Acylierungsmittel oder einem Sulfonierungsmittel, um Derivate der Formel

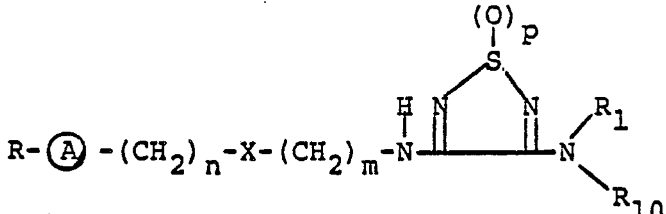
(IIIA)

und

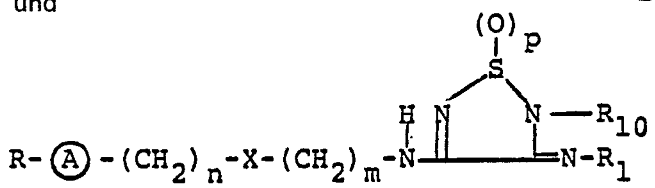
(IIIB)

zu erhalten, worin R, $R_{10}$, $R_1$, Ⓐ, X, m, n und p wie in Anspruch 1 definiert sind, und die physiologisch akzeptablen Salze derselben.

12. Verfahren zur Herstellung von Verbindungen von Anspruch 6 durch Behandlung einer Verbindung der Formel

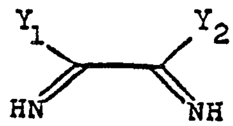

worin $Y_1$ und $Y_2$ wie in a) von Anspruch 6 definiert sind, mit Thionylchlorid in Anwesenheit einer Base, um eine Verbindung der Formel

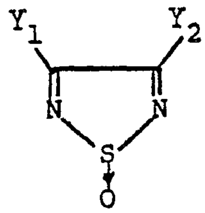

(XX)

zu erhalten, die mit einem Amin der Formel

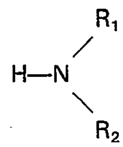

behandelt wird, worin $R_1$ und $R_2$ wie in Anspruch 6 definiert sind, um Verbindungen zu erhalten, in denen $Y_1$ und $Y_2$ wie in b) von Anspruch 6 definiert sind, oder Behandlung einer Verbindung der Formel

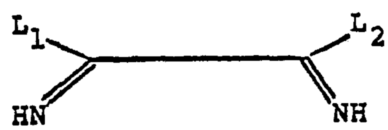

worin $L_1$ und $L_2$ austretende Gruppen sind, ausgewählt aus der Gruppe der Alkoxygruppen mit 1 bis 5 C-Atomen, Alkylthio mit 1 bis 5 C-Atomen, Phenoxy, oder Phenylthio, mit Thionylchlorid in Anwesenheit einer Base, um eine Zwischenverbindung der Formel

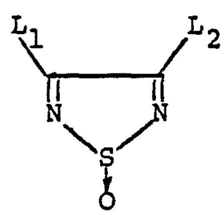

zu erhalten, die mit einem Amin der Formel

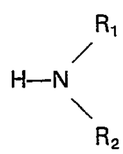

oder nacheinander mit zwei unterschiedlichen Aminen dieser Formel umgesetzt wird, worin

$R_1$ und $R_2$ unabhängig wie in Anspruch 6 definiert sind oder

$R_1$ und $R_2$ zusammen mit dem Stickstoffatom, an das sie gebunden sind, eine Gruppe wie in Anspruch 6 definiert bilden, um eine Verbindung der Formel XXI

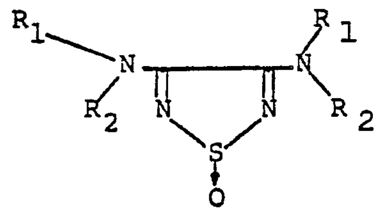

(XXI)

zu erhalten, in der $R_1$ und $R_2$ wie in Anspruch 6 definiert sind.

13. Eine Zusammensetzung, brauchbar für die Unterdrückung gastrischer saurer Sekretionen in einem Tier mit überschüssigen gastrischen sauren Sekretionen, die einen inerten Träger und eine wirksame Menge einer Verbindung von Anspruch 1 umfasst.

**Patentansprüche für der Vertragsstaat: AT**

1. Verfahren zur Herstellung von Verbindungen der Formel

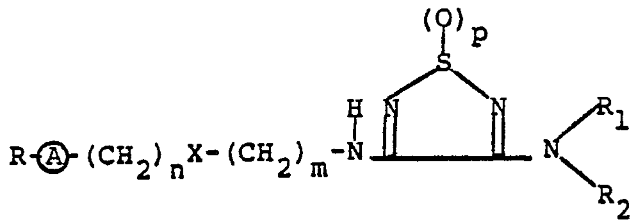

(I)

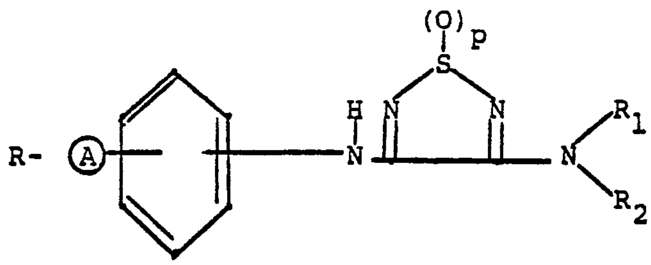

(II)

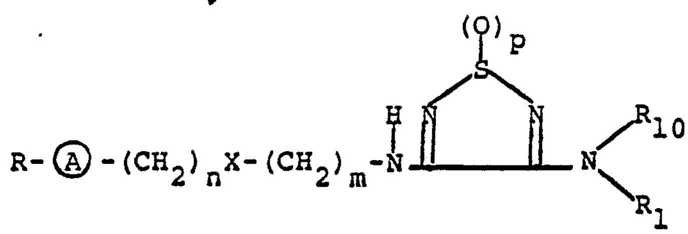

(IIIA)

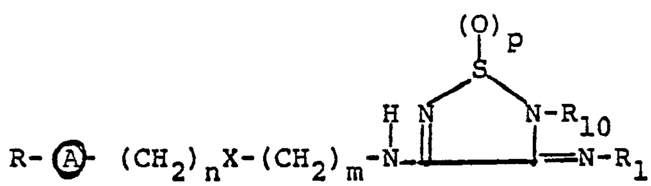

(IIIB)

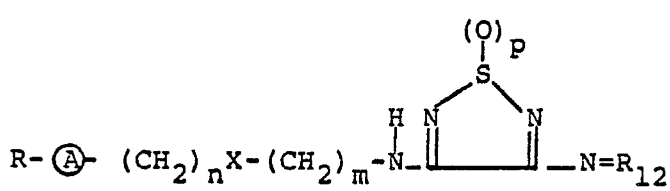

(IVA)

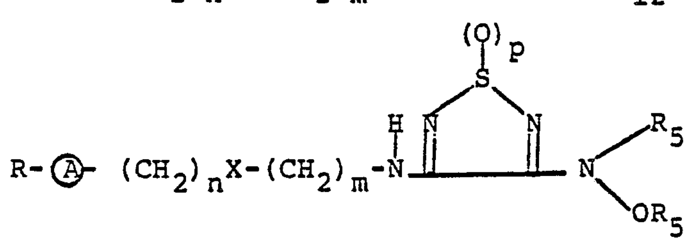

(IVB)

worin R Wasserstoff, Alkyl mit 1 bis 5 C-Atomen oder eine Gruppe von der Formel

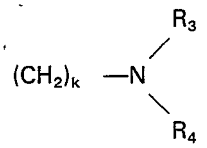 oder 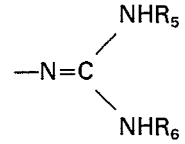 ist,

worin

$R_3$ und $R_4$ unabhängig Wasserstoff, Alkyl mit 1 bis 5 C-Atomen, Cycloalkyl mit 3 bis 7 C-Atomen oder Phenylalkyl sind, worin die Alkylgruppe 1 bis 5 C-Atome hat, oder

$R_3$ und $R_4$ zusammen mit dem Stickstoffatom, an das sie gebunden sind, enen 5- oder 6-gliedrigen Heterozyklus bilden, der auch ein Sauerstoff-, Schwefel- oder N—$R_7$-Heteroatom enthalten kann, worin $R_7$ Wasserstoff, Alkyl mit 1 bis 3 C-Atomen oder Benzyl ist;

$R_5$ und $R_6$ unabhängig Wasserstoff oder Alkyl mit 1 bis 5 C-Atomen sind,

n 0 oder 1,

m 2 bis 4,

k 1 bis 4,

X Sauerstoff, Schwefel oder Methylen,

p 1 oder 2 ist;

$R_1$ und $R_2$ unabhängig Wasserstoff, Alkyl mit 1 bis 5 C-Atomen, Alkenyl mit 3 bis 5 C-Atomen, Alkynyl mit 3 bis 5 C-Atomen, Cycloalkyl mit 3 bis 7 C-Atomen, Phenyl, Pyridyl und substituiertes Alkyl mit 1 bis 5 C-Atomen in der Alkylgruppe, worin der Substituent Phenyl, Cycloalkyl mit 3 bis 7 C-Atomen, Pyridyl, Imidazolyl, Morpholin, Hydroxy, Alkoxy mit 1 bis 5 C-Atomen, Alkylthio mit 1 bis 5 C-Atomen oder Dialkylamino ist, worin jede Alkylgruppe 1 bis 5 C-Atome hat, oder

$R_1$ und $R_2$ zusammen mit dem Stickstoff, an das sie gebunden sind, einen 5- oder 6-gliedrigen Heterozyklusbilden, der auch ein Sauerstoff-, Schwefel- oder N—$R_7$-Heteroatom enthalten kann, worin $R_7$ wie oben definiert ist;

Ⓐ Phenylen oder ein 5- oder 6-gliedriger Heterozyklus ist, der ein bis drei Heteroatome aus der Gruppe Sauerstoff, Schwefel oder Stickstoff enthält, der gegebenenfalls mit einem Benzolring kondensiert ist;

vorausgesetzt, dass wen Ⓐ in Formel I wie oben ein 5-gliedriger Heterozyklus oder ein mit Benzol kondensierter 5-gliedriger Heterozyklus ist, n 1 ist, und

vorausgesetzt ferner, dass wenn Ⓐ in Formel I oder II ein Furan von der Struktur

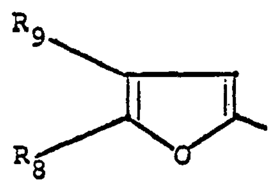

ist,

$R_8$ die gleiche Bedeutung wie oben für R definiert hat und

$R_9$ Alkyl mit 1 bis 5 C-Atomen, Alkoxyalkyl, in welchem die Alkoxygruppe und die Alkylgruppe jeweils 1 bis 5 C-Atome enthalten, Alkoxycarbonyl, in welcher die Alkoxygruppe 1 bis 5 C-Atome hat, Halogen, vorzugsweise Chlor oder Brom, oder Aryl, vorzugsweise Phenyl,

$R_{10}$ Alkanoyl, in welchem die Alkylgruppe 1 bis 5 C-Atome enthält, eine Phenylcarbonylgruppe, in welcher der Phenylkern gegebenenfalls mit 1, 2 oder 3 Substituenten, ausgewählt aus der Gruppe Halogen, Alkyl und Alkoxy, die beide 1 bis 5 C-Atome enthalten, Heteroaroyl, in welcher der heterocyclische Ring ein 6-gliedriger N-enthaltender Ring mit 1 bis 2 Heteroatomen is, Alkylsulfonyl, in welcher die Alkylgruppe 1 bis 5 C-Atome enthält, Arylsulfonyl, Heteroarylsulfonyl, in welcher der heterocyclische Ring ein 6-gliedriger N-enthaltender Ring mit 1 bis 2 Heteroatomen ist, Cyan, Amidin, Dialkylcarbamoyl, in welcher die Alkylgruppe 1 bis 5 C-Atome enthält, Dialkylsulfamoyl, in welcher die Alkylgruppe 1 bis 5 C-Atome enthält, Alkoxycarbonyl, in welcher die Alkoxygruppe 1 bis 5 C-Atome enthält, oder eine Gruppe der Formel $-(CH_2)_yCOR_{11}$, in der

y 1 oder 2 und

$R_{11}$ ein Alkyl mit 1 bis 5 C-Atomen, Hydroxy, Alkoxy mit

1 bis 5 C-Atomen, Amino, Alkylamino, in der die Alkylgruppe

1 bis 5 C-Atome hat, oder Dialkylamino, in der die Alkylgruppen jeweils 1 bis 5 C-Atome hat jeweils 1 bis 5 C-Atome haben, und

$R_{12}$ Aralkyliden oder Diarylmethyliden ist und die Salze der Verbindungen von Formel, I, II, IIIa, IIIb, IVa und IVb und Solvate dieser Verbindungen oder Solvate der Salze dieser Verbindungen sowie quartäre Ammoniumsalze und N-Oxide sind, dadurch gekennzeichnet, dass die Verbindungen der Formel I, II, IVa oder IVb hergestellt werden durch Behandlung einer Verbindung der Formel

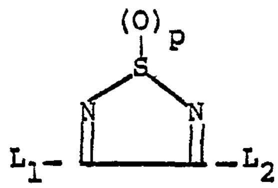

worin $L_1$ und $L_2$ austretende Gruppen sind, erstens mit einem der Amine von der Formel

R–Ⓐ–$(CH_2)_n$–X–$(CH_2)_m$–$NH_2$

oder

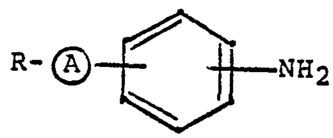

oder

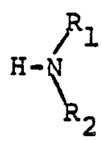

unter Entstehung eines Zwischenproduktes von Formel VI

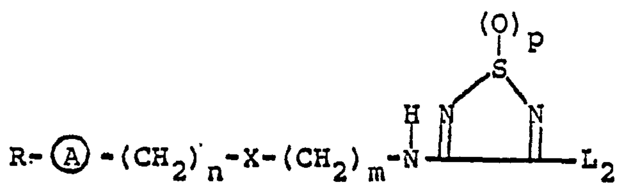

(VI)

oder VIa

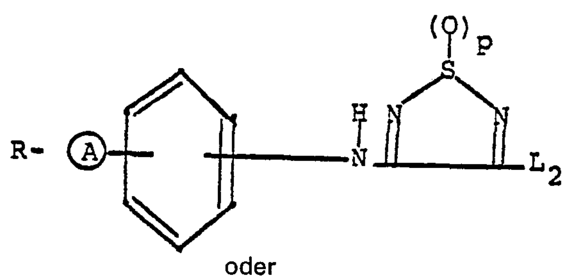

(VIa)

oder

VII

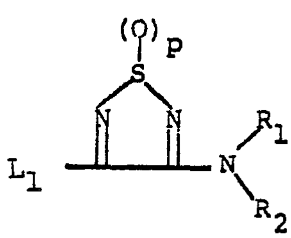

(VII)

und Umsetzung des Zwischenproduktes von Formel VI und VIa mit dem Amin der Formel

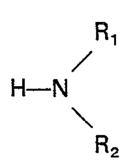

oder dem Zwischenprodukt von Formel VII mit entweder dem Amin

$$R-Ⓐ-(CH_2)_n-X-(CH_2)_m-NH_2$$

oder dem Amin

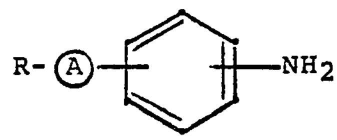

unter Entstehung der Endprodukte von Formel I oder II, und eventuelle Umsetzung einer Verbindung der Formel I oder II, in der die Gruppe

$$R—Ⓐ—$$

ein unsubstituierter Furanring ist mit entweder

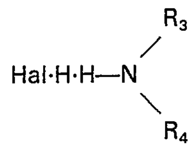

$+ CH_2O$ oder

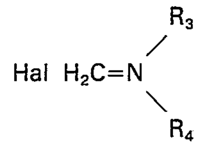

worin Hal Halogen ist, um die entsprechenden Verbindungen der Formel I oder II zu erhalten, in denen Ⓐ ein Furanring ist, der mit einer Gruppe R der Formel

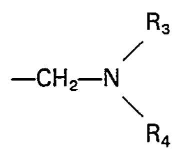

substituiert ist, oder Umsetzung des Zwischenproduktes von Formel VI

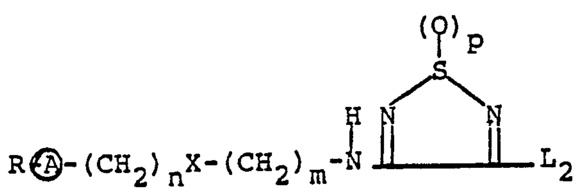

(VI)

mit einem Imin der Formel

$$H—N=R_{12}$$

um Verbindungen der Formel IVa

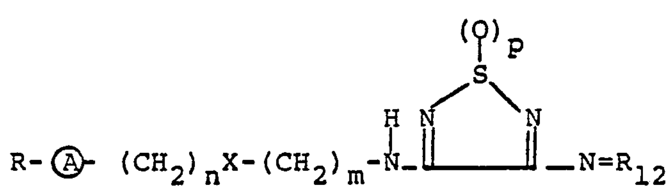

(IVA)

zu erhalten, oder Umsetzung des Zwischenproduktes von Formel VI

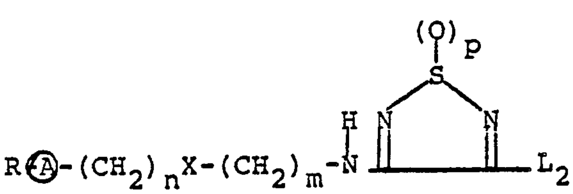

(VI)

mit einem gegebenenfalls substituierten Hydroxylamin der Formel

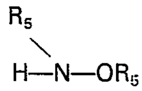

um Verbindungen der Formel IVb zu erhalten

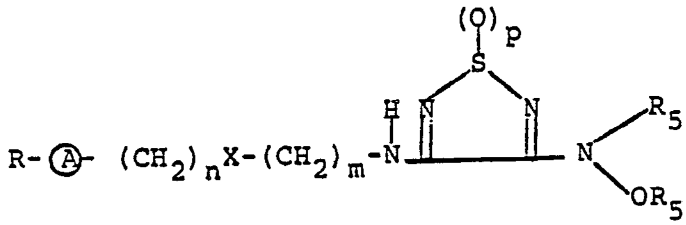

(IVB)

und dass die Verbindungen von Formel I, II, IVa oder IIb als freie Basen oder Salze oder als Solvate der freien Basen oder der Salze isoliert oder in quartäre Ammoniumsalze oder N—Oxide dieser Verbindungen umgewandelt werden, oder dass die Verbindungen der Formel I, II, IIIa und IIIb hergestellt werden durch Umsetzung eines Alkohols der Formel XVI

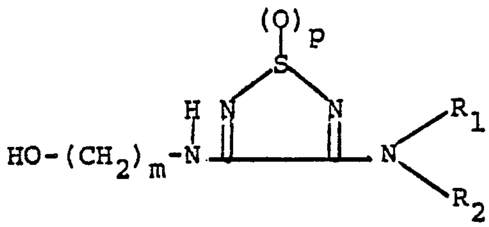

(XVI)

worin m > 2 ist, mit einem Acylierungsmittel, um ein aktiviertes Ester-Zwischenprodukt zu erhalten; Umsetzung dieses Ester-Zwischen-produktes mit einem Alkalisalz von 3-Hydroxybenzaldehyd, um ein Zwischenprodukt der Formel

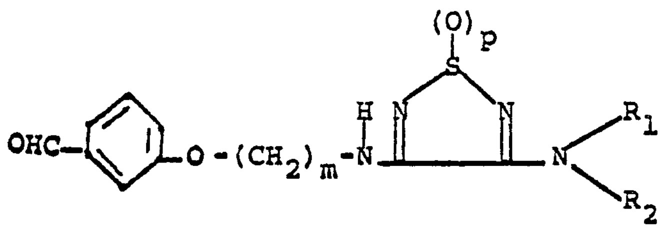

(XIX)

zu erhalten, und Reduktion des genannten Zwischenproduktes in Anwesenheit von Amin $R_3NHR_4$; oder Umsetzung des genannten Ester-Zwischenproduktes, wenn m = 2, mit einem Mercaptan der Formel R—Ⓐ—$(CH_2)_n$—SH; oder Umsatzung eines Alkohols der Formel

R—Ⓐ—$(CH_2)_n$—X—$CH_2CH_2OH$

mit, nach Aktivierung des Alkohols gegen Nucleophile, einer Aminoverbindung der Formel

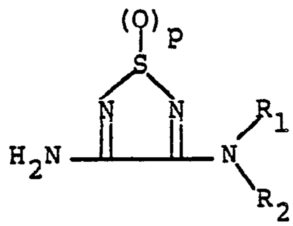

oder Umsetzung eines Mercaptans der Formel

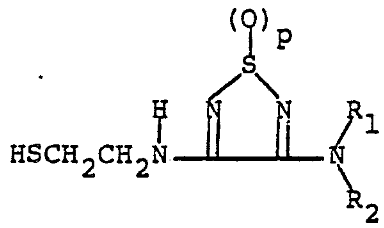

mit einem Alkohol der Formel

$$R-Ⓐ-(CH_2)_n-OH$$

in Anwesenheit einer Mineralsäure, um die Verbindungen der Formel I oder II zu erhalten und die physiologisch akzeptablen Salze derselben; oder Umsetzung der Verbindungen der Formel I und ihrer Tautomeren mit einem Alkylierungsmittel, einem Acylierungsmittel oder einem Sulfonierungsmittel, um Derivate der Formel

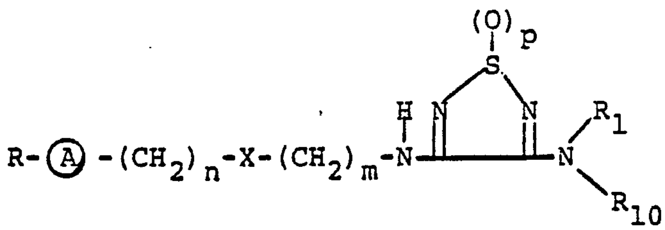

(IIIA)

und

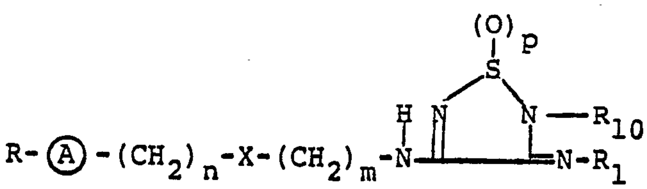

(IIIB)

zu erhalten, und die physiologisch akzeptablen Salze derselben.

2. Verfahren nach Ansruch 1, dadurch gekennzeichnet, dass Verbindungen hergestellt werden, ausgewählt aus der Gruppe von:

3-N[3-(2-Guanidino-4-thiazolyl)phenyl]amino-4-methylamino-1,2,5-thiadiazol-1-oxid;
3-N[3-(2-Guanidino-4-thiazolyl)phenyl]amino-4-amino-1,2,5-thiadiazol-1-oxid;
3-N-[3-[3-[Dimethylaminomethyl)phenoxy]propyl]amino-4-amino-1,2,5-thiadiazol-1-oxid;
3-N-[3-[3-(Dimethylaminomethyl)phenoxy]propyl]amino-4-methylamino-1,2,5-thiadiazol-1-oxid;
3-N-[3-[3-(Dimethylaminomethyl)phenoxy]propyl]amino-4-amino-1,2,5-thiadiazol-1,1-dioxid;
3-N-[2-[(2-Furanyl)methylthio]ethyl]amino-4-amino-1,2,5-thiadiazol-1-oxid;
3-N-[2-[(5-Dimethylaminomethyl-2-thienyl)methylthio]ethyl]amino-4-amino-1,2,5-thiadiazol-1-oxid;
3-N-[2-[(4-methyl-5-oxazolyl)methylthio]ethyl]amino-4-amino-1,2,5-thiadiazol-1-oxid;
3-N-[2-[(2-Furanyl)methylthio]ethyl]amino-4-amino-1,2,5-thiadiazol-1,1-dioxid;
3-N-[2-[[5-(1-Piperidinylmethyl)-2-furanyl]methylthio]ethyl]amino-4-amino-1,2,5-thiadiazol-1-oxid;
3-N-[2-[(5-Dimethylaminomethyl-2-furanyl)methylthio]ethyl]amino-4-amino-1,2,5-thiadiazol-1-oxid;
3-N-[2-[5-Dimethylaminomethyl-2-furanyl)methylthio]ethyl]amino-4-methylamino-1,2,5-thiadiazol-1-oxid;
3-N-[2-[(5-Dimethylaminomethyl-2-furanyl)methylthio]ethyl]amino-4-methylamino-1,2,5-thiadiazol-1,1-oxid;
3-N-[2-[(5-Dimethylaminomethyl-2-furanyl)methylthio]ethyl]amino-4-amino-1,2,5-thiadiazol-1,1-dioxid;
3-N-[2-[(5-Methylaminomethyl-2-furanyl)methylthio]ethyl]amino-4-amino-1,2,5-thiadiazol-1-oxid;
3-N-[2-[(5-Methylaminomethyl-2-furanyl)methylthio]ethyl]amino-4-amino-1,2,5-thiadiazol-1,1-dioxid;
3-N-[2-[(2-Guanidino-4-thiazolyl)methylthio]ethyl]amino-4-methylamino-1,2,5-thiadiazol-1,1-dioxid;
3-N-[2-[(2-Guanidino-4-thiazolyl)methylthio]ethyl]amino-4-amino-1,2,5-thiadiazol-1-oxid;
3-N-[2-[(Guanidino-4-thiazolyl)methylthio]ethyl]amino-4-methylamino-1,2,5-thiadiazol-1-oxid, und
3-N-[2-[(6-[4-Morpholinylmethyl-2-benzofuranyl)methylthio]ethyl]-amino-4-amino-1,2,5-thiadiazol-1-oxid.

3. Verfahren nach Anspruch 1, dadurch gekennzeichnet, dass eine Verbindung der Formel I hergestellt wird, nämlich das 3-N-[3-[3-(1-Piperidinylmethyl)phenoxy]propyl]amino-4-amino-1,2,5-thiadiazol-1-oxid.

4. Verfahren nach Anspruch 3, dadurch gekennzeichnet, dass 3-N-[3-[3-(1-Piperidinylmethyl)phenoxy]-propyl]amino-4-amino-1,2,5-thiadiazol-1-oxid hergestellt wird, indem 3-Ethoxy-4-amino-1,2,5-thiadiazol-1-oxid mit 3-[3-(1-Piperidinylmethyl)phenoxy]propan-amin unter Gewinnung des gewünschten Endproduktes umgesetzt wird.

5. Verfahren zur Herstellung von Verbindungen der Formeln:

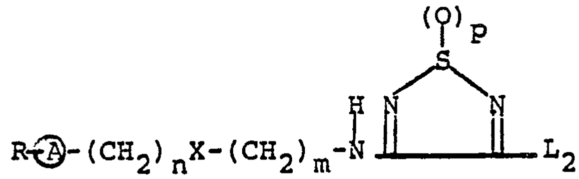

(VI)

ou

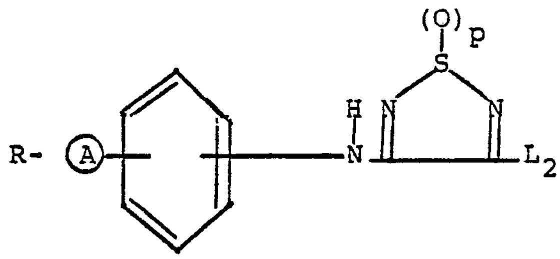

(VI A)

worin

R, Ⓐ, n, X und m wie in Anspruch 1 definiert sind, p 1 oder 2,

$L_2$ Alkoxy mit 1 bis 5 C-Atomen, Alkylthio mit 1 bis 5 C-Atomen, Phenoxy, Phenylthio oder Halogen ist; und die Salze derselben, dadurch gekennzeichnet, dass eine Verbindung der Formel

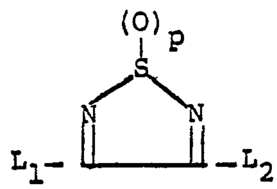

worin $L_1$ eine austretende Gruppe ist, mit einem Amin der Formeln:

R—Ⓐ—$(CH_2)_n$—X—$(CH_2)_mNH_2$

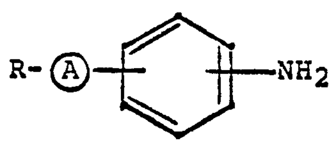

umgesetzt wird, und dass die Verbindungen der Formeln VI oder VIA oder Salze derselben isoliert werden.

6. Verfahren nach Anspruch 5, dadurch gekennzeichnet, dass Verbindungen hergestellt werden, die aus der Gruppe ausgewählt sind:

3-N-[2-[5-Dimethylyaminomethyl-2-furanyl)methylthio]ethyl]amino-4-ethoxy-1,2,5-thiadiazol-1-oxid und

3-N-[2-[5-Dimethylaminomethyl-2-furanyl)methylthio]ethyl]amino-4-ethoxy-1,2,5-thiadiazol-1,1-dioxid.

7. Verfahren zur Herstellung von Verbindungen der Formel

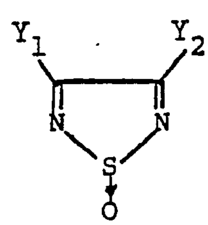

(XX)

worin $Y_1$ und $Y_2$ ausgewählt sind aus den folgenden Gruppen:

a) $Y_1$ und $Y_2$ sind gleich und sind Alkoxy mit 1 bis 5 C-Atomen, Alkylthio mit 1 bis 5 C-Atomen, Phenoxy oder Phenylthio, oder

b) $Y_1$ und $Y_2$ sind unterschiedlich und eines von $Y_1$ und $Y_2$ ist Alkoxy mit 1 bis 5 C-Atomen, Phenoxy oder Phenylthio, und das andere von $Y_1$ und $Y_2$ ist eine Gruppe der Formel

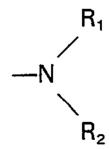

worin $R_1$ und $R_2$ unabhängig wie in Anspruch 1 definiert sind oder $R_1$ und $R_2$ zusammen mit dem Stickstoffatom, an das sie gebunden sind, eine Gruppe wie in Anspruch 1 definiert bilden, und

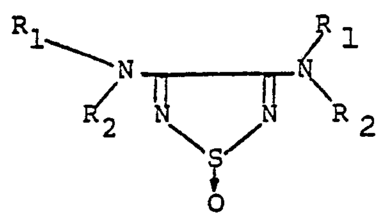

(XXI)

worin $R_1$ und $R_2$ unabhängig wie in Anspruch 1 definiert sind oder $R_1$ und $R_2$ zusammen mit dem Stickstoffatom, an das sie gebuden sind, eine Gruppe wie in Anspruch 1 definiert bilden, dadurch gekennzeichnet, dass eine Verbindung der Formel

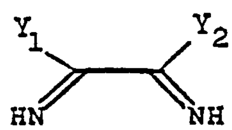

worin $Y_1$ und $Y_2$ wie in a) definiert sind, mit Thionylchlorid in Anwesenheit einer Base behandelt wird, um eine Verbindung der Formel

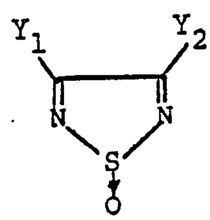

(XX)

zu erhalten, die mit einem Amin der Formel

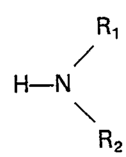

behandelt wird, um Verbindungen zu erhalten, in denen $Y_1$ und $Y_2$ wie in b) definiert sind, oder eine Verbindung der Formel

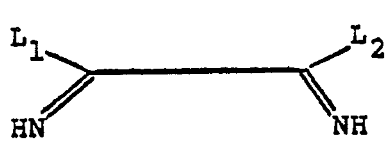

worin $L_1$ und $L_2$ austretende Gruppen sind, ausgewählt aus der Gruppe der Alkoxygruppen mit 1 bis 5 C-Atomen, Alkylthio mit 1 bis 5 C-Atomen, Phenoxy, oder Phenylthio, mit Thionylchlorid in Anwesenheit einer Base behandelt wird, um eine Zwischenverbindung der Formel

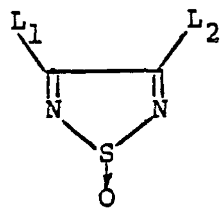

zu erhalten, die mit einem Amin der Formel

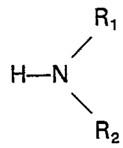

oder nacheinander mit zwei unterschiedlichen Aminen dieser Formel umgesetzt wird, um eine Verbindung der Formel XXI

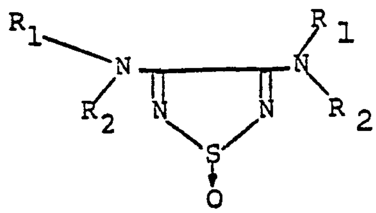

(XXI)

zu erhalten.

8. Verbindungen von Anspruch 7, dadurch gekennzeichnet, dass Verbindungen der Formel XX oder XXI hergestellt werden, die aus der Gruppe ausgewählt werden:
3,4-Diethoxy-1,2,5-thiadiazol-1-oxid;
3,4-Bis-methylthio,1,2,5-thiadiazol-1-oxid;
3-Isopropylamino-4-methylthio-1,2,5-thiadiazol-1-oxid;
3-Ethoxy-4-amino,1,2,5-thiadiazol-1-oxid;
3-Ethoxy-4-methylamino-1,2,5-thiadiazol-1-oxid;
3-Ethoxy-4-(1-pyrrolidinyl)-1,2,5-thiadiazol-1-oxid;
3-Ethoxy-4-(4-morpholino)-1,2,5-thiadiazol-1-oxid;
3,4-Diamino-1,2,5-thiadiazol-1-oxid;
3-Amino-4-(2-dimethylaminoethyl)amino-1,2,5-thiadiazol-1-oxid;
3-Amino-4-methylamino-1,2,5-thiadiazol-1-oxid;
3-Amino-4-dodecylamino-1,2,5-thiadiazol-1-oxid;
3-Amino-4-(4-fluoranilino)-1,2,5-thiadiazol-1-oxid;
3-Amino-4-Morpholino-1,2,5-thiadiazol-1-oxid; und
3-Morpholino-4-(2-pyridinomethyl)amino-1,2,5-thiadiazol-1-oxid.

9. Verfahren zur Herstellung von Verbindungen der Formel

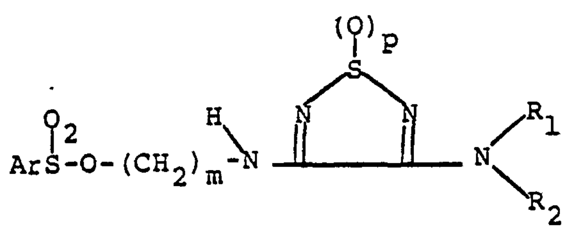

(XVII)

worin Ar Aryl ist und $R_1$, $R_2$, m und p wie in Anspruch 1 definiert sind und

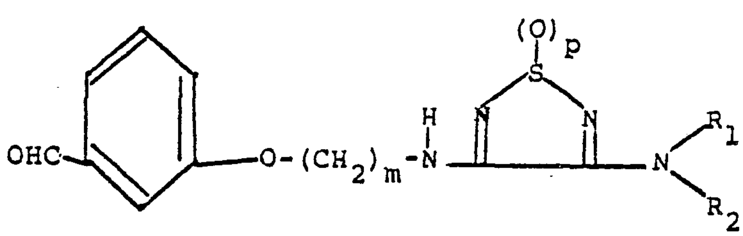

(XIX)

worin $R_1$, $R_2$, m und p wie in Anspruch 1 definiert sind, dadurch gekennzeichnet, dass eine Verbindung der Formel

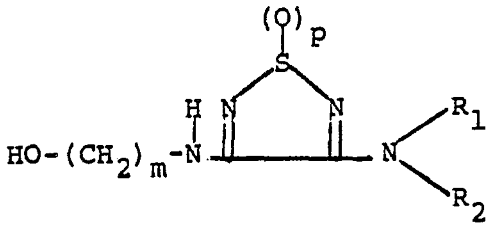

(XVI)

in Gegenwart einer Base mit einer Verbindung der Formel

$ArSO_2Cl$

umgesetzt wird, um die Verbindung der Formel XVII zu erhalten, die gegebenenfalls mit einer Verbindung der Formel XVIII umgesetzt wird,

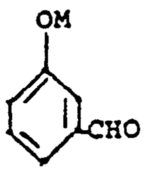

(XVIII)

um die Verbindung der Formel XIX zu erhalten.

10. Verfahren zur Herstellung einer Zusammensetzung, brauchbar für die Unterdrückung gastrischer saurer Sekretionen in einem Tier mit überschüssigen gastrischen sauren Sekretionen, dadurch gekennzeichnet, dass eine Verbindung der Formel I, II, III, IIIa, IIIb, IVa oder IVb oder Solvate dieser Verbindungen oder Solvate der Salze dieser Verbindungen sowie quartäre Ammoniumsalze und N-Oxide derselben, hergestellt nach dem Verfahren von Anspruch 1, mit einem inerten Träger formuliert werden.

**Revendications pour les Etats contractants: BE CH DE FR GB IT LI LU NL SE**

1. Composés de formule:

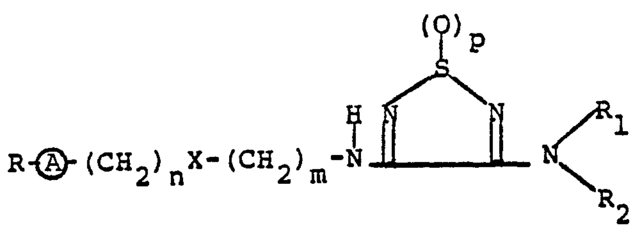

(I)

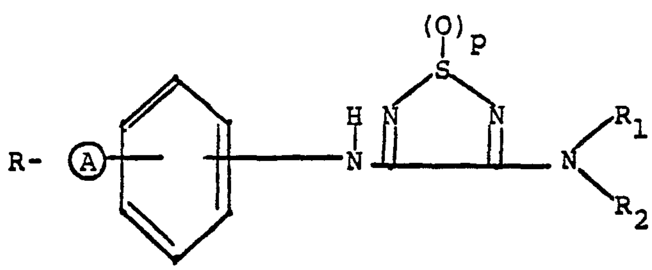

(II)

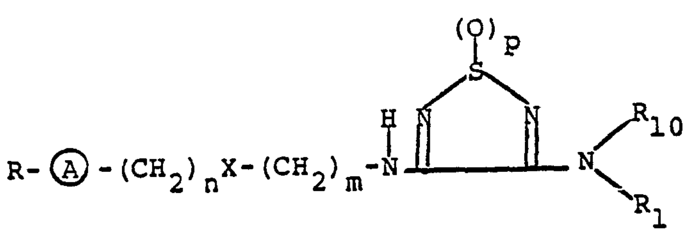

(IIIA)

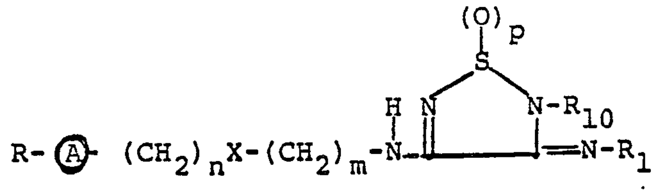

(IIIB)

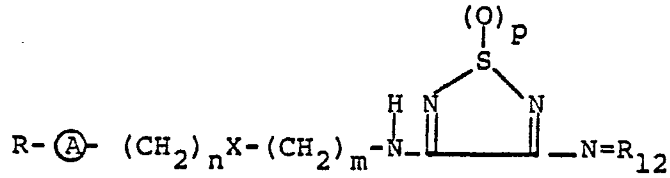

(IVA)

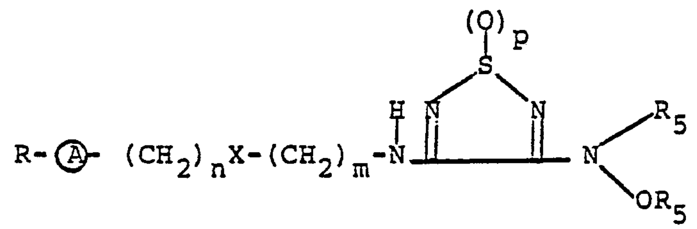

(IVB)

où R est un hydrogène, un alcoyle ayant de 1 à 5 atomes de carbone ou un groupe de formule

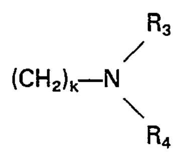

ou

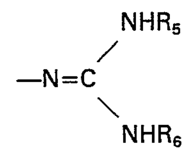

où

$R_3$ et $R_4$ représentent indépendamment un hydrogène, un alcoyle ayant de 1 à 5 atomes de carbone, un cycloalcoyle ayant de 3 à 7 atomes de carbone ou un phénylalcoyle où le groupe alcoyle a de 1 à 5 atomes de carbone, ou

$R_3$ et $R_4$ forment ensemble avec l'atome d'azote auquel ils sont attachés, un hétérocycle à 5 ou 6 chaînons, qui peut également contenir un oxygène, un soufre ou un N-R7-hétéroatome, où

$R_7$ est un hydrogène, un alcoyle ayant de 1 à 3 atomes de carbone ou un benzyle,

$R_5$ et $R_6$ représentent indépendamment un hydrogène ou un alcoyle ayant de 1 à 5 atomes de carbone

n vaut 0 ou 1;

m vaut de 2 à 4;

k vaut de 1 à 4;

X est un oxygène, un soufre ou un méthylène;

p vaut 1 ou 2;

$R_1$ et $R_2$ représentent indépendamment un hydrogène, un alcoyle ayant de 1 à 5 atomes de carbone, un alcényle ayant de 3 à 5 atomes de carbone, un alcynyle ayant de 3 à 5 atomes de carbone, un cycloalcoyle ayant de 3 à 7 atomes de carbone, un phényle, un pyridyle et un alcoyle substitué ayant de 1 à 5 atomes de carbone dans le groupe alcoyle, où le substituant est un phényle, un cycloalcoyle ayant de 3 à 7 atomes de carbone, un pyridyle, un imidazolyle, un morpholino, un hydroxy, un alcoxy ayant de 1 à 5 atomes de carbone, un alcoylthio ayant de 1 à 5 atomes de carbone ou un dialcoylamino où chaque groupe alcoyle a de 1 à 5 atomes de carbone ou

$R_1$ et $R_2$ forment ensemble avec l'azote auquel ils sont attachés, un hétérocycle à 5 ou 6 chaînons qui peut également contenir un oxygène, un soufre ou un N-$R_7$-Hétéroatome où $R_7$ est tel que défini ci-dessus;

Ⓐ est un phénylène ou un hétérocycle à 5 ou 6 chaînons contenant de 1 à 3 hétéroatomes choisis entre l'oxygène, le soufre ou l'azote, et sur lequel peut éventuellement être condensé un noyau benzo;

à condition que lorsque Ⓐ dans la formule 1 ci-dessus est un hétérocycle à 5 chaînons ou un hétérocycle à 5 chaînons condensé avec un benzo, n vaut 1; et,

à condition en outre que lorsque Ⓐ dans la formule I ou la formule II est un furanne de structure

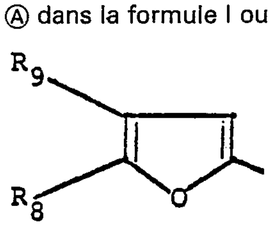

alors

$R_8$ est semblable à R défini ci-dessus et

$R_9$ est un alcoyle ayant de 1 à 5 atomes de carbone, un alcoxyalcoyle où le groupe alcoxy et le groupe alcoyle ont chacun de 1 à 5 atomes de carbone, un alcoxycarbonyle où le groupe alcoxy a de 1 à 5 atomes de carbone, un halo, de préférence un chlore ou un brome, ou un aryle, de préférence un phényle;

$R_{10}$ est un alcanoyle où le groupe alcoyle contient de 1 à 5 atomes de carbone, un groupe phénylcarbonyle où le noyau phényle est éventuellement substitué par 1, 2 ou 3 substituants, choisi entre halo, alcoyle et alcoxy ayant l'un et l'autre de 1 à 5 atomes de carbone, un hétéroaroyle où le noyau hétérocyclique est un noyau à 6 chaînons contenant de l'azote ayant 1 ou 2 hétéroatomes, un alcoylsulfonyle où le groupe alcoyle a de 1 à 5 atomes de carbone, un arylsulfonyle, un hétéroarylsulfonyle, où le noyau hétérocyclique est un noyau à 6 chaînons contenant de l'azote ayant 1 ou 2 hétéroatomes, un cyano, un amidino, un dialcoylcarbamoyle où le groupe alcoyle contient de 1 à 5 atomes de carbone, un dialcoylsulfamoyle où le groupe alcoyle contient de 1 à 5 atomes de carbone, un alcoxycarbonyle, où le groupe alcoxy contient de 1 à 5 atomes de carbone ou un groupe de formule

$$—(CH_2)_yCOR$$

où

y vaut 1 ou 2; et

$R_{11}$ est un alcoyle ayant de 1 à 5 atomes de carbone, un hydroxy, un alcoxy où le groupe alcoyle contient de 1 à 5 atomes de carbone, un amino, un alcoylamino où le groupe alcoyle a de 1 à 5 atomes de carbone ou un dialcoylamino où les groupes alcoyle ont chacun de 1 à 5 atomes de carbone; et

$R_{12}$ est un aralcoylidène ou un diarylméthylidène;

ou sont des sels des composés de formules I, II, IIIa, IIIb, IVa et Vb ou des produits de solvatation des sels desdits composés ainsi que des sels d'ammonium quaternaires et des N-oxydes.

2. Composés de la revendication 1 qui sont des membres choisis dans le groupe constitué par:

3-N-[3-(2-guanidino-4-thiazolyl)phényl]amino-4-méthylamino-1,2,5-thiadiazole-1-oxyde;

3-N-[3-(2-guanidino-4-thiazolyl)phényl]amino-4-amino-1,2,5-thiadazole-1-oxyde;

3-N-[3-[3-(diméthylaminométhyle)phénoxy]propyl]amino-4-amino-1,2,5-thiadiazole-1-oxyde;

3-N-[3-[3-(diméthylaminométhyle)phénoxy]propyl]amino-4-méthylamino-1,2,5-thiadiazole-1-oxyde;

3-N-[3-[3-(diméthylaminométhyle)phénoxy]propyl]amino-4-amino-1,2,5-thiadiazole-1,1-dioxyde;

3-N-[2-[(2-furanyl)méthylthio]éthyl]amino-4-amino-1,2,5-thiadiazole-1-oxyde;

3-N-[2-[(5-diméthylaminoéthyl-2-thiényl]méthylthio]éthyl/amino-4-amino-1,2,5-thiadiazole-1-oxyde;

3-N-[2-[(4-méthyl-5-oxazolyl)méthylthio]éthyl]amino-4-amino-1,2,5-thiadiazole-1-oxyde;

3-N-[2-[(2-furanyl)méthylthio]éthyl]amino-4-amino-1,2,5-thiadiazole-1-oxyde;

3-N-[2-[[5-(1-pipéridinylméthyl)-2-furanyl]méthylthio]éthyl]amino-4-amino-1,2,5-thiadiazole-1-oxyde;

3-N-[2-[(5-diméthylaminométhyl-2-furanyl)méthylthio]éthyl]amino-4-amino-1,2,5-thiadiazole-1-oxyde;

3-N-[2-[(5-diméthylaminoéthyl-2-furanyl)méthylthio]éthyl]amino-4-méthyl-amino-1,2,5-thiadiazole-1-oxyde;

3-N-[2-[(5-diméthylaminoéthyl-2-furanyl)méthylthio]éthyl]amino-4-méthyl-amino-1,2,5-thiadiazole-1,1-dioxyde;

3-N-[2-[(5-diméthylaminoéthyl-2-furanyl)méthylthio]éthyl]amino-4-amino-1,2,5-thiadiazole-1,1-dioxyde;

3-N-[2-[(5-méthylaminométhyl-2-furanyl)méthylthio]éthyl]amino-4-amino-1,2,5-thiadiazole-1-oxyde;

3-N-[2-[(5-méthylaminométhyl-2-furanyl)méthylthio]éthyl]amino-4-amino-1,2,5-thiadiazole-1,1-dioxyde;

3-N-]2-[(2-guanidino-4-thiazolyl)méthylthio]éthyl]amino-4-méthylamino-1,2,5-thiadiazole-1,1-dioxyde;

3-N-[2-[(2-guanidino-4-thiazolyl)méthylthio]éthyl]amino-4-amino-1,2,5-thiadiazole-1-oxyde;

3-N-[2-[(2-guanidino-4-thiazolyl)méthylthio]éthyl]amino-4-méthylamino-1,2,5-thiadiazole-1-oxyde;

3-N-[2-[(6-(4-morpholinylméthyl-2-benzofuranyl)méthylthio]éthyl]amino-4-amino-1,2,5-thiadiazole-1-oxyde.

3. Composé de formule I de la revendication 1 qui est le 3-N-[3-[3-(1-pipéridinylméthyl)-phénoxy]propyl]amino-4-amino-1,2,5-thiadiazole-1-oxyde.

4. Composés de formules:

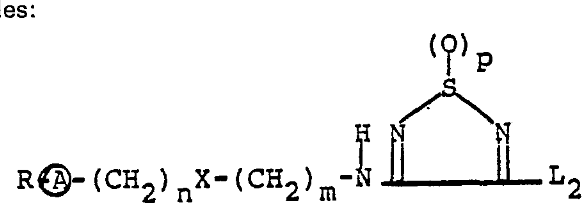

(VI)

and

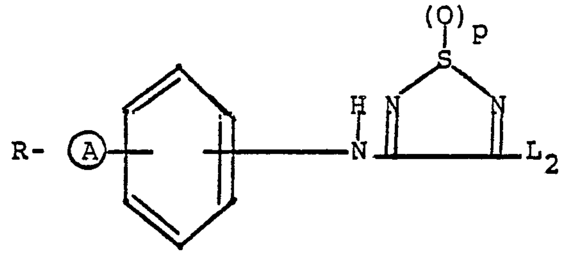

(VI A)

où R, Ⓐ, n, X et m sont tels que définis dans la revendication 1; p vaut 1 ou 2; L2 est un alcoxy ayant de 1 à 5 atomes de carbone, un alcoylthio ayant de 1 à 5 atomes de carbone, un phénoxy, un phénylthio ou un halogène; et leurs sels.

5. Les composés de la revendication 4 qui sont des membres choisis dans le groupe constitué par; 3-N-[2-[5-diméthylaminoéthyl-2-furanyl)méthylthio]éthyl]amino-4-éthoxy-1,2,5-thiadiazole-1-oxyde et 3-N-[2-[(5-diméthylaminoéthyl-2-furanyl)méthylthio]éthyl]amino-4-éthoxy-1,2,5-thiadiazole-1-dioxyde.

6. Composés de formules:

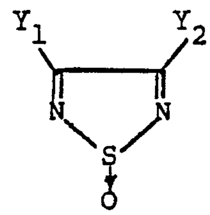

(XX)

où $Y_1$ et $Y_2$ sont des membres choisis dans les groupes suivants:

a) $Y_1$ et $Y_2$ sont semblables et représentent un alcoxy ayant de 1 à 5 atomes de carbone, un alcoylthio ayant de 1 à 5 atomes de carbone, un phénoxy ou un phénylthio; ou

b) $Y_1$ et $Y_2$ sont différents et l'un des radicaux $Y_1$ et $Y_2$ est un alcoxy ayant de 1 à 5 atomes de carbone, un phénoxy ou un phénylthio et l'autre des radicaux $Y_1$ et $Y_2$ est un groupe ayant la formule:

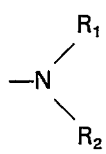

où $R_1$ et $R_2$ sont indépendamment tels que définis dans la revendication 1 ou $R_1$ et $R_2$ forment ensemble avec l'atome d'azote auquel ils sont attachés un groupe tel que défini dans la revendication 1, et

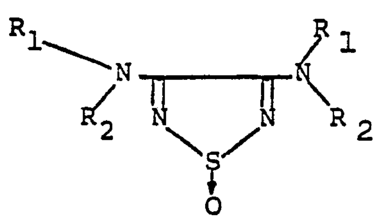

(XXI)

où $R_1$ et $R_2$ sont indépendamment tels que définis dans la revendication 1 ou $R_1$ et $R_2$ forment ensemble avec l'atome d'azote auquel ils sont attachés un groupe tel que défini dans la revendication 1.

7. Composés de formules

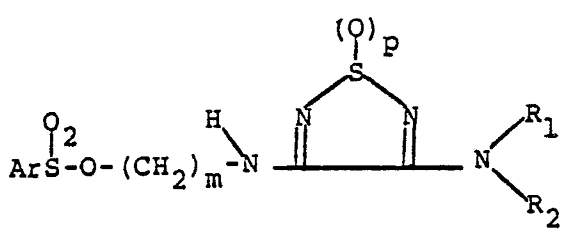

(XVII)

où Ar représente un aryle et $R_1$, $R_2$, m et p sont tels que définis dans la revendication 1 et

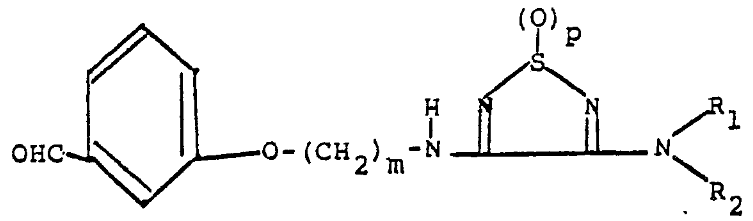

(XIX)

où $R_1$, $R_2$, m et p sont tels que définis dans la revendication 1.

8. Composés de la revendication 6 qui sont des composés de formule XX ou XXI et des membres choisis dans le groupe constitué par:

3,4-diéthoxy-1,2,5-thiadiazole-1-oxyde;
3,4-bis-méthylthio-1,2,5-thiadiazole-1-oxyde;
3-isopropylamino-4-méthylthio-1,2,5-thiadiazole-1-oxyde;
3-éthoxy-4-amino-1,2,5-thiadiazole-1-oxyde;
3-éthoxy-4-méthylamino-1,2,5-thiadiazole-1-oxyde;
3-éthoxy-4-(1-pyrrolidinyl)-1,2,5-thiadiazole-1-oxyde;
3-éthoxy-4-(4-morpholino)-1,2,5-thiadiazole-1-oxyde;
3,4-diamino-1,2,5-thiadiazole-1-oxyde;
3-amino-4-(2-diméthylaminoéthyl)amino-1,2,5-thiadiazole-1-oxyde;
3-amino-4-méthylamino-1,2,5-thiadiazole-1-oxyde;
3-amino-4-dodécylamino-1,2,5-thiadiazole-1-oxyde;
3-amin-4-(4-fluoranilino)-1,2,5-thiadiazole-1-oxyde;
3-amino-4-morpholino-1,2,5-thiadiazole-1-oxyde;
3-morpholino-4-(2-pyridinométhyl)amino-1,2,5-thiadiazole-1-oxyde.

9. Procédé de préparation d'un composé de formules I, II, IVA ou IVB selon la revendication 1 dans lequel on traite un composé de formule:

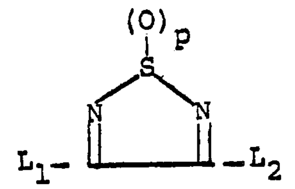

où p vaut 1 ou 2 et $L_1$ et $L_2$ sont des groupes sortants, tout d'abord avec l'une des amines de formules

$$R-(A)-(CH_2)_n-X-(CH_2)_m-NH_2$$

ou

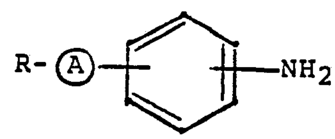

ou

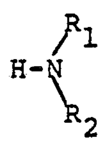

où R, A, n, X, m, $R_1$ et $R_2$ sont tels définis dans la revendication 1, ce qui donne un produit intermédiaire de formule VI

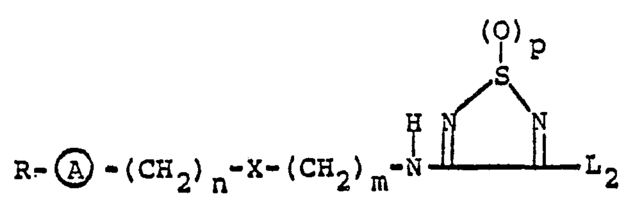

(VI)

ou VIa

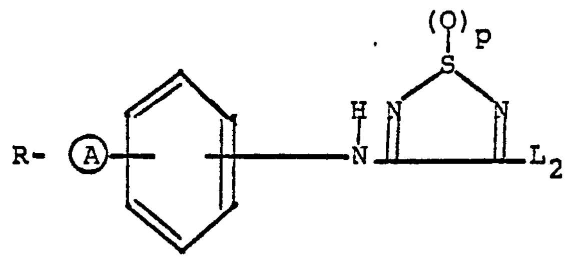

(VIa)

ou

VII

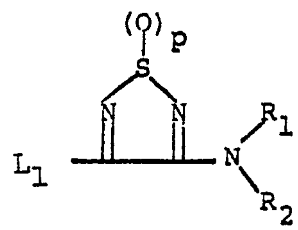

(VII)

puis on fait réagir le produit intermédiaire de formules VI et VIa avec l'amine de formule

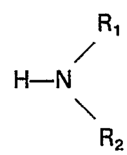

ou le produit intermédiaire de formule VII avec soit l'amine

R-Ⓐ-$(CH_2)_n$-X-$(CH_2)_m$-$NH_2$

sont l'amine

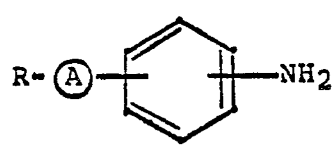

pour donner les produits finals de formule II ou III et le cas échéant on fiat réagir un composé de formule I ou II où le groupe

R—Ⓐ—

est un noyau furanne non substitué avec soit

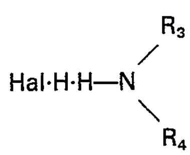 + $CH_2O$ soit 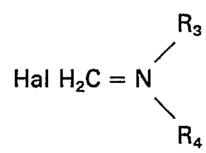

où Hal est un halo pour produire les composés correspondants de formule I ou II où A est un noyau furanne substitué par un groupe R de formule

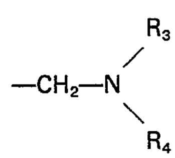

où $R_3$ et $R_4$ sont tels que définis dans la revendication 1 ou l'on fait réagir le composé intermédiaire de formule VI

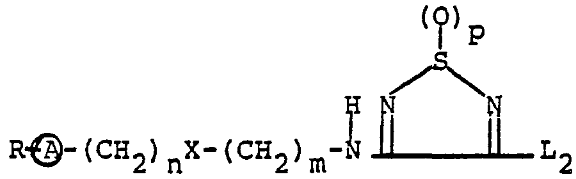

(VI)

avec une dimine de formule

$$H—N = R_{12}$$

pour produire des composés de formule IVA

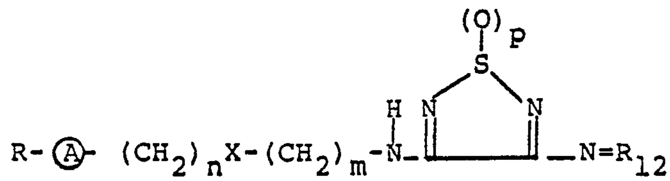

(IVA)

ou on fait réagir le produit intermédiaire de formule VI

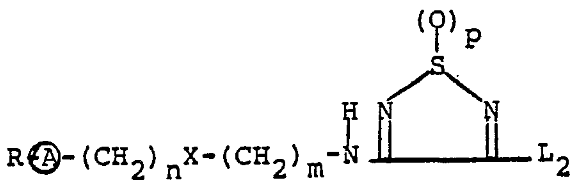

(VI)

avec une hydroxylamine éventuellement substituée de formule

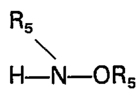

pour produire des composés de formule IVB

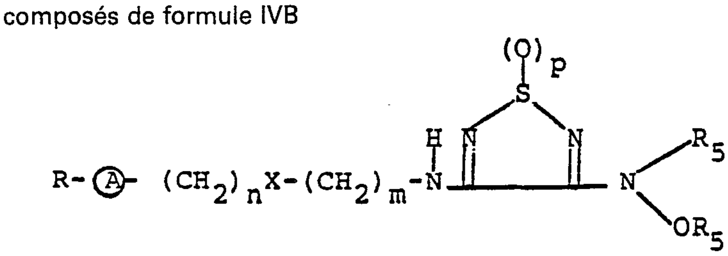

(IVB)

où R, $R_5$, Ⓐ, X, m, n, et p sont tels que définis dans la revendication 1, et on isole les composés de formules I, II, IVa ou IVb sous forme de bases libres ou de sels ou sous forme de produits de solvatation des bases libres ou des sels ou on les transforme en sels d'ammonium quaternaires ou N-oxydes desdits composés.

10. Procédé de préparation de 3-N-[3-[3-(1-pipéridinylméthyl)phénoxy]propyl]amino-4-amino-1,2,5-thiadiazole-1-oxyde selon la revendication 3, caractérisé en ce qu'on fait réagir du 3-éthoxy-4-amino-1,2,5-thiadiazole-1-oxyde avec de la 3-[3-(1-pipéridinylméthyl)phénoxy]propaneamine, ce qui donne le produit final désiré.

11. Procédé de préparation de composés de formules I, II, IIIa et IIIb selon la revendication 1 dans lequel on fait réagir un alcool de formule XVI

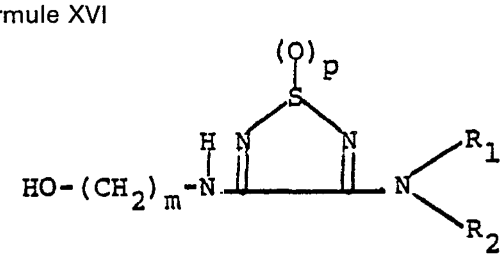

(XVI)

où $R_1$, $R_2$ et p sont tels que définis dans la revendication 1 et m est supérieur à 2, avec un agent acylant pour obtenir un intermédiaire ester activé; on fait réagir ledit intermédiaire ester avec un sel de métal alcalin du 3-hydroxybenzaldéhyde pour obtenir un intermédiaire de formule:

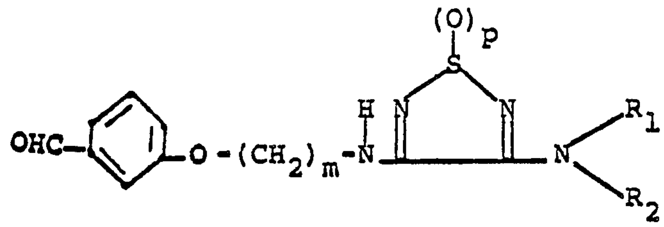

(XIX)

et on réduit ledit intermédiaire en présence de l'amine $R_3NHR4$, où $R_3$ et $R_4$ sont tels que définis dans la revendication 1; ou, on fait réagir ledit intermédiaire ester où m = 2 avec un mercaptan de formule R—Ⓐ—$(CH_2)_n$—SH où R, Ⓐ et n sont tels que définis dans la revendication 1; ou on fait réagir un alcool de formule

R—Ⓐ—$(CH_2)_n$—X—$CH_2CH_2OH$

où R, Ⓐ, X et n sont tels que définis dans la revendication 1 avec, après activation dudit alcool vis-à-vis des neucléophiles, un composé amino de formule:

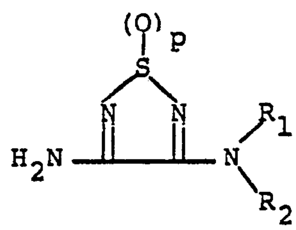

où $R_1$, $R_2$ et p sont tels que définis dans la revendication 1; ou, on fait réagir un mercaptan de formule:

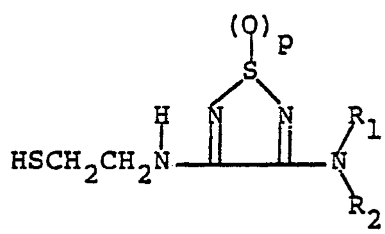

où $R_1$, $R_2$ et p sont tels que définis dans la revendication 1, avec un alcool de formule R—Ⓐ—$(CH_2)_n$—OH où R, Ⓐ et n sont tels que définis dans la revendication 1, en présence d'un acide minéral pour donner les composés de formula 1; et leurs sels physiologiquement acceptables; ou, on fait réagir les composés définis dans la revendication 1 et leurs tautomères avec un réactif alcoylant, un agent acylant ou un agent de sulfonation pour produire des dérivés de formules

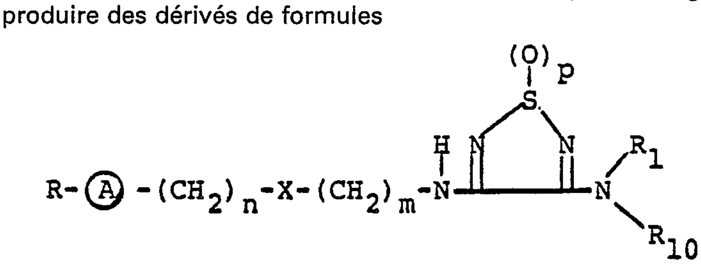

(IIIA)

et

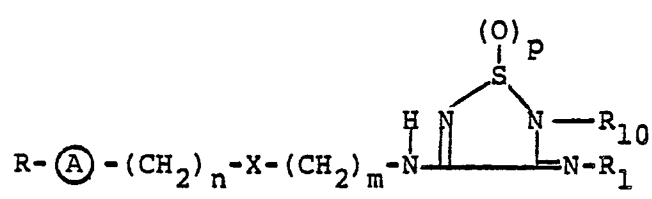

(IIIB)

où R, $R_{10}$, $R_1$, Ⓐ, X, m, n et p sont tels que définis dans la revendication 1; et leurs sels physiologiquement acceptables.

12. Procédé de préparation de composés de la revendication 6 dans lequel on traite un composé de formule:

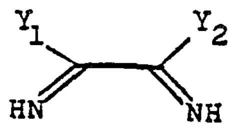

où $Y_1$ et $Y_2$ sont tels que définis en a) dans la revendication 6 avec du chlorure de thionyle en présence d'une base pour former un composé de formule:

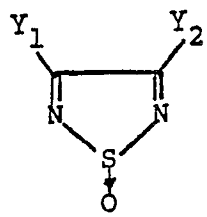

(XX)

que l'on traite avec une amine de formule:

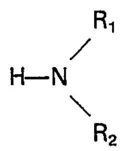

où $R_1$ et $R_2$ sont tels que définis dans la revendication 6 pour former des composés où $Y_1$ et $Y_2$ sont tels que définis en b) dans la revendication 6,

ou

on traite un composé de formule:

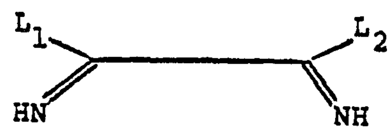

où $L_1$ et $L_2$ sont des groupes sortants choisis dans le groupe constitué par alcoxy ayant de 1 à 5 atomes de carbone, alcoylthio ayant de 1 à 5 atomes de carbone, phénoxy, ou phénylthio, avec du chlorure de thionyle en présence d'une base pour former un composé intermédiaire de formule:

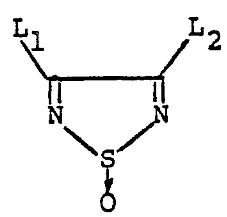

qu l'on fait réagir avec une amine de formule:

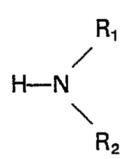

ou successivement avec deux amines différentes ayant ladite formule

où

$R_1$ et $R_2$ sont indépendamment tels que définis dans la revendication 6 ou $R_1$ et $R_2$ forment ensemble avec l'atome d'azote auquel ils sont attachés un groupe tel que défini dans la revendication 6 pour donner un composé de formule XXI

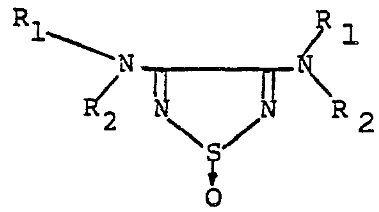

(XXI)

13. Composition servant à la suppression de sécrétions acides gastriques d'un animal souffrant de sécrétions gastriques excessives, laquelle comprend un support inerte et une quantité efficace d'un composé de la revendication 1.

**Revendications pour l'Etat contractant: AT**

1. Procédé de préparation de composés de formule

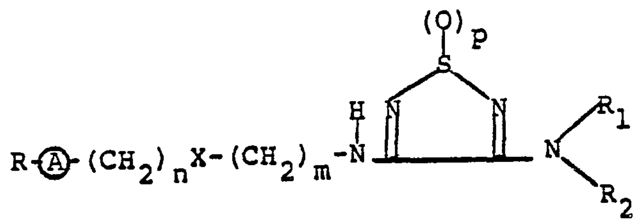

(I)

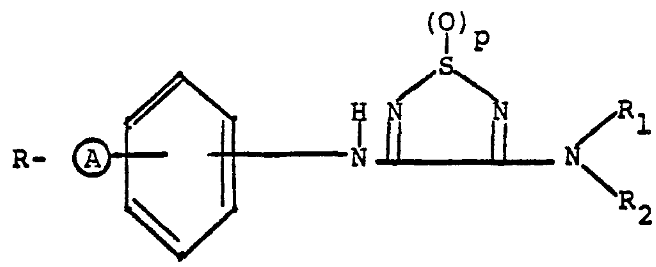

(II)

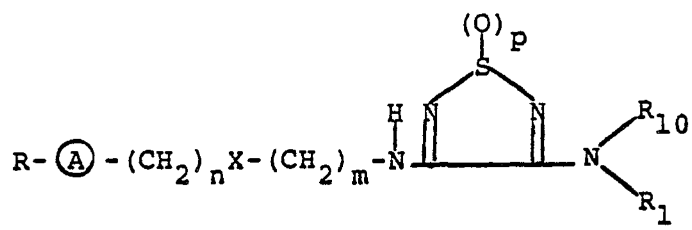

(IIIA)

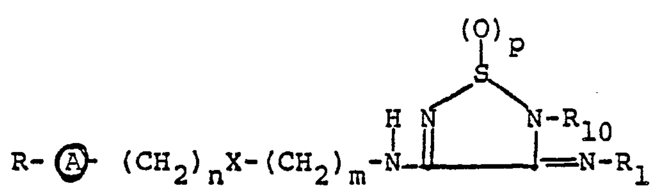

(IIIB)

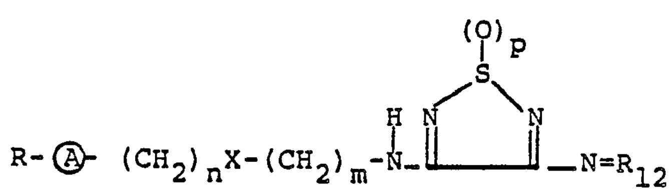

(IVA)

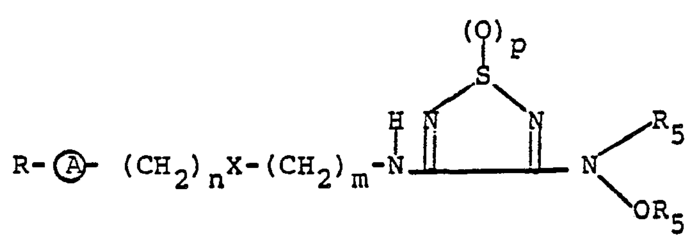

(IVB)

où R est un hydrogène, un alcoyle ayant de 1 à 5 atomes de carbone ou un groupe de formule

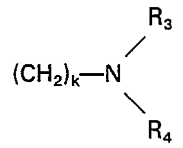
ou
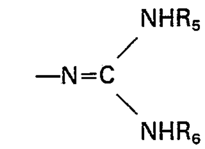

où

$R_3$ et $R_4$ représentant indépendamment un hydrogène, un alcoyle ayant de 1 à 5 atomes de carbone, un cycloalcoyle ayant de 3 à 7 atomes de carbone ou un phénylalcoyle où le groupe alcoyle a de 1 à 5 atomes de carbone, ou

$R_3$ et $R_4$ forment ensemble avec l'atome d'azote auquel ils sont attachés, un hétérocycle à 5 ou 6 chaînons, qui peut également contenir un oxygène, un soufre ou un N-R7-hétéroatome, où

$R_7$ est un hydrogène, un alcoyle ayant de 1 à 3 atomes de carbone ou un benzyle,

$R_5$ et $R_6$ représentent indépendamment un hydrogène ou un alcoyle ayant de 1 à 5 atomes de carbone

n vaut 0 ou 1;

m vaut de 2 à 4;

k vaut de 1 à 4;

X est un oxygène, un soufre ou un méthylène;

p vaut 1 ou 2;

$R_1$ et $R_2$ représentent indépendamment un hydrogène, un alcoyle ayant de 1 à 5 atomes de carbone, un alcényle ayant de 3 à 5 atomes de carbone, un alcynyle ayant de 3 à 5 atomes de carbone, un cycloalcoyle ayant de 3 à 7 atomes de carbone, un phényle, un pyridyle et un alcoyle substitué ayant de 1 à 5 atomes de carbone dans le groupe alcoyle, où le substituant est un phényle, un cycloalcoyle ayant de 3 à 7 atomes de carbone, un pyridyle, un imidazolyle, un morpholino, un hydroxy, un alcoxy ayant de 1 à 5 atomes de carbone, un alcoylthio ayant de 1 à 5 atomes de carbone ou un dialcoylamino où chaque groupe alcoyle a de 1 à 5 atomes de carbone ou

$R_1$ et $R_2$ forment ensemble avec l'azote auquel ils sont attachés, un hétérocycle à 5 ou 6 chaînons qui peut également contenir un oxygène, un soufre ou un N-$R_7$-Hétéroatome où $R_7$ est tel que défini ci-dessus;

Ⓐ est un phénylène ou un hétérocycle à 5 ou 6 chaînons contenant de 1 à 3 hétéroatomes choisis entre l'oxygène, le soufre ou l'azote, et sur lequel peut éventuellement être condensé un noyau benzo;

à condition que lorsque Ⓐ dans la formule 1 ci-dessus est un hétérocycle à 5 chaînons ou un hétérocycle à 5 chaînons condensé avec un benzo, n vaut 1; et,

à condition en outre que lorsque Ⓐ dans la formule I ou la formule II est un furanne de structure

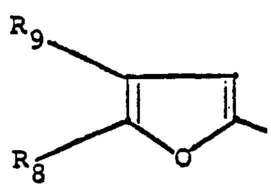

alors

$R_8$ est semblable à R défini ci-dessus et

$R_9$ est un alcoyle ayant de 1 à 5 atomes de carbone, un alcoxyalcoyle où le groupe alcoxy et le groupe alcoyle ont chacun de 1 à 5 atomes de carbone, un alcoxycarbonyle où le groupe alcoxy a de 1 à 5 atomes de carbone, un halo, de préférence un chlore ou un brome, ou un aryle, de préférence un phényle;

$R_{10}$ est un alcanoyle où le groupe alcoyle contient de 1 à 5 atomes de carbone, un groupe phénylcarbonyle où le noyau phényle est éventuellement substitué par 1, 2 ou 3 substituants, choisi entre halo, alcoyle et alcoxy ayant l'un et l'autre de 1 à 5 atomes de carbone, un hétéroaroyle où le noyau hétérocyclique est un noyau à 6 chaînons contenant de l'azote ayant 1 ou 2 hétéroatomes, un alcoylsulfonyle où le groupe alcoyle a de 1 à 5 atomes de carbone, un arylsulfonyle, un hétéroarylsulfonyle, où le noyau hétérocyclique est un noyau à 6 chaînons contenant de l'azote ayant 1 ou 2 hétéroatomes, un cyano, un amidino, un dialcoylcarbamoyle où le groupe alcoyle contient de 1 à 5 atomes de carbone, un dialcoylsulfamoyle où le groupe alcoyle contient de 1 à 5 atomes de carbone, un alcoxycarbonyle, où le groupe alcoxy contient de 1 à 5 atomes de carbone ou un groupe de formule

$$—(CH_2)_yCOR_{11}$$

où

y vaut 1 ou 2; et

$R_{11}$ est un alcoyle ayant de 1 à 5 atomes de carbone, un hydroxy, un alcoxy où le groupe alcoyle contient de 1 à 5 atomes de carbone, un amino, un alcoylamino où le groupe alcoyle a de 1 à 5 atomes de carbone ou un dialcoylamino où les groupes alcoyle ont chacun de 1 à 5 atomes de carbone; et

$R_{12}$ est un aralcoylidène ou un diarylméthylidène et,

sont des sels des composés de formules I, II, IIIa, IIIb, IVa et Vb et des produits de solvatation des sels desdits composés ainsi que des sels d'ammonium quaternaires et des N-oxydes, caractérisé en ce que les composés de formule I, II, IVa et Vb sont préparés par traitement d'un composé de formule:

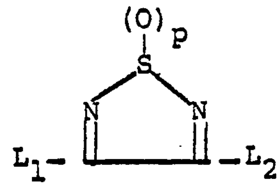

où $L_1$ et $L_2$ sont des groupes sortants, tout d'abord avec l'une des amines de formules

$R-Ⓐ-(CH_2)_n-X-(CH_2)_m-NH_2$

ou

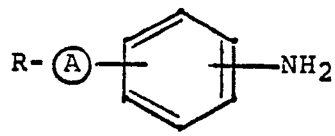

ou

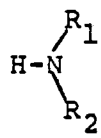

ce qui donne un produit intermédiaire de formule VI

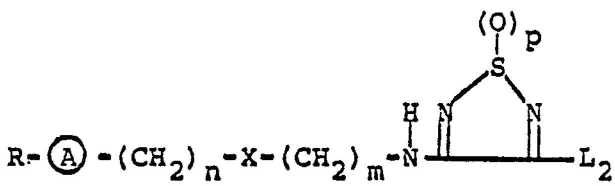

(VI)

ou VIa

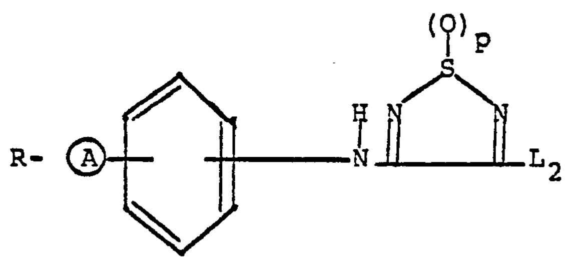

(VIa)

ou

VII

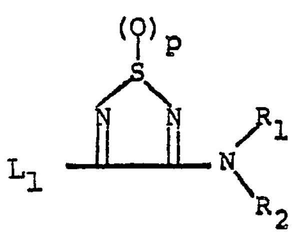

(VII)

puis on fait réagir le produit intermédiaire de formules VI et VIa avec l'amine de formule

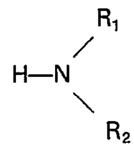

ou le produit intermédiaire de formule VII avec soit l'amine

$R-Ⓐ-(CH_2)_n-X-(CH_2)_m-NH_2$

soit l'amine

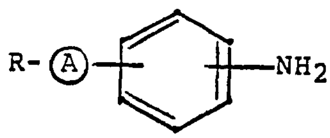

pour donner les produits finals de formule I ou II et le cas échéant on fait réagir un composé de formule I ou II où le groupe

R—Ⓐ—

est un noyau furanne non substitué avec soit

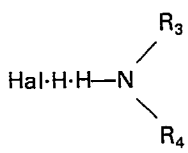 $+ CH_2O$ soit 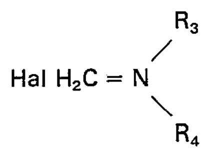

où Hal est un halo pour produire les composés correspondants de formule I ou II où A est un noyau furanne substitué par un groupe R de formule

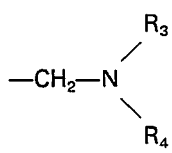

ou l'on fait réagir le composé intermédiaire de formule VI

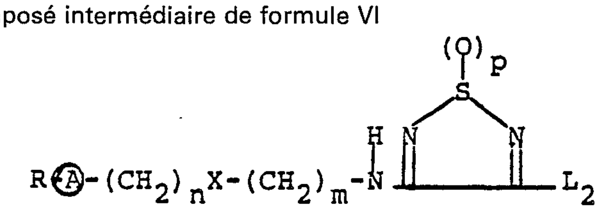

(VI)

avec une imine de formule

$H—N—R_{12}$

pour produire des composés de formule IVA

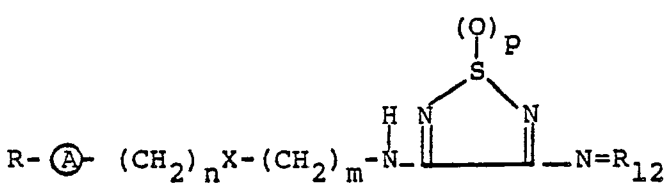

(IVA)

ou on fait réagir le produit intermédiaire de formule VI

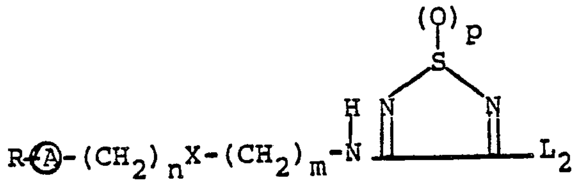

(VI)

avec une hydroxylamine éventuellement substituée de formule

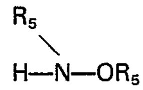

pour produire des composés de formule IVB

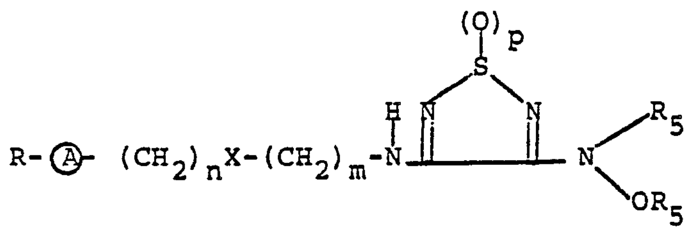

(IVB)

on isole les composés de formules I, II, IVa ou IVb sous forme de bases libres ou de sels ou sous forme de produits de solvatation des bases libres ou des sels ou on les transforme en sels d'ammonium quaternaires ou N-oxydes desdits composés,
ou
en ce qu'on prépare les composés de formules I, II, IIIa et IIIb en faisant réagir un alcool de formula XVI

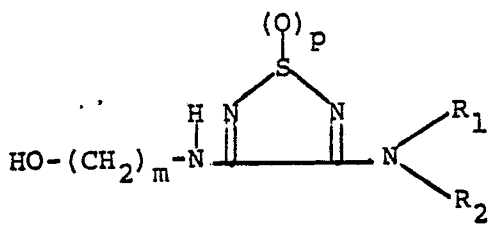

(XVI)

où m est supérieur à 2, avec un agent acylant pour obtenir un intermédiaire ester activé; on fait réagir ledit intermédiaire ester avec un sel de métal alcalin du 3-hydroxy-benzaldéhyde pour obtenir un intermédiaire de formule:

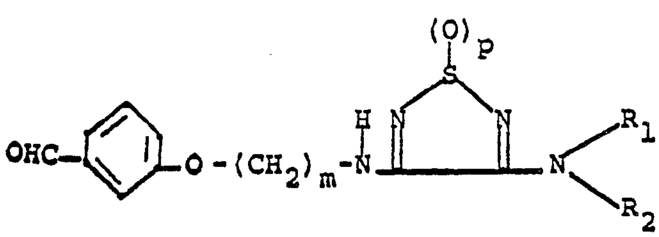

(XIX)

et on réduit ledit intermédiaire en présence de l'amine $R_3NHR_4$; ou, on fait réagir ledit intermediaire ester où m = 2 avec un mercaptan de formule R—Ⓐ—$(CH_2)_n$—SH; ou on fait réagir un alcool de formule

R—Ⓐ—$(CH_2)_n$—X—$CH_2CH_2OH$

avec, après activation dduit alcool vis-à-vis des nucléophiles, un composé amino de formule:

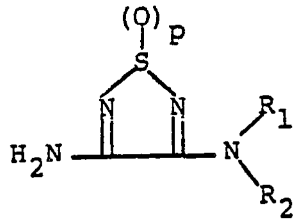

ou, on fait réagir un mercaptan de formula:

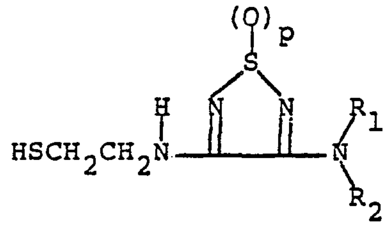

avec un alcool de formule R—Ⓐ—$(CH_2)_n$—OH en présence d'un acide minéral pour donner les composés de formule I ou II et leurs séls physiologiquement acceptables; ou, on fait réagir les composés de formule I et leurs tautomères avec un réactif alcoylant, un agent acylant ou un agent de sulfonation pour produite des dérivés de formules

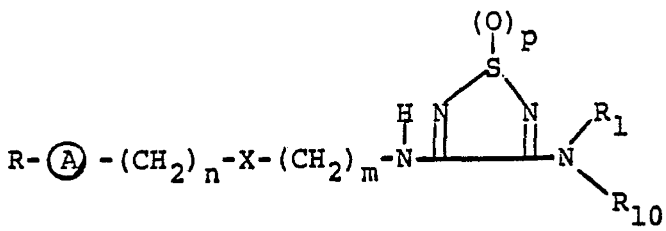

(IIIA)

et

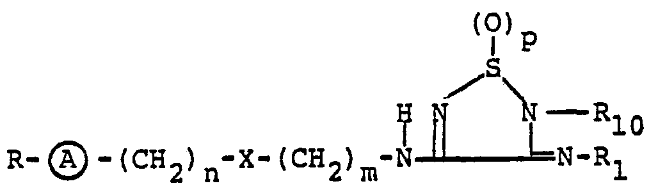

(IIIB)

et leurs sels physiologiquement acceptables.

2. Procédé selon la revendication 1, caractérisé en ce qu'on prépare des composés qui sont des membres du groupe constitué par:

3-N-[3-(2-guanidino-4-thiazolyl)phényl]amino-4-méthylamino-1,2,5-thiadiazole-1-oxyde;
3-N-[3-(2-guanidino-4-thiazolyl)phényl]amino-4-amino-1,2,5-thiadiazole-1-oxyde;
3-N-[3-[3-(diméthylaminométhyl)phénoxy]propyl]amino-4-amino-1,2,5-thiadiazole-1-oxyde;
3-N-[3-[3-(diméthylaminométhyl)phénoxy]propyl]amino-4-méthylamino-1,2,5-thiadiazole-1-oxyde;
3-N-[3-[3-(diméthylaminométhyl)phénoxy]propyl]amino-4-amino-1,2,5-thiadiazole-1,1-dioxyde;
3-N-[2-[(2-furanyl)méthylthio]éthyl]amino-4-amino-1,2,5-thiadiazole-1-oxyde;
3-N-[2-[(5-diméthylaminoéthyl-2-thiényl]méthylthio]éthyl/amino-4-amino-1,2,5-thiadiazole-1-oxyde;
3-N-[2-[(4-méthyl-5-oxazolyl)méthylthio]éthyl]amino-4-amino-1,2,5-thiadiazole-1-oxyde;
3-N-[2-[(2-furanyl)méthylthio]éthyl]amino-4-amino-1,2,5-thiadiazole-1,1-oxyde;
3-N-[2-[[5-(1-pipéridinylméthyl)-2-furanyl]méthylthio]éthyl]amino-4-amino-1,2,5-thiadiazole-1-oxyde;
3-N-[2-[(5-diméthylaminoéthyl-2-furanyl)méthylthio]éthyl]amino-4-amino-1,2,5-thiadiazole-1-oxyde;
3-N-[2-[(5-diméthylaminoéthyl-2-furanyl)méthylthio]éthyl]amino-4-méthylamino-1,2,5-thiadiazole-1-oxyde;
3-N-[2-[(5-diméthylaminoéthyl-2-furanyl)méthylthio]éthyl]amino-4-méthyl-amino-1,2,5-thiadiazole-dioxyde;
3-N-[2-[(5-diméthylaminoéthyl-2-furanyl)méthylthio]éthyl]amino-4-amino-1,2,5-thiadiazole-dioxyde;
3-N-[2-[(5-méthylaminométhyl-2-furanyl)méthylthio]éthyl]amino-4-méthylamino-1,2,5-thiadiazole-1,1-oxyde;
3-N-[2-[(5-méthylaminométhyl-2-furanyl)méthylthio]éthyl]amino-4-amino-1,2,5-thiadiazole-1-dioxyde;
3-N-[2-[(2-guanidino-4-thiazolyl)méthylthio]éthyl]amino-4-méthylamino-1,2,5-thiadiazole-1,1-dioxyde;
3-N-[2-[(2-guanidino-4-thiazolyl)méthylthio]éthyl]amino-4-amino-1,2,5-thiadiazole-1,1-oxyde;
3-N-[2-[(2-guanidino-4-thiazolyl)méthylthio]éthyl]amino-4-méthylamino-1,2,5-thiadiazole-1-oxyde;
3-N-[2-[(6-(4-morpholinylméthyl-2-benzofuranyl)méthylthio]éthyl]amino-4-amino-1,2,5-thiadiazole-1-oxyde.

3. Procédé selon la revendication 1, caractérisé en ce qu'on prépare un composé de formula I, qui est le 3-N-[3-[3-(1-pipéridinylméthyl)phénoxy]propyl]amino-4-amino-1,2,5-thiadiazole-1-oxyde.

4. Procédé selon la revendication 3, caractérisé en ce qu'on prépare la 3-N-[3-[3-(1-piperidinylméthyl)phénoxy]propyl]amino-4-amino-1,2,5-thiadiazole-1-oxyde en faisant réagir el 3-éthoxy-4-amino-1,2,5-thiadiazole-1-oxyde avec la 3-[3-(1-pipéridinylméthyl)phénoxy]propane-amine, donnant le produit final désiré.

5. Procédé du préparation de composés de formules

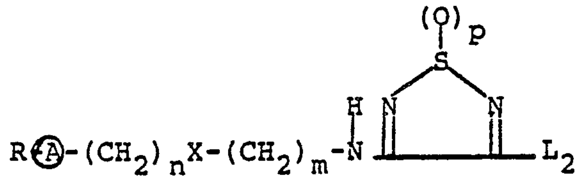

(VI)

et

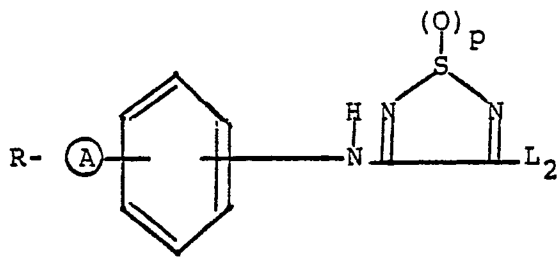

(VI A)

où R, Ⓐ, n, X et m sont tels que définis dans la revendication 1; p vaut 1 ou 2; $L_2$ est un alcoxy ayant de 1 à 5 atomes de carbone, un alcoylthio ayant de 1 à 5 atomes de carbone, un phénoxy, un phénylthio ou un halogène; et leurs sels, caractérisé en ce qu'on fair réagir un composé de formule

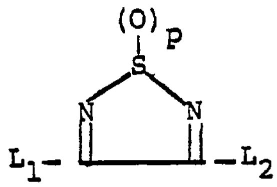

où $L_1$ est un groupe sortant avec une amine de formules

R-Ⓐ-$(CH_2)_n$-X-$(CH_2)_m$-$NH_2$

ou

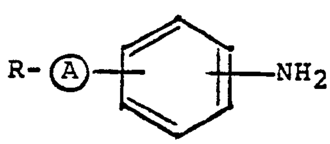

et en ce qu'on isole les composés de formules VI ou VIA ou leurs sels.

6. Procédé selon la revendication 5, caractérisé en ce qu'on prépare des composés qui sont choisis dans le groupe constitué par:

3-N-[2-[5-diméthylaminoéthyl-2-furanyl)méthylthio]éthyl]amino-4-éthoxy-1,2,5-thiadiazole-1-oxyde et

3-N-[2-[(5-diméthylaminoéthyl-2-furanyl)méthylthio]éthyl]amino-4-éthoxy-1,2,5-thiadiazole-1-dioxyde.

7. Procédé de préparation de composés de formules

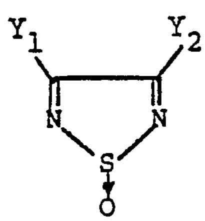

(XX)

où $Y_1$ et $Y_2$ sont des membres choisis dans les groupes suivants:

a) $Y_1$ et $Y_2$ sont semblables et représentent un alcoxy ayant de 1 à 5 atomes de carbone, un alcoylthio ayant de 1 à 5 atomes de carbone, un phénoxy ou un phénylthio; ou

b) $Y_1$ et $Y_2$ sont différents et l'un des radicaux $Y_1$ et $Y_2$ est un alcoxy ayant de 1 à 5 atomes de carbone, un phénoxy ou un phénylthio et l'autre des radicaux $Y_1$ et $Y_2$ est un groupe ayant la formule:

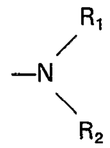

où $R_1$ et $R_2$ sont indépendamment tels que définis dans la revendication 1 ou $R_1$ et $R_2$ forment ensemble avec l'atome d'azote auquel ils sont attachés un groupe tel que défini dans la revendication 1, et

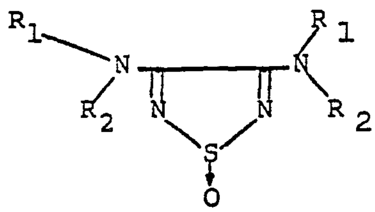

(XXI)

où $R_1$ et $R_2$ sont indépendamment tels que définis dans la revendication 1 ou

où $R_1$ et $R_2$ sont indépendamment tels que définis dans la revendication 1 ou $R_1$ et $R_2$ forment ensemble avec l'atome d'azote auquel ils sont attachés un groupe tel que défini dans la revendication 1, caractérisé en ce qu'on traite un composé de formule

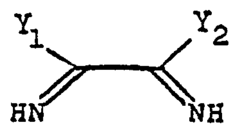

où $Y_1$ et $Y_2$ sont tels que définis en a) avec du chlorure de thionyle en présence d'une base pour former un composé de formule

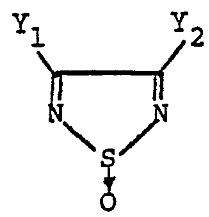

(XX)

que l'on traite avec une amine de formule:

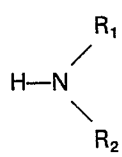

pour former des composés où $Y_1$ et $Y_2$ sont tels que définis en b) ou on traite un composé de formule:

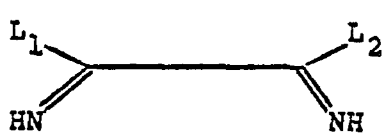

où $L_1$ et $L_2$ sont des groupes sortants choisis dans le groupe constitué par alcoxy ayant de 1 à 5 atomes de carbone, alcoylthio ayant de 1 à 5 atomes de carbone, phénoxy, ou phénylthio, avec du chlorure de thionyle en présence d'une base pour former un composé intermédiaire de formule:

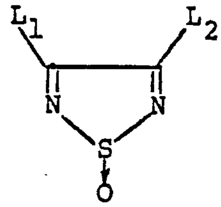

que l'on fait réagir avec une amine de formule

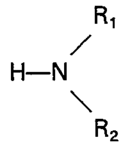

ou successivement avec deux amines différentes ayant ladite formule pour donner un composé de formula XXI

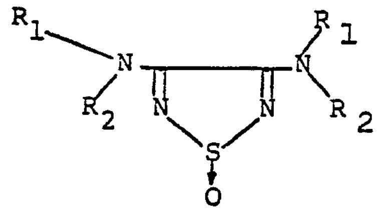

(XXI)

8. Procédé selon la revendication 7, caractérisé en ce qu'on prépare des composés de formules XX ou XXI qui sont des membres choisis dans le groupe constitué par
3,4-diéthoxy-1,2,5-thiadiazole-1-oxyde;
3,4-bis-méthylthio-1,2,5-thiadiazole-1-oxyde;
3-isopropylamino-4-méthylthio-1,2,5-thiadiazole-1-oxyde;
3-éthoxy-4-amino-1,2,5-thiadiazole-1-oxyde;
3-éthoxy-4-méthylamino-1,2,5-thiadiazole-1-oxyde;
3-éthoxy-4-(1-pyrrolidinyl)-1,2,5-thiadiazole-1-oxyde;
3-éthoxy-4-(4-morpholino)-1,2,5-thiadiazole-1-oxyde;
3,4-diamino-1,2,5-thiadiazole-1-oxyde;
3-amino-4-(2-diméthylaminoéthyl)amino-1,2,5-thiadiazole-1-oxyde;
3-amino-4-méthylamino-1,2,5-thiadiazole-1-oxyde;
3-amino-4-dodécylamino-1,2,5-thiadiazole-1-oxyde;
3-amin-4-(4-fluoranilino)-1,2,5-thiadiazole-1-oxyde;
3-amino-4-morpholino-1,2,5-thiadiazole-1-oxyde; et
3-morpholino-4-(2-pyridinométhyl)amino-1,2,5-thiadiazole-1-oxyde.

9. Procédé de préparation d'un composé de formules

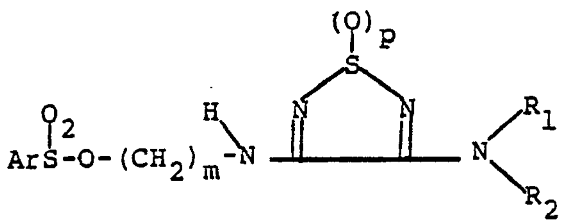

(XVII)

où Ar représente un aryle et $R_1$, $R_2$, m et p sont tels que définis dans la revendication 1 et

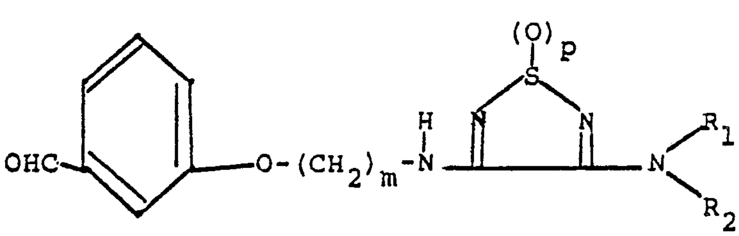

(XIX)

où $R_1$, $R_2$, m et p sont tels que définis dans la revendication 1, caractérisé en ce qu'on fait réagir un composé de formule

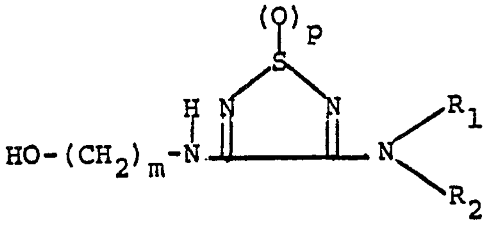

(XVI)

en présence d'une base avec un composé de formule

$ArSO_2Cl$

donnant le composé de formule XVII, que l'on fait éventuellement réagir avec un composé de formule XVIII

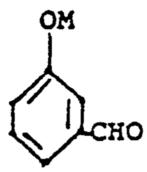

(XVIII)

donnant le composé de formule XIX.

10. Procédé de préparation d'une composition utile pour supprimer les sécrétions d'acide gastrique chez un animal ayant un excès de sécrétions d'acide gastrique, caractérisé en ce qu'on formule un composé de formules I, II, III, IIIa, IIIb, IVa ou IVb ou les produits de solvatation desdits composés ou les produits de solvatation du sel desdits composés ainsi que leurs sels d'ammonium quaternaires et N-oxydes préparés selon le procédé de la revendication 1, avec un support inerte.